# EUROPEAN PATENT APPLICATION

(11) **EP 4 740 966 A1**
(43) Date of publication of application: **13.05.2026**
(21) Application number: 24835343.5
(22) Date of filing: 02.07.2024
(51) Int. Cl.: A61K 47/68, C07K 16/46, C07K 19/00, C12N 15/13, A61K 39/395, A61P 35/00

(54) **THERAPEUTIC USE OF PROTEIN HETERODIMER**

(30) Priority: 03.07.2023 CN 202310800212
(71) Applicant: Shihuida Pharmaceutical Group (Jilin) Co., Ltd., Baishan Jilin 134399 (CN)
(72) Inventor: ZHANG, Xin, Suzhou, Jiangsu 215127 (CN); PAN, Xiaolong, Suzhou, Jiangsu 215127 (CN); FU, Shilong, Suzhou, Jiangsu 215127 (CN); ZHOU, Liyao, Suzhou, Jiangsu 215127 (CN)
(74) Representative: Murgitroyd & Company
(86) International application number: PCT/CN2024/103169
(87) International publication number: WO 2025/007862

(57) **Abstract**

Use of a protein heterodimer in the preparation of a drug, which drug is used for preventing and/or treating malignant effusion. The protein heterodimer contains a first member and a second member different from the first member, wherein the first member contains a light chain and a heavy chain, the heavy chain contains a first Fc region, and the light chain is complexed with the heavy chain to form a targeting moiety that exhibits binding specificity for a tumor antigen; and the second member contains a polypeptide containing an immunomodulator fused to a second Fc region. The first member and the second member are associated by means of the complexing of the first Fc region with the second Fc region so as to form the heterodimer. In addition, the first Fc region contains a first modification and/or the second Fc region contains a second modification, and these modifications can more effectively promote heterodimerization between the first member and the second member.

## Description

### TECHNICAL FIELD

The present disclosure relates to the field of biomedicine, and in particular to therapeutic use of a proteinaceous heterodimer.

### BACKGROUND

Malignant pleural effusion (MPE) is one of the most common complications of advanced metastatic cancer, which is caused by pleural metastasis and contains malignant cells. Tumor cells infiltrate the pleural space by direct infiltration, hematogenous metastasis, or lymphatic diffusion, and pleural effusion may be the result of tumor growth obstructing lymphatic drainage.

Malignant ascites is a clinical manifestation of advanced stages of various malignant tumors, which generally occurs at the late stage of the disease. The occurrence of malignant ascites is often accompanied by distant metastasis of malignant tumors, at which point drug treatment has become difficult. Therefore, the formation of ascites indicates that there is generally a lack of effective treatment methods in clinical practice. The formation of ascites is particularly prominent in patients with advanced ovarian, pancreatic, and gastrointestinal malignant tumors. At present, the clinical treatment mainly includes: simple ascites extraction, intra-abdominal injection of chemotherapeutic drugs, hyperthermic intraperitoneal perfusion therapy, and the like. However, the means described above are often accompanied by significant toxic effects.

Therefore, there is an urgent need to obtain an effective method for treating malignant pleural effusion and malignant ascites.

### SUMMARY

The present disclosure provides use of a proteinaceous heterodimer, a mixture for producing the proteinaceous heterodimer, a nucleic acid encoding the proteinaceous heterodimer, and the like in the treatment of a malignant effusion, such as malignant pleural effusion and malignant ascites. The use described herein provides high utilization efficiency and low side effects for the proteinaceous heterodimer, and has very broad application prospects.

In one aspect, the present disclosure provides use of a proteinaceous heterodimer in the manufacture of a medicament for preventing and/or treating a malignant effusion, wherein the proteinaceous heterodimer may comprise a first member and a second member different from the first member, the first member may comprise a light chain and a heavy chain comprising a first Fc region, the light chain may be complexed with the heavy chain to form a targeting moiety exhibiting binding specificity to a tumor antigen; the second member may comprise a polypeptide comprising an immunoregulator fused to a second Fc region; the first member may associate with the second member to form the heterodimer through complexation of the first Fc region with the second Fc region; and the first Fc region may comprise a first modification and/or the second Fc region may comprise a second modification, wherein the first modification and/or the second modification may more effectively promote heterodimerization between the first member and the second member than a knob-and-hole modification comprising a knob modification and a hole modification. For the knob-and-hole modification comprising a knob modification and a hole modification, the first Fc region may comprise the knob modification, and the second Fc region may comprise the hole modification. Alternatively, the first Fc region may comprise the hole modification, and the second Fc region may comprise the knob modification.

In some embodiments, the first modification is different from the knob modification or the hole modification, and/or the second modification is different from the knob modification or the hole modification.

In some embodiments, when expressed in a mammalian cell, a yield of the proteinaceous heterodimer is at least 10% higher than that of a reference protein, and the reference protein differs from the proteinaceous heterodimer in that the reference protein: i) comprises the knob modification in the first Fc region, ii) comprises the hole modification in the second Fc region, and iii) does not comprise the first modification and the second modification of the proteinaceous heterodimer of the present disclosure simultaneously. The mammalian cell may be selected from the group consisting of a HEK293 cell, a CHO cell, a COS-1 cell, and an NS0 cell.

In some embodiments, the first Fc region comprises the first modification, the second Fc region comprises the second modification, and neither the first modification nor the second modification is the same as the knob modification or the hole modification.

In some embodiments, the polypeptide comprised in the second member is a fusion protein, and a C-terminus of the immunoregulator is directly or indirectly fused to an N-terminus of the second Fc region to form the fusion protein.

In some embodiments, the tumor antigen is selected from the group consisting of EGFR, an EGFR mutant, HER2/neu, GPC3, FAP, Muc1, MUC5AC, and Mesothelin.

In some embodiments, the light chain of the targeting moiety contains CDRs comprising amino acid sequences that are at least 60%, 70%, 75%, 80%, 85%, 90%, 95%, 96%, 97%, 98%, 99%, or 100% identical to those comprised in the corresponding CDRs of a light chain of an antibody specifically directed to a tumor antigen.

In some embodiments, the light chain of the targeting moiety contains a variable region comprising an amino acid sequence that is at least 60%, 70%, 75%, 80%, 85%, 90%, 95%, 96%, 97%, 98%, 99%, or 100% identical to that comprised in the corresponding variable region of a light chain of an antibody specifically directed to a tumor antigen.

In some embodiments, the light chain of the targeting moiety contains an amino acid sequence that is at least 60%, 70%, 75%, 80%, 85%, 90%, 95%, 96%, 97%, 98%, 99%, or 100% identical to that comprised in the corresponding amino acid sequence of a light chain of an antibody specifically directed to a tumor antigen.

In some embodiments, the heavy chain of the targeting moiety contains CDRs comprising amino acid sequences that are at least 60%, 70%, 75%, 80%, 85%, 90%, 95%, 96%, 97%, 98%, 99%, or 100% identical to those comprised in the corresponding CDRs of a heavy chain of an antibody specifically directed to a tumor antigen.

In some embodiments, the heavy chain of the targeting moiety contains a variable region comprising an amino acid sequence that is at least 60%, 70%, 75%, 80%, 85%, 90%, 95%, 96%, 97%, 98%, 99%, or 100% identical to that comprised in the corresponding variable region of a heavy chain of an antibody specifically directed to a tumor antigen.

In some embodiments, the heavy chain of the targeting moiety contains an amino acid sequence that is at least 60%, 70%, 75%, 80%, 85%, 90%, 95%, 96%, 97%, 98%, 99%, or 100% identical to that comprised in the corresponding amino acid sequence of a heavy chain of an antibody specifically directed to a tumor antigen.

In some embodiments, the light chain of the targeting moiety contains CDRs comprising amino acid sequences that are at least 60%, 70%, 75%, 80%, 85%, 90%, 95%, 96%, 97%, 98%, 99%, or 100% identical to those comprised in the corresponding CDRs of a light chain of an antibody specifically directed to a tumor antigen; and the heavy chain of the targeting moiety contains CDRs comprising amino acid sequences that are at least 60%, 70%, 75%, 80%, 85%, 90%, 95%, 96%, 97%, 98%, 99%, or 100% identical to those comprised in the corresponding CDRs of a heavy chain of an antibody specifically directed to a tumor antigen.

In some embodiments, the light chain of the targeting moiety contains a variable region comprising an amino acid sequence that is at least 60%, 70%, 75%, 80%, 85%, 90%, 95%, 96%, 97%, 98%, 99%, or 100% identical to that comprised in the corresponding variable region of a light chain of an antibody specifically directed to a tumor antigen; and the heavy chain of the targeting moiety contains a variable region comprising an amino acid sequence that is at least 60%, 70%, 75%, 80%, 85%, 90%, 95%, 96%, 97%, 98%, 99%, or 100% identical to that comprised in the corresponding variable region of a heavy chain of an antibody specifically directed to a tumor antigen.

In some embodiments, the light chain of the targeting moiety contains an amino acid sequence that is at least 60%, 70%, 75%, 80%, 85%, 90%, 95%, 96%, 97%, 98%, 99%, or 100% identical to that comprised in the corresponding amino acid sequence of a light chain of an antibody specifically directed to a tumor antigen; and the heavy chain of the targeting moiety contains an amino acid sequence that is at least 60%, 70%, 75%, 80%, 85%, 90%, 95%, 96%, 97%, 98%, 99%, or 100% identical to that comprised in the corresponding amino acid sequence of a heavy chain of an antibody specifically directed to a tumor antigen.

In some embodiments, the antibody specifically directed to a tumor antigen is selected from the group consisting of anti-EGFR, anti-EGFR mutant, anti-HER2/neu, anti-GPC3, anti-FAP, anti-Muc1, anti-MUC5AC, and anti-Mesothelin.

In some embodiments, the immunoregulator augments an immune response. In some embodiments, the immunoregulator reduces an immune response.

In some embodiments, the immunoregulator is a cytokine. For example, the immunoregulator may be a cytokine selected from the group consisting of an interferon, an interleukin, a chemokine, a lymphokine, and a tumor necrosis factor.

In some embodiments, the immunoregulator is an interferon selected from the group consisting of interferon alpha, interferon lambda, and interferon beta.

In some embodiments, the immunoregulator is an interleukin, and the interleukin comprises interleukin 10, interleukin 2, and/or super interleukin 2.

In some embodiments, the first Fc region and the second Fc region are from an Fc region of an immunoglobulin.

For example, the immunoglobulin may be selected from the group consisting of IgG1, IgG2, IgG3, and IgG4.

In some embodiments, the first Fc region and the second Fc region are from an Fc region of an immunoglobulin, and the immunoglobulin is a human IgG1.

In some embodiments, the second Fc region is fused in frame to the immunoregulator.

In some embodiments, the second Fc region is fused in frame to the immunoregulator via a linker.

In some embodiments, the polypeptide comprised in the second member comprises two or more immunoregulators, the two or more immunoregulators are fused in frame to each other and to the second Fc region, and wherein the two or more immunoregulators are located N-terminal to the second Fc region. For example, the two or more immunoregulators may be the same.

In some embodiments, the first modification comprises an amino acid substitution at position T366, and an amino acid substitution at one or more positions selected from the group consisting of: Y349, F405, K409, D399, K360, Q347, K392, and S354, wherein the position of the amino acid is determined according to the EU index of the KABAT number.

In some embodiments, the first modification comprises an amino acid substitution selected from the group consisting of Y349C, Y349D, D399S, F405K, K360E, K409A, K409E, Q347E, Q347R, S354D, K392D, and T366W.

In some embodiments, the first modification comprises 2-5 amino acid substitutions.

In some embodiments, the first modification comprises an amino acid substitution at a group of positions selected from any of the following groups: 1) Y349 and T366; 2) Y349, T366, and F405; 3) Y349, T366, and K409; 4) Y349, T366, F405, K360, and Q347; 5) Y349, T366, F405, and Q347; 6) Y349, T366, K409, K360, and Q347; 7) Y349, T366, K409, and Q347; 8) T366, K409, and K392; 9) T366 and K409; 10) T366, K409, Y349, and S354; 11) T366 and F405; 12) T366, F405, and D399; and 13) T366, F405, Y349, and S354.

In some embodiments, the first modification comprises a group of amino acid substitutions selected from any of the following groups: 1) Y349C and T366W; 2) Y349C, T366W, and F405K; 3) Y349C, T366W, and K409E; 4) Y349C, T366W, and K409A; 5) Y349C, T366W, F405K, K360E, and Q347E; 6) Y349C, T366W, F405K, and Q347R; 7) Y349C, T366W, K409A, K360E, and Q347E; 8) Y349C, T366W, K409A, and Q347R; 9) T366W, K409A, and K392D; 10) T366W and K409A; 11) T366W, K409A, and Y349D; 12) T366W, K409A, Y349D, and S354D; 13) T366W and F405K; 14) T366W, F405K, and D399S; 15) T366W, F405K, and Y349D; and 16) T366W, F405K, Y349D, and S354D. In some embodiments, the second modification comprises amino acid substitutions at positions T366, L368, and Y407, as well as an amino acid substitution at one or more positions selected from the group consisting of D356, D399, E357, F405, K360, K392, K409, and Q347, wherein the position of the amino acid is determined according to the EU index of the KABAT number.

In some embodiments, the amino acid substitution comprised by the second modification is selected from the group consisting of D356C, D399S, E357A, F405K, K360E, K392D, K409A, L368A, L368G, Q347E, Q347R, T366S, Y407A, and Y407V.

In some embodiments, the second modification comprises amino acid substitutions at 4-6 positions. In some embodiments, the second modification comprises an amino acid substitution at a group of positions selected from any of the following groups: 1) D356, T366, L368, Y407, and F405; 2) D356, T366, L368, and Y407; 3) D356, T366, L368, Y407, and Q347; 4) D356, T366, L368, Y407, K360, and Q347; 5) D356, T366, L368, Y407, F405, and Q347; 6) D356, T366, L368, Y407, F405, K360, and Q347; 7) T366, L368, Y407, D399, and F405; 8) T366, L368, Y407, and F405; 9) T366, L368, Y407, F405, and E357; 10) T366, L368, Y407, and K409; 11) T366, L368, Y407, K409, and K392; and 12) T366, L368, Y407, K409, and E357.

In some embodiments, the second modification comprises a group of amino acid substitutions selected from any of the following groups: 1) D356C, T366S, L368A, Y407V, and F405K; 2) D356C, T366S, L368A, and Y407V; 3) D356C, T366S, L368A, Y407V, and Q347R; 4) D356C, T366S, L368A, Y407V, K360E, and Q347E; 5) D356C, T366S, L368A, Y407V, F405K, and Q347R; 6) D356C, T366S, L368A, Y407V, F405K, K360E, and Q347E; 7) T366S, L368A, Y407V, D399S, and F405K; 8) T366S, L368G, Y407A, and F405K; 9) T366S, L368A, Y407V, F405K, and E357A; 10) T366S, L368A, Y407V, and K409A; 11) T366S, L368A, F409K, Y407V, K409A, and K392D; 12) T366S, L368G, Y407A, and K409A; and 13) T366S, L368A, Y407V, K409A, and E357A.

In some embodiments, the first Fc region comprises the first modification, and the second Fc region comprises the second modification. The first modification comprises an amino acid substitution at position T366, and an amino acid substitution at one or more positions selected from the group consisting of: Y349, F405, K409, D399, K360, Q347, K392, and S354, wherein the position of the amino acid is determined according to the EU index of the KABAT number; and the second modification comprises amino acid substitutions at positions T366, L368, and Y407, as well as an amino acid substitution at one or more positions selected from the group consisting of D356, D399, E357, F405, K360, K392, K409, and Q347, wherein the position of the amino acid is determined according to the EU index of the KABAT number. The first modification and the second modification may be as defined in the present disclosure.

In some embodiments, the first Fc region comprises the first modification, the second Fc region comprises the second modification, and the first modification and the second modification comprise an amino acid substitution at a group of positions selected from any of the following groups: 1) the first modification: Y349 and T366; and the second modification: D356, T366, L368, Y407, and F405; 2) the first modification: Y349, T366, and F405; and the second modification: D356, T366, L368, and Y407; 3) the first modification: Y349, T366, and K409; and the second modification: D356, T366, L368, Y407, and F405; 4) the first modification: Y349, T366, F405, K360, and Q347; and the second modification: D356, T366, L368, Y407, and Q347; 5) the first modification: Y349, T366, F405, and Q347; and the second modification: D356, T366, L368, Y407, K360, and Q347; 6) the first modification: Y349, T366, K409, K360, and Q347; and the second modification: D356, T366, L368, Y407, F405, and Q347; 7) the first modification: Y349, T366, K409, and Q347; and the second modification: D356, T366, L368, Y407, F405, K360, and Q347; 8) the first modification: T366, K409, and K392; and the second modification: T366, L368, Y407, D399, and F405; 9) the first modification: T366 and K409; and the second modification: T366, L368, Y407, and F405; 10) the first modification: T366, K409, and Y349; and the second modification: T366, L368, Y407, F405, and E357; 11) the first modification: T366, K409, Y349, and S354; and the second modification: T366, L368, Y407, F405, and E357; 12) the first modification: T366 and F405; and the second modification: T366, L368, Y407, and K409; 13) the first modification: T366, F405, and D399; and the second modification: T366, L368, Y407, K409, and K392; 14) the first modification: T366, F405, and Y349; and the second modification: T366, L368, Y407, K409, and E357; and 15) the first modification: T366, F405, Y349, and S354; and the second modification: T366, L368, Y407, K409, and E357; wherein the position of the amino acid is determined according to the EU index of the KABAT number.

In some embodiments, the first Fc region comprises the first modification, and the second Fc region comprises the second modification, wherein the first modification and the second modification comprise a group of amino acid substitutions selected from any of the following groups: 1) the first modification: Y349C and T366W; and the second modification: D356C, T366S, L368A, Y407V, and F405K; 2) the first modification: Y349C, T366W, and F405K; and the second modification: D356C, T366S, L368A, and Y407V; 3) the first modification: Y349C, T366W, and K409E; and the second modification: D356C, T366S, L368A, Y407V, and F405K; 4) the first modification: Y349C, T366W, and K409A; and the second modification: D356C, T366S, L368A, Y407V, and F405K; 5) the first modification: Y349C, T366W, F405K, K360E, and Q347E; and the second modification: D356C, T366S, L368A, Y407V, and Q347R; 6) the first modification: Y349C, T366W, F405K, and Q347R; and the second modification: D356C, T366S, L368A, Y407V, K360E, and Q347E; 7) the first modification: Y349C, T366W, K409A, K360E, and Q347E; and the second modification: D356C, T366S, L368A, Y407V, F405K, and Q347R; 8) the first modification: Y349C, T366W, K409A, and Q347R; and the second modification: D356C, T366S, L368A, Y407V, F405K, K360E, and Q347E; 9) the first modification: T366W, K409A, and K392D; and the second modification: T366S, L368A, Y407V, D399S, and F405K; 10) the first modification: T366W and K409A; and the second modification: T366S, L368G, Y407A, and F405K; 11) the first modification: T366W, K409A, and Y349D; and the second modification: T366S, L368A, Y407V, F405K, and E357A; 12) the first modification: T366W, K409A, Y349D, and S354D; and the second modification: T366S, L368A, Y407V, F405K, and E357A; 13) the first modification: T366W and F405K; and the second modification: T366S, L368A, Y407V, and K409A; 14) the first modification: T366W, F405K, and D399S; and the second modification: T366S, L368A, Y407V, K409A, and K392D; 15) the first modification: T366W and F405K; and the second modification: T366S, L368G, Y407A, and K409A; 16) the first modification: T366W, F405K, and Y349D; and the second modification: T366S, L368A, Y407V, K409A, and E357A; and 17) the first modification: T366W, F405K, Y349D, and S354D; and the second modification: T366S, L368A, Y407V, K409A, and E357A; wherein the position of the amino acid is determined according to the EU index of the KABAT number.

In some embodiments, the first Fc region comprises the first modification, the second Fc region comprises the second modification, the first modification comprises the amino acid substitutions T366W and K409A, and the second modification comprises the amino acid substitutions T366S, L368G, Y407A, and F405K, wherein the position of the amino acid is determined according to the EU index of the KABAT number.

In some embodiments, the targeting moiety specifically binds to EGFR, the light chain of the first member comprises CDR1-3, the amino acid sequence of the CDR1 is as set forth in SEQ ID NO: 101, the amino acid sequence of the CDR2 is as set forth in SEQ ID NO: 102, and the amino acid sequence of the CDR3 is as set forth in SEQ ID NO: 103.

In some embodiments, the targeting moiety specifically binds to EGFR, the light chain of the first member comprises a light chain variable region, and the amino acid sequence of the light chain variable region is as set forth in SEQ ID NO: 104.

In some embodiments, the targeting moiety specifically binds to EGFR, and the amino acid sequence of the light chain of the first member is as set forth in SEQ ID NO: 37.

In some embodiments, the targeting moiety specifically binds to EGFR, the heavy chain of the first member comprises CDR1-3, the amino acid sequence of the CDR1 is as set forth in SEQ ID NO: 105, the amino acid sequence of the CDR2 is as set forth in SEQ ID NO: 106, and the amino acid sequence of the CDR3 is as set forth in SEQ ID NO: 107.

In some embodiments, the targeting moiety specifically binds to EGFR, the heavy chain of the first member comprises a heavy chain variable region, and the amino acid sequence of the heavy chain variable region is as set forth in SEQ ID NO: 108.

In some embodiments, the targeting moiety specifically binds to EGFR, and the amino acid sequence of the heavy chain of the first member is as set forth in SEQ ID NO: 39.

In some embodiments, the targeting moiety specifically binds to an EGFR mutant, the light chain of the first member comprises CDR1-3, the amino acid sequence of the CDR1 is as set forth in SEQ ID NO: 109, the amino acid sequence of the CDR2 is as set forth in SEQ ID NO: 110, and the amino acid sequence of the CDR3 is as set forth in SEQ ID NO: 111.

In some embodiments, the targeting moiety specifically binds to an EGFR mutant, the light chain of the first member comprises a light chain variable region, and the amino acid sequence of the light chain variable region is as set forth in SEQ ID NO: 112.

In some embodiments, the targeting moiety specifically binds to an EGFR mutant, and the amino acid sequence of the light chain of the first member is as set forth in SEQ ID NO: 53.

In some embodiments, the targeting moiety specifically binds to an EGFR mutant, the heavy chain of the first member comprises CDR1-3, the amino acid sequence of the CDR1 is as set forth in SEQ ID NO: 113, the amino acid sequence of the CDR2 is as set forth in SEQ ID NO: 114, and the amino acid sequence of the CDR3 is as set forth in SEQ ID NO: 115.

In some embodiments, the targeting moiety specifically binds to an EGFR mutant, the heavy chain of the first member comprises a heavy chain variable region, and the amino acid sequence of the heavy chain variable region is as set forth in SEQ ID NO: 116.

In some embodiments, the targeting moiety specifically binds to an EGFR mutant, and the amino acid sequence of the heavy chain of the first member is as set forth in SEQ ID NO: 55.

In some embodiments, the targeting moiety specifically binds to HER2/neu, the light chain of the first member comprises CDR1-3, the amino acid sequence of the CDR1 is selected from SEQ ID NO: 117 and SEQ ID NO: 125, the amino acid sequence of the CDR2 is selected from SEQ ID NO: 118 and SEQ ID NO: 126, and the amino acid sequence of the CDR3 is selected from SEQ ID NO: 119 and SEQ ID NO: 127.

In some embodiments, the targeting moiety specifically binds to HER2/neu, the light chain of the first member comprises a light chain variable region, and the amino acid sequence of the light chain variable region is selected from SEQ ID NO: 120 and SEQ ID NO: 128.

In some embodiments, the targeting moiety specifically binds to HER2/neu, and the amino acid sequence of the light chain of the first member is selected from SEQ ID NO: 45 and SEQ ID NO: 49. In some embodiments, the targeting moiety specifically binds to HER2/neu, the heavy chain of the first member comprises CDR1-3, the amino acid sequence of the CDR1 is selected from SEQ ID NO: 121 and SEQ ID NO: 129, the amino acid sequence of the CDR2 is selected from SEQ ID NO: 122 and SEQ ID NO: 130, and the amino acid sequence of the CDR3 is selected from SEQ ID NO: 123 and SEQ ID NO: 131.

In some embodiments, the targeting moiety specifically binds to HER2/neu, the heavy chain of the first member comprises a heavy chain variable region, and the amino acid sequence of the heavy chain variable region is selected from SEQ ID NO: 124 and SEQ ID NO: 132.

In some embodiments, the targeting moiety specifically binds to HER2/neu, and the amino acid sequence of the heavy chain of the first member is selected from SEQ ID NO: 47 and SEQ ID NO: 51.

In some embodiments, the targeting moiety specifically binds to GPC3, the antibody light chain of the first member comprises CDR1-3, the amino acid sequence of the CDR1 is as set forth in SEQ ID NO: 133, the amino acid sequence of the CDR2 is as set forth in SEQ ID NO: 134, and the amino acid sequence of the CDR3 is as set forth in SEQ ID NO: 135.

In some embodiments, the targeting moiety specifically binds to GPC3, the light chain of the first member comprises a light chain variable region, and the amino acid sequence of the light chain variable region is as set forth in SEQ ID NO: 136.

In some embodiments, the targeting moiety specifically binds to GPC3, and the amino acid sequence of the light chain of the first member is as set forth in SEQ ID NO: 57.

In some embodiments, the targeting moiety specifically binds to GPC3, the heavy chain of the first member comprises CDR1-3, the amino acid sequence of the CDR1 is as set forth in SEQ ID NO: 137, the amino acid sequence of the CDR2 is as set forth in SEQ ID NO: 138, and the amino acid sequence of the CDR3 is as set forth in SEQ ID NO: 139.

In some embodiments, the targeting moiety specifically binds to GPC3, the antibody heavy chain of the first member comprises a heavy chain variable region, and the amino acid sequence of the heavy chain variable region is as set forth in SEQ ID NO: 140.

In some embodiments, the targeting moiety specifically binds to GPC3, and the amino acid sequence of the heavy chain of the first member is as set forth in SEQ ID NO: 59.

In some embodiments, the targeting moiety specifically binds to FAP, the light chain of the first member comprises CDR1-3, the amino acid sequence of the CDR1 is as set forth in SEQ ID NO: 141, the amino acid sequence of the CDR2 is as set forth in SEQ ID NO: 142, and the amino acid sequence of the CDR3 is as set forth in SEQ ID NO: 143.

In some embodiments, the targeting moiety specifically binds to FAP, the light chain of the first member comprises a light chain variable region, and the amino acid sequence of the light chain variable region is as set forth in SEQ ID NO: 144.

In some embodiments, the targeting moiety specifically binds to FAP, and the amino acid sequence of the light chain of the first member is as set forth in SEQ ID NO: 61.

In some embodiments, the targeting moiety specifically binds to FAP, the heavy chain of the first member comprises CDR1-3, the amino acid sequence of the CDR1 is as set forth in SEQ ID NO: 145, the amino acid sequence of the CDR2 is as set forth in SEQ ID NO: 146, and the amino acid sequence of the CDR3 is as set forth in SEQ ID NO: 147.

In some embodiments, the targeting moiety specifically binds to FAP, the heavy chain of the first member comprises a heavy chain variable region, and the amino acid sequence of the heavy chain variable region is as set forth in SEQ ID NO: 148.

In some embodiments, the targeting moiety specifically binds to FAP, and the amino acid sequence of the heavy chain of the first member is as set forth in SEQ ID NO: 63.

In some embodiments, the targeting moiety specifically binds to Muc1, the light chain of the first member comprises CDR1-3, the amino acid sequence of the CDR1 is as set forth in SEQ ID NO: 149, the amino acid sequence of the CDR2 is as set forth in SEQ ID NO: 150, and the amino acid sequence of the CDR3 is as set forth in SEQ ID NO: 151.

In some embodiments, the targeting moiety specifically binds to Muc1, the light chain of the first member comprises a light chain variable region, and the amino acid sequence of the light chain variable region is as set forth in SEQ ID NO: 152.

In some embodiments, the targeting moiety specifically binds to Muc1, and the amino acid sequence of the light chain of the first member is as set forth in SEQ ID NO: 65.

In some embodiments, the targeting moiety specifically binds to Muc1, the heavy chain of the first member comprises CDR1-3, the amino acid sequence of the CDR1 is as set forth in SEQ ID NO: 153, the amino acid sequence of the CDR2 is as set forth in SEQ ID NO: 154, and the amino acid sequence of the CDR3 is as set forth in SEQ ID NO: 155.

In some embodiments, the targeting moiety specifically binds to Muc1, the heavy chain of the first member comprises a heavy chain variable region, and the amino acid sequence of the heavy chain variable region is as set forth in SEQ ID NO: 156.

In some embodiments, the targeting moiety specifically binds to Muc1, and the amino acid sequence of the heavy chain of the first member is as set forth in SEQ ID NO: 67.

In some embodiments, the targeting moiety specifically binds to Mesothelin, the light chain of the first member comprises CDR1-3, the amino acid sequence of the CDR1 is as set forth in SEQ ID NO: 165, the amino acid sequence of the CDR2 is as set forth in SEQ ID NO: 166, and the amino acid sequence of the CDR3 is as set forth in SEQ ID NO: 167.

In some embodiments, the targeting moiety specifically binds to Mesothelin, the light chain of the first member comprises a light chain variable region, and the amino acid sequence of the light chain variable region is as set forth in SEQ ID NO: 168.

In some embodiments, the targeting moiety specifically binds to Mesothelin, and the amino acid sequence of the light chain of the first member is as set forth in SEQ ID NO: 73.

In some embodiments, the targeting moiety specifically binds to Mesothelin, the heavy chain of the first member comprises CDR1-3, the amino acid sequence of the CDR1 is as set forth in SEQ ID NO: 169, the amino acid sequence of the CDR2 is as set forth in SEQ ID NO: 170, and the amino acid sequence of the CDR3 is as set forth in SEQ ID NO: 171.

In some embodiments, the targeting moiety specifically binds to Mesothelin, the heavy chain of the first member comprises a heavy chain variable region, and the amino acid sequence of the heavy chain variable region is as set forth in SEQ ID NO: 172.

In some embodiments, the targeting moiety specifically binds to Mesothelin, and the amino acid sequence of the heavy chain of the first member is as set forth in SEQ ID NO: 75.

In some embodiments, the targeting moiety specifically binds to MUC5AC, the light chain of the first member comprises CDR1-3, the amino acid sequence of the CDR1 is as set forth in SEQ ID NO: 157, the amino acid sequence of the CDR2 is as set forth in SEQ ID NO: 158, and the amino acid sequence of the CDR3 is as set forth in SEQ ID NO: 159.

In some embodiments, the targeting moiety specifically binds to MUC5AC, the light chain of the first member comprises a light chain variable region, and the amino acid sequence of the light chain variable region is as set forth in SEQ ID NO: 160.

In some embodiments, the targeting moiety specifically binds to MUC5AC, and the amino acid sequence of the light chain of the first member is as set forth in SEQ ID NO: 69.

In some embodiments, the targeting moiety specifically binds to MUC5AC, the heavy chain of the first member comprises CDR1-3, the amino acid sequence of the CDR1 is as set forth in SEQ ID NO: 161, the amino acid sequence of the CDR2 is as set forth in SEQ ID NO: 162, and the amino acid sequence of the CDR3 is as set forth in SEQ ID NO: 163.

In some embodiments, the targeting moiety specifically binds to MUC5AC, the heavy chain of the first member comprises a heavy chain variable region, and the amino acid sequence of the heavy chain variable region is as set forth in SEQ ID NO: 164.

In some embodiments, the targeting moiety specifically binds to MUC5AC, and the amino acid sequence of the heavy chain of the first member is as set forth in SEQ ID NO: 71.

In some embodiments, in the heavy chain of the first member, the amino acid sequence of the first Fc region is selected from SEQ ID NOs: 1, 4, 5, 6, 7, 9, 11, 13, 15, 17, 19, 21, 22, 24, 26, 27, and 29. In some embodiments, the amino acid sequence of the immunoregulator comprised in the second member is selected from SEQ ID NOs: 173-180.

In some embodiments, the amino acid sequence of the second Fc region comprised in the second member is selected from SEQ ID NOs: 2, 3, 8, 10, 12, 14, 16, 18, 20, 23, 25, and 28.

In some embodiments, the amino acid sequence of the polypeptide comprised in the second member is selected from SEQ ID NOs: 77, 80, 82, 84, 86, 89, 91, and 97.

In some embodiments, the amino acid sequence of the light chain comprised in the first member is selected from SEQ ID NOs: 37, 45, 49, 53, 57, 61, 65, 69, and 73, the amino acid sequence of the heavy chain comprised in the first member is selected from SEQ ID NOs: 39, 47, 51, 55, 59, 63, 67, 71, and 75, and the amino acid sequence of the polypeptide comprised in the second member is selected from SEQ ID NOs: 77, 80, 82, 84, 86, 89, 91, and 97.

In some embodiments, the knob-and-hole modification comprises a knob modification and a hole modification, wherein the knob modification comprises the amino acid substitutions Y349C and T366W, and the hole modification comprises the amino acid substitutions D356C, T366S, L368A, and Y407V, wherein the position of the amino acid is determined according to the EU index of the KABAT number. In some cases, the knob modification is comprised in the first Fc region, and the hole modification is comprised in the second Fc region. In some cases, the knob modification is comprised in the second Fc region, and the hole modification is comprised in the first Fc region.

In some embodiments, the medicament comprises a pharmaceutical composition comprising the proteinaceous heterodimer described herein and a pharmaceutically acceptable carrier.

In some embodiments, the medicament comprises a nucleic acid molecule encoding the proteinaceous heterodimer described herein.

In some embodiments, the medicament comprises a vector comprising the nucleic acid molecule.

In some embodiments, the medicament comprises a cell comprising the nucleic acid molecule or the vector.

In some embodiments, the medicament comprises a protein mixture comprising: 1) the proteinaceous heterodimer described herein; 2) a first homodimer formed by two of the first member of the proteinaceous heterodimer; and 3) a second homodimer formed by two of the second member of the proteinaceous heterodimer; wherein the percentage of the proteinaceous heterodimer in the protein mixture is at least 50%.

In some embodiments, a patient suffering from the malignant effusion comprises a tumor patient.

In some embodiments, the patient has symptoms comprising fatigue, vomiting, edema in the dorsum of the foot, loss of appetite, dyspnea, cough, chest pain, and/or abdominal pain.

In some embodiments, the tumor comprises a solid tumor and/or a non-solid tumor.

In some embodiments, the tumor comprises lung cancer, lymph cancer, liver cancer, stomach cancer, intestinal cancer, breast cancer, pancreatic cancer, ovarian cancer, and/or mesothelioma.

In some embodiments, the malignant effusion comprises malignant cells.

In some embodiments, the malignant effusion comprises blood and/or exudative fluid.

In some embodiments, the malignant effusion is associated with metastasis of the tumor to the pleura. In some embodiments, the malignant effusion is associated with metastasis of the tumor to the peritoneum.

In some embodiments, the malignant effusion is caused by a tumor comprising a primary tumor of the lung, lymph, liver, stomach, colon, breast, ovarian cancer, pancreatic cancer, bile duct, esophagus, brain, nose, and/or prostate.

In some embodiments, the malignant effusion is caused by metastasis to the thoracic cavity of a tumor comprising a primary cancer of the lung, lymph, liver, stomach, intestine, breast, ovarian cancer, and/or mesothelioma.

In some embodiments, the malignant effusion is caused by metastasis to the abdominal cavity of a tumor comprising ovarian cancer, pancreatic cancer, and a primary cancer of the bile duct, esophagus, sarcoma, and/or liver.

In some embodiments, the malignant effusion comprises malignant pleural effusion (MPE) and/or malignant ascites.

In some embodiments, the malignant pleural effusion is associated with non-small cell lung cancer. In some embodiments, the medicament is formulated for oral administration, intravenous administration, intramuscular administration, in-situ administration at the site of a tumor, inhalation, rectal administration, vaginal administration, transdermal administration, or administration via subcutaneous repository.

In some embodiments, the medicament is configured to comprise a drainage device.

In some embodiments, the medicament is capable of inhibiting growth of a tumor or a tumor cell. In another aspect, the present disclosure further provides a method for constructing an animal model with a malignant effusion, comprising injecting a tumor cell into the thoracic cavity and/or the abdominal cavity of an animal.

In some embodiments, a dose for the injection is about 1 × 10⁵ to about 2 × 10⁶ cells injected per animal.

In some embodiments, the animal comprises a mouse.

In some embodiments, the injection comprises intraperitoneal injection, intra-abdominal injection, and/or intrathoracic injection.

In some embodiments, the tumor cell is derived from a solid tumor and/or a non-solid tumor.

In some embodiments, the tumor comprises lung cancer, lymph cancer, liver cancer, stomach cancer, intestinal cancer, breast cancer, pancreatic cancer, sarcoma, ovarian cancer, and/or mesothelioma. In some embodiments, the tumor cells comprise an H22-Luc cell, a B16-EGFR cell, an MC38-EGFR cell, a Pan02 mouse pancreatic cancer cell, an S180 mouse sarcoma cell, and/or a Lewis-luc cell.

In some embodiments, the tumor cell is capable of causing a tumor in the animal.

In some embodiments, the tumor metastasizes to the pleura and/or the peritoneum of the animal and produces the malignant effusion.

In some embodiments, the malignant effusion comprises malignant cells.

In some embodiments, the malignant effusion comprises blood and/or exudative fluid.

In some embodiments, the malignant effusion comprises malignant pleural effusion (MPE) and/or malignant ascites.

In some embodiments, the malignant pleural effusion is associated with non-small cell lung cancer. The present disclosure further provides a tissue or organ, or a culture thereof, in an animal model with a malignant effusion prepared by the method described above.

The present disclosure further provides an animal model with a malignant effusion or progeny thereof prepared by the method described above.

The present disclosure further provides use of the tissue or organ, or the culture thereof described above, or the animal model with a malignant effusion or the progeny thereof in the screening, validation, evaluation, and/or research of an anti-tumor drug or as a model system for pharmacological, immunological, microbiological, and medical research.

Additional aspects and advantages of the present disclosure will become readily apparent to those skilled in the art from the following detailed description, wherein only illustrative embodiments of the present disclosure are shown and described. As will be realized, the present disclosure is capable of other and different embodiments, and its several details are capable of modifications in various obvious respects, all without departing from the present disclosure. Accordingly, the drawings and description are to be regarded as illustrative in nature, and not as restrictive.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the present disclosure are set forth with particularity in the appended claims. A better understanding of the features and advantages of the present disclosure will be obtained by reference to the following detailed description that sets forth illustrative embodiments, in which the principles of the present disclosure are utilized, and the accompanying drawings of which:
FIG. 1 illustrates examples of the proteinaceous heterodimers according to the present disclosure;
FIGs. 2A-2C illustrate results of formation of the proteinaceous heterodimers according to the present disclosure, as analyzed by SDS-PAGE;
FIG. 3 illustrates the survival rate of H22-Luc mouse ascites models with malignant ascites caused by liver cancer following treatment of the proteinaceous heterodimer according to the present disclosure;
FIGs. 4A-4B illustrate malignant ascites caused by liver cancer and *in vivo* imaging data in H22-Luc mouse ascites models after the completion of inoculation according to the present disclosure;
FIGs. 5A-5B illustrate malignant ascites caused by liver cancer and *in vivo* imaging data in H22-Luc mouse ascites models following treatment of the proteinaceous heterodimer according to the present disclosure;
FIGs. 6A-6B illustrate the effects of the treatment with the proteinaceous heterodimer according to the present disclosure on malignant pleural effusion and body weight in Lewis mouse lung cancer models;
FIG. 7 illustrates the effects of the treatment with the proteinaceous heterodimer according to the present disclosure on malignant pleural effusion and the number of metastatic lesions in Lewis mouse lung cancer models.

### DETAILED DESCRIPTION OF THE PRESENT DISCLOSURE

Before the embodiments of the present disclosure are described, it is to be understood that such embodiments are provided by way of example only, and that various alternatives to the embodiments of the present disclosure described herein may be employed in practicing the present disclosure. Numerous variations, changes, and substitutions will occur to those skilled in the art without departing from the present disclosure.

Unless otherwise defined, all technical and scientific terms used herein have the same meaning as commonly understood by one of ordinary skill in the art to which the present disclosure belongs. Although methods and materials similar or equivalent to those described herein can be used in the practice or testing of the present disclosure, suitable methods and materials are described below. In case of conflict, the patent specification, including definitions, will control. In addition, the materials, methods, and examples are illustrative only and not intended to be limiting. Numerous variations, changes, and substitutions will occur to those skilled in the art without departing from the present disclosure.

The singular forms "a," "an," and "the," as used herein, generally include plural references unless the context clearly dictates otherwise.

The term "proteinaceous," as used herein, generally refers to a material or molecule that is of, relating to, resembling, or being a polypeptide or a protein. For example, a proteinaceous heterodimer of the present disclosure may be a heterodimer protein, or a heterodimer comprising two or more polypeptides.

The term "heterodimer," as used herein, generally refers to a molecule (e.g., a proteinaceous molecule) composed of two different members. The two members of a heterodimer may differ in structure, function, activity, and/or composition. For example, the two different members may comprise polypeptides differing in the order, number, or kind of amino acid residues forming these polypeptides. Each of the two different members of a heterodimer may independently comprise one, two, or more units, polypeptide chains, or moieties.

The term "targeting moiety," as used herein, generally refers to a molecule, complex, or aggregate, that binds specifically, selectively, or preferentially to a target molecule, cell, particle, tissue, or aggregate. For example, a targeting moiety may be an antibody, antigen-binding antibody fragment, bispecific antibody, or other antibody-based molecule or compound. Other examples of targeting moieties may include, but are not limited to, aptamers, avimers, receptor-binding ligands, nucleic acids, biotin-avidin binding pairs, binding peptides or proteins, etc. The terms "targeting moiety" and "binding moiety" are used interchangeably herein.

The term "tumor antigen," as used herein, generally refers to an antigenic substance produced in or by tumor cells, which may have the ability to trigger an immune response in a host. For example, a tumor antigen may be a protein, a polypeptide, a peptide, or a fragment thereof, which constitutes part of a tumor cell and is capable of inducing tumor-specific cytotoxic T lymphocytes. A tumor antigen peptide may be a peptide that is generated as a result of degradation of the tumor antigen in a tumor cell and can induce or activate tumor-specific cytotoxic T lymphocytes upon being expressed on the cell surface by binding to an HLA molecule. In some embodiments, the term "tumor antigen" may also refer to biomolecules (e.g., proteins, carbohydrates, glycoproteins, etc.) that are exclusively or preferentially or differentially expressed on a cancer cell and/or are found in association with a cancer cell and thereby provide targets preferential or specific to the cancer. For example, the preferential expression can be preferential expression as compared to any other cell in the organism, or preferential expression within a particular area of the organism (e.g., within a particular organ or tissue).

The terms "tumor antigen epitope" and "tumor antigen determinant" are used interchangeably herein and generally refer to the site of an amino acid sequence present in a tumor antigen that can induce tumor-specific cytotoxic T lymphocytes.

The terms "immunoregulator" and "immunomodulator" are used interchangeably herein, and generally refer to a substance that affects the functioning of the immune system. An immunoregulator may augment or reduce an immune response. For example, an immunoregulator may be an active agent of immunotherapy, including but not limited to, e.g., recombinant, synthetic, and/or natural formulations of cytokines, granulocyte colony-stimulating factors (G-CSF), interferons, imiquimod, and cellular membrane fractions from bacteria, chemokines, interleukins, cytosine phosphate-guanosine (CpG) oligodeoxynucleotides, and glucans. In some examples, the immunoregulator is a cytokine. In some cases, the immunoregulator is not an antibody or an antigen-binding fragment thereof. In some cases, the immunoregulator is not an immunoglobulin molecule or a fragment (such as an antigen-binding fragment) thereof.

In some embodiments, the immunoregulator is selected from the group consisting of an interferon, an interleukin, a chemokine, a lymphokine, and a tumor necrosis factor. For example, the immunoregulator may be selected from the group consisting of interferon alpha, interferon lambda, interferon beta, interleukin 10, interleukin 2, and super interleukin 2.

The term "expression yield," as used in the context of proteinaceous heterodimers herein, generally refers to an amount of a proteinaceous heterodimer being produced in functional form upon expression, e.g., when expressed by a host cell.

The term "dimerization sequence," as used herein, generally refers to an amino acid sequence capable of forming a dimer, or undergoing dimerization. In some embodiments, a dimer is a heterodimer formed by two different members. In some cases, the two different members of a heterodimer may comprise different dimerization sequences.

The term "heterodimerization," as used herein, generally refers to the process of forming a heterodimer between two different members (e.g., two different polypeptides), such as through complexation, association, or aggregation, with or without formation of covalent bonds between the two different members.

The term "covalent bond," as used herein, generally refers to a chemical bond formed between atoms by the sharing of electrons. For example, a covalent bond may be polar or non-polar. In some embodiments, a covalent bond is a disulfide bond.

The term "non-covalent pairwise affinity," as used herein, generally refers to dimerization sequences or heterodimerization sequences capable of binding each other via non-covalent interaction, e.g., via ion pairs, hydrogen bonds, dipole-dipole interactions, charge transfer interactions, π-π interactions, cation-π-electron interactions, van der Waals interactions and disperse interactions, hydrophobic (lipophilic) interactions, complex formation (e.g., complex formation of transition metal cations), or a combination of these interactions.

The term "linker," as used herein, generally refers to a synthetic amino acid sequence that connects or links two polypeptide sequences, e.g., that links two polypeptide domains. A linker may connect two amino acid sequences via peptide bonds. In some embodiments, a linker of the present disclosure connects a biologically active moiety to a second moiety in a linear sequence.

The terms "polypeptide," "peptide," and "protein" are used interchangeably herein to refer to polymers of amino acids of any length. The polymer may be linear or branched, it may comprise modified amino acids, and it may be interrupted by non-amino acids. The terms also encompass an amino acid polymer that has been modified, for example, by disulfide bond formation, glycosylation, lipidation, acetylation, phosphorylation, or any other manipulation, such as conjugation with a labeling component. The terms may apply to amino acid polymers in which one or more amino acid residues are artificial chemical analogs of the corresponding naturally occurring amino acids, as well as to naturally occurring amino acid polymers. The terms may also include variants on the traditional peptide linkage joining the amino acids making up the polypeptide. For example, the "peptides," "polypeptides," and "proteins" may be chains of amino acids whose alpha carbons are linked through peptide bonds. The terminal amino acid at one end of the chain (amino terminal) therefore may have a free amino group, while the terminal amino acid at the other end of the chain (carboxy terminal) may have a free carboxyl group. As used herein, the term "amino terminus" (abbreviated N-terminus) generally refers to the free α-amino group on an amino acid at the amino terminus of a peptide or to the α-amino group (imino group when participating in a peptide bond) of an amino acid at any other location within the peptide. Similarly, the term "carboxy terminus" generally refers to the free carboxyl group on the carboxy terminus of a peptide or the carboxyl group of an amino acid at any other location within the peptide. Peptides may also include essentially any poly-amino acid, including but not limited to, peptide mimetics such as amino acids joined by an ether as opposed to an amide bond.

The term "amino acid," as used herein, generally refers to either natural and/or unnatural or synthetic amino acids, including but not limited to, the D or L optical isomers or both, amino acid analogs and peptidomimetics. Standard single or three letter codes are used to designate amino acids.

The term "natural L-amino acid," as used herein, generally refers to the L optical isomer forms of glycine (G), proline (P), alanine (A), valine (V), leucine (L), isoleucine (I), methionine (M), cysteine (C), phenylalanine (F), tyrosine (Y), tryptophan (W), histidine (H), lysine (K), arginine (R), glutamine (Q), asparagine (N), glutamic acid (E), aspartic acid (D), serine (S), and threonine (T).

The term "non-naturally occurring," as used herein, generally refers to polypeptide or polynucleotide sequences that do not have a counterpart to, are not complementary to, or do not have a high degree of homology with a wild-type or naturally-occurring sequence (e.g., those found in a subject). For example, a non-naturally occurring polypeptide or fragment may share less than 99%, 98%, 95%, 90%, 80%, 70%, 60%, 50%, or even less amino acid sequence identity as compared to a natural sequence when suitably aligned. Alternatively, a non-naturally occurring polypeptide or fragment may share more than 99%, 98%, 95%, 90%, 80%, 70%, 60%, 50%, or even more amino acid sequence identity as compared to a natural sequence when suitably aligned.

The terms "hydrophilic" and "hydrophobic," as used herein, generally refer to the degree of affinity that a substance has with water. A hydrophilic substance has a strong affinity for water, tending to dissolve in, mix with, or be wetted by water, while a hydrophobic substance substantially lacks affinity for water, tending to repel and not absorb water and tending not to dissolve in or mix with or be wetted by water. Amino acids can be characterized based on their hydrophobicity. A number of scales have been developed. An example is a scale developed by Levitt, M, et al., J Mol Biol (1976) 104:59, which is listed in Hopp, TP, et al., Proc Natl Acad Sci U S A (1981) 78:3824. Examples of "hydrophilic amino acids" are arginine, lysine, threonine, alanine, asparagine, and glutamine. Of particular interest are the hydrophilic amino acids aspartate, glutamate, serine, and glycine. Examples of "hydrophobic amino acids" are tryptophan, tyrosine, phenylalanine, methionine, leucine, isoleucine, and valine.

The term "fragment," when used in the context of a proteinaceous molecule (e.g., a polypeptide or a protein), generally refers to a truncated form of a native biologically active protein that may or may not retain a portion of the therapeutic and/or biological activity.

The term "variant," when used in the context of a proteinaceous molecule (e.g., a polypeptide or a protein), generally refers to a proteinaceous molecule with sequence homology to the native biologically active protein that retains at least a portion of the therapeutic and/or biological activity of the biologically active protein. For example, a variant protein may share at least 60%, 65%, 70%, 75%, 80%, 85%, 90%, 95%, 96%, 97%, 98%, or 99% amino acid sequence identity compared with the reference biologically active protein. In some embodiments, the "variant" may include proteins modified deliberately, as for example, by site directed mutagenesis, synthesis of the encoding gene, insertions, or accidentally through mutations.

The terms "conjugated," "linked," "fused," and "fusion" are used interchangeably herein, and generally refer to the joining together of two or more chemical elements, sequences, or components, e.g., by means including chemical conjugation or recombinant means. For example, a promoter or enhancer is operably linked to a coding sequence if it affects the transcription of the sequence. Generally, "operably linked" means that the DNA sequences being linked are contiguous, and in reading phase or in-frame. An "in-frame fusion" refers to the joining of two or more open reading frames (ORFs) to form a continuous longer ORF, in a manner that maintains the correct reading frame of the original ORFs. Thus, the resulting "fusion polypeptide" is a single protein containing two or more fragments that correspond to polypeptides encoded by the original ORFs (which segments are not normally so joined in nature). The "fusion site" refers to the sequence where the two or more fragments are joined together. In some cases, the fusion site can be a sequence that is identical to sequences in the two or more fragments being joined. In some cases, the fusion site can further comprise a gap segment that is not identical to either of the sequences of the two or more fragments being joined.

In the context of polypeptides, a "linear sequence" or a "sequence" is an order of amino acids in a polypeptide in an amino to carboxyl terminus direction in which residues next to each other in the sequence are contiguous in the primary structure of the polypeptide. A "partial sequence" is a linear sequence forming part of a polypeptide that is known to comprise additional residues in one or both directions.

The terms "polynucleotides," "nucleic acids," "nucleotides," and "oligonucleotides" are used interchangeably herein, and they generally refer to a polymeric form of nucleotides of any length, either deoxyribonucleotides or ribonucleotides, or analogs thereof. Polynucleotides may have any three-dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component.

The terms "gene" and "gene fragment" are used interchangeably herein and generally refer to a polynucleotide containing at least one open reading frame that is capable of encoding a particular protein after being transcribed and translated. A gene or gene fragment may be genomic or cDNA, as long as the polynucleotide contains at least one open reading frame, which may cover the entire coding region or a segment thereof. A "fusion gene" is a gene composed of at least two heterologous polynucleotides that are linked together.

The term "antibody," as used herein, generally refers to a protein comprising one or more polypeptides substantially encoded by immunoglobulin genes or fragments of immunoglobulin genes. The immunoglobulin genes may include the kappa, lambda, alpha, gamma, delta, epsilon, and mu constant region genes, as well as myriad immunoglobulin variable region genes. As used herein, light chains may be classified as either kappa or lambda. Heavy chains may be classified as gamma, mu, alpha, delta, or epsilon, which in turn define the immunoglobulin classes, IgG, IgM, IgA, IgD, and IgE, respectively. An antibody as used in the present disclosure may have a structural unit comprising a tetramer. Each tetramer may be composed of two identical pairs of polypeptide chains, each pair having one "light" chain (about 25 KD) and one "heavy" chain (about 50-70 KD). The N-terminus of each chain may define a variable region of about 100 to 110 or more amino acids primarily responsible for antigen recognition. The terms "light chain variable region" (VL) and "heavy chain variable region" (VH), as used herein, generally refer to these regions of the light and heavy chains, respectively. Antibodies may exist as intact immunoglobulins or as a number of well characterized fragments produced by digestion with various peptidases or expressed de novo. Thus, for example, pepsin may digest an antibody below the disulfide linkages in the hinge region to produce F(ab)'2 (a dimer of Fab which itself is a light chain joined to VH-CH1 by a disulfide bond). The F(ab)'2 may be reduced under mild conditions to break the disulfide linkage in the hinge region thereby converting the (Fab')2 dimer into a Fab' monomer. The Fab' monomer is essentially a Fab with part of the hinge region (see, Fundamental Immunology, W. E. Paul, ed., Raven Press, N.Y. (1993), for a more detailed description of other antibody fragments). While various antibody fragments are defined in terms of the digestion of an intact antibody, one of ordinary skill in the art will appreciate that such Fab' fragments may be synthesized de novo either chemically or by utilizing recombinant DNA methodologies. Thus, the term antibody, as used herein, may also include antibody fragments either produced by the modification of whole antibodies or synthesized de novo using recombinant DNA methodologies, including but not limited to, Fab'2, IgG, IgM, IgA, IgE, scFv, dAb, nanobodies, unibodies, and diabodies. In some embodiments, the antibodies include, but are not limited to Fab'2, IgG, IgM, IgA, IgE, and single chain antibodies, for example, single chain Fv (scFv) antibodies in which a variable heavy chain and a variable light chain are joined together (directly or through a peptide linker) to form a continuous polypeptide.

The term "antigen-binding site" or "binding portion," as used herein, generally refers to a part of an antibody that participates in antigen binding. An antigen-binding site may be formed by amino acid residues of the N-terminal variable ("V") regions of a heavy ("H") chain and/or a light ("L") chain. Three highly divergent stretches within the V regions of the heavy and light chains are referred to as "hypervariable regions" which are interposed between more conserved flanking stretches known as "framework regions" or "FRs". Thus, the term "FR," as used herein, generally refers to amino acid sequences that are naturally found between and adjacent to hypervariable regions in immunoglobulins. In an antibody molecule, the three hypervariable regions of a light chain and the three hypervariable regions of a heavy chain are disposed relative to each other in three-dimensional space to form an antigen-binding "surface". This surface may mediate recognition and binding of the target antigen. The three hypervariable regions of each of the heavy and light chains are referred to as "complementarity-determining regions" or "CDRs" and are characterized, for example, by Kabat et al. Sequences of proteins of immunological interest, 4th ed. U.S. Dept. Health and Human Services, Public Health Services, Bethesda, Md. (1987).

In some embodiments, antibodies and fragments thereof used herein can be bispecific. Bispecific antibodies or fragments thereof can be of various configurations. For example, bispecific antibodies may resemble single antibodies (or antibody fragments) but have two different antigen-binding sites (variable regions). In various embodiments, bispecific antibodies can be produced by chemical techniques (Kranz et al. (1981) Proc. Natl. Acad. Sci., USA, 78: 5807), by "polydoma" techniques (see, e.g., U.S. Pat. No. 4,474,893), or by recombinant DNA techniques. In some embodiments, bispecific antibodies as used herein may have binding specificities for at least two different epitopes, at least one of which is a tumor antigen. In some embodiments, the antibodies and fragments thereof may also be heteroantibodies. Heteroantibodies are two or more antibodies, or antibody-binding fragments (e.g., Fab) linked together, each antibody or fragment having a different specificity.

The term "homology," "homologous," or "sequence identity," as used herein, generally refers to sequence similarity or interchangeability between two or more polynucleotide sequences or between two or more polypeptide sequences. When using a program (e.g., Emboss Needle or BestFit) to determine sequence identity, similarity, or homology between two different amino acid sequences, the default settings may be used, or an appropriate scoring matrix, such as blosum45 or blosum80, may be selected to optimize identity, similarity, or homology scores. In some embodiments, polynucleotides that are homologous are those which hybridize under stringent conditions and have at least 60%, at least 65%, at least 70%, at least 80%, at least 90%, at least 95%, at least 97%, at least 98%, at least 99%, and even 100% sequence identity compared to those sequences. Polypeptides that are homologous have sequence identities of at least 80%, or at least 90%, or at least 95%, or at least 97%, or at least 98%, or at least 99% sequence identity when sequences of comparable length are optimally aligned.

The terms "percent identity" and "% identity," as used in the context of polynucleotide sequences, generally refer to the percentage of residue matches between at least two polynucleotide sequences aligned using a standardized algorithm. Such an algorithm may insert, in a standardized and reproducible way, gaps in the sequences being compared in order to optimize alignment between two sequences, and therefore achieve a more meaningful comparison of the two sequences. Percent identity may be measured over the length of an entire defined polynucleotide sequence, or may be measured over a shorter length, for example, over the length of a fragment taken from a larger, defined polynucleotide sequence. It is understood that any fragment length supported by the sequences shown herein, in the tables, figures, or Sequence Listing, may be used to describe a length over which percentage identity may be measured.

The term "percent (%) sequence identity," as used in the context of polypeptide sequences identified herein, generally refers to the percentage of amino acid residues in a query sequence that are identical to the amino acid residues of a second, reference polypeptide sequence or a portion thereof, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, NEEDLE, or Megalign (DNASTAR) software. Those skilled in the art can determine appropriate parameters for measuring alignment, including any algorithms needed to achieve maximal alignment over the full length of the sequences being compared. Percent identity may be measured over the length of an entire defined polypeptide sequence, or may be measured over a shorter length, for example, over the length of a fragment taken from a larger, defined polypeptide sequence. It is understood that any fragment length supported by the sequences shown herein, in the tables, figures, or Sequence Listing, may be used to describe a length over which percentage identity may be measured.

The term "host cell," as used herein, generally includes an individual cell, a cell line, or cell culture, which can be or has been a recipient for the plasmids or vectors of the present disclosure, comprises the polynucleotide of the present disclosure, or expresses the proteinaceous heterodimer (e.g., heterodimer protein) of the present disclosure. Host cells may include progeny of a single host cell. The progeny may not necessarily be completely identical (in morphology or in genomic of total DNA complement) to the original parent cell due to natural, accidental, or deliberate mutation. A host cell may include cells transfected *in vitro* with a vector of the present disclosure. A host cell may be a bacterial cell (e.g., E. coli), a yeast cell, or other eukaryotic cells, e.g., a COS cell, a Chinese hamster ovary (CHO) cell, a HeLa cell, a HEK293 cell, a COS-1 cell, an NS0 cell, or a myeloma cell. In some embodiments, a host cell is a mammalian cell. In some embodiments, the mammalian cell is a HEK293 cell.

The term "vector," as used herein, generally refers to a nucleic acid molecule capable of self-replicating in an appropriate host, which transfers an inserted nucleic acid molecule into and/or between host cells. The term may include vectors that function primarily for insertion of DNA or RNA into a cell, replication of vectors that function primarily for the replication of DNA or RNA, and expression vectors that function for transcription and/or translation of the DNA or RNA. Also included are vectors that provide more than one of the above functions. An "expression vector" is a polynucleotide which, when introduced into an appropriate host cell, can be transcribed and translated into a polypeptide(s). An "expression system" usually connotes a suitable host cell comprising an expression vector that can function to yield a desired expression product.

The term "effective amount" or "therapeutically effective amount" refers to an amount of a composition (e.g., a proteinaceous heterodimer described herein) that is sufficient to effect the intended application, including but not limited to disease treatment. The therapeutically effective amount may vary depending upon the intended application (e.g., *in vitro* or *in vivo),* or the subject and disease condition being treated, e.g., the weight and age of the subject, the severity of the disease condition, the manner of administration and the like, which can be readily determined by one of ordinary skill in the art. The term may also apply to a dose that will induce a particular response in target cells, e.g., target gene induction, proliferation, and/or apoptosis. The specific dose will vary depending on the particular compounds chosen, the dosing regimen to be followed, whether it is administered in combination with other compounds, timing of administration, the tissue to which it is administered, and the physical delivery system in which it is carried.

The terms "treatment" or "treating," "palliating," and "ameliorating" are used interchangeably herein, and refer to an approach for obtaining beneficial or desired results, including but not limited to, a therapeutic benefit and/or a prophylactic benefit. As used herein, a therapeutic benefit generally refers to eradication or reduced severity of the underlying disorder being treated. Also, a therapeutic benefit is achieved with the eradication, reduced severity or reduced incidence of one or more of the physiological symptoms associated with the underlying disorder such that an improvement is observed in the subject, notwithstanding that the subject may still be afflicted with the underlying disorder. For prophylactic benefit, the compositions may be administered to a subject at risk of developing a particular disease, or to a subject reporting one or more of the physiological symptoms of a disease, even though a diagnosis of this disease may not have been made.

The term "therapeutic effect," as used herein, generally encompasses a therapeutic benefit and/or a prophylactic benefit as described above. A prophylactic effect includes delaying or eliminating the appearance of a disease or condition, delaying or eliminating the onset of symptoms of a disease or condition, slowing, halting, or reversing the progression of a disease or condition, or any combination thereof.

The term "co-administration," "administered in combination with," and their grammatical equivalents, as used herein, generally encompass administration of two or more agents to an animal so that both agents and/or their metabolites are present in the subject at the same time. Co-administration includes simultaneous administration in separate compositions, administration at different times in separate compositions, or administration in a composition in which both agents are present.

The terms "antagonist" and "inhibitor" are used interchangeably herein, and they generally refer to a compound having the ability to inhibit a biological function of a target protein, whether by inhibiting the activity or expression of the target protein. Accordingly, the terms "antagonist" and "inhibitor" are defined in the context of the biological role of the target protein. While preferred antagonists herein specifically interact with (e.g., bind to) the target, compounds that inhibit a biological activity of the target protein by interacting with other members of the signal transduction pathway of which the target protein is a member are also specifically included within this definition. A preferred biological activity inhibited by an antagonist is associated with the development, growth, or spread of a tumor.

The term "agonist," as used herein, generally refers to a compound having the ability to initiate or enhance a biological function of a target protein, whether by inhibiting or enhancing the activity or expression of the target protein. Accordingly, the term "agonist" is defined in the context of the biological role of the target polypeptide. While preferred agonists herein specifically interact with (e.g., bind to) the target, compounds that initiate or enhance a biological activity of the target polypeptide by interacting with other members of the signal transduction pathway of which the target polypeptide is a member are also specifically included within this definition.

The term "agent" or "biologically active agent," as used herein, generally refers to a biological, pharmaceutical, or chemical compound or other moieties. Non-limiting examples include a simple or complex organic or inorganic molecule, a peptide, a protein, an oligonucleotide, an antibody, an antibody derivative, an antibody fragment, a vitamin derivative, a carbohydrate, a toxin, or a chemotherapeutic compound. Various compounds can be synthesized, for example, small molecules and oligomers (e.g., oligopeptides and oligonucleotides), and synthetic organic compounds based on various core structures. In addition, various natural sources can provide compounds for screening, such as plant or animal extracts, and the like.

The term "anti-cancer agent," "anti-tumor agent," or "chemotherapeutic agent," as used herein, generally refers to any agent useful in the treatment of a neoplastic condition. One class of anti-cancer agents comprises chemotherapeutic agents.

The term "chemotherapy," as used herein, generally refers to the administration of one or more chemotherapeutic drugs and/or other agents to a cancer patient by various methods, including intravenous, oral, intramuscular, intraperitoneal, intravesical, subcutaneous, transdermal, buccal, or inhalation or in the form of a suppository.

The term "cell proliferation," as used herein, generally refers to a phenomenon by which the cell number has changed as a result of division. For example, cell proliferation may result in an increase in the number of cells. This term also encompasses cell growth by which the cell morphology has changed (e.g., increased in size) consistent with a proliferative signal.

The term *"in vivo,"* as used herein, generally refers to an event that takes place in a subject's body.

The term *"in vitro,"* as used herein, generally refers to an event that takes place outside of a subject's body. For example, an *in vitro* assay encompasses any assay conducted outside of a subject. *In vitro* assays encompass cell-based assays in which dead or living cells are employed. *In vitro* assays also encompass a cell-free assay in which no intact cells are employed.

The term "interferon" (IFN), as used herein, generally refers to a signaling protein made and released by a host cell in response to the presence of pathogens, such as viruses, bacteria, parasites, or tumor cells. There are three major types of interferons, i.e., type I, type II, and type III, wherein type I interferons may include IFN-α and IFN-β, and IFN-α may further comprise IFN-α subtypes, e.g., IFN-α2, IFN-α4, etc. Type I interferons may inhibit virus replication, have anti-parasitic activity, inhibit cell proliferation, stimulate cytotoxic activity of immune cells, be involved in immune regulation, and exhibit anti-tumor effects. Type II and Type III interferons may include IFN-γ, IFN-λ1(IL-29), IFN-λ2(IL-28a), and IFN-λ3(IL-28b). As used herein, the term "interferon" may include full length interferons, or a fragment (e.g., a truncated form) or variant thereof substantially maintaining the biological activity of a corresponding wild-type interferon (e.g., having a biological activity that is at least 80%, at least 90%, at least 95%, at least 98%, at least 99%, or even at least 100% of the biological activity of a corresponding wild-type interferon). An interferon, as used herein, may be from any mammalian species. In some embodiments, the interferon is from a species selected from the group consisting of human, horse, cattle, mouse, pig, rabbit, cat, dog, rat, goat, sheep, and non-human primate.

The term "interleukin," as used herein, generally refers to a secreted protein or a signaling molecule capable of promoting the development and differentiation of T and/or B lymphocytes and/or hematopoietic cells. An interleukin may be synthesized by helper CD4 T lymphocytes, as well as through monocytes, macrophages, and endothelial cells. As used herein, an interleukin (IL) may include IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-34, IL-35, and/or IL-36. As used herein, the term "interleukin" may include full length interleukins, or a fragment (e.g., a truncated form) or variant thereof substantially maintaining the biological activity of a corresponding wild-type interleukin (e.g., having a biological activity that is at least 80%, at least 90%, at least 95%, at least 98%, at least 99%, or even at least 100% of the biological activity of a corresponding wild-type interleukin). An interleukin, as used herein, may be from any mammalian species. In some embodiments, the interleukin is from a species selected from the group consisting of human, horse, cattle, mouse, pig, rabbit, cat, dog, rat, goat, sheep, and non-human primate. In some embodiments, the interleukin can be in a mutated form, for example, with increased or decreased affinity for its receptors. In specific embodiments, the interleukin can be a super IL-2 (also known as sIL2, see Nature 484, 529-533, 26 April 2012), which may be obtained by modifying IL-2 to increase its binding affinity for IL-2Rβ. Mutations in sIL-2 are principally in the core of the cytokine, and molecular dynamics simulations indicated that the evolved mutations stabilized IL-2, reducing the flexibility of a helix in the IL-2Rβ binding site, into an optimized receptor-binding conformation resembling that when bound to CD25. Compared to IL-2, sIL-2 induced superior expansion of cytotoxic T cells, leading to improved anti-tumor responses *in vivo,* and elicited proportionally less expansion of T regulatory cells and reduced pulmonary edema. The term "anti-HER2/neu antibody," as used herein, generally refers to an antibody that specifically or preferentially binds to a HER2/neu receptor. For example, an anti-HER2/neu antibody or anti-HER2 antibody may be Trastuzumab, Pertuzumab, or antigen-binding fragments thereof.

The term "anti-EGFR antibody," as used herein, generally refers to an antibody that specifically or preferentially binds to an EGFR. In some cases, an anti-EGFR antibody may bind to a mutated form of EGFR (e.g., EGFR variant III (also known as EGFRvIII), which is the most common extracellular domain mutation of EGFR, this mutation leads to a deletion of exons 2-7 of the EGFR gene, which is characterized by a truncated extracellular domain with ligand-independent constitutive activity). For example, an anti-EGFR antibody may be Cetuximab, Mab806, or antigen-binding fragments thereof.

The term "subject," as used herein, generally refers to a human or non-human animal, including but not limited to, a cat, dog, horse, pig, cow, sheep, goat, rabbit, mouse, rat, or monkey.

The term "anti-EGFR family antibody," as used herein, generally refers to an antibody that specifically binds to a member of the epidermal growth factor receptor family. For example, it may be an antibody that binds to ErbB-1 (also named as epidermal growth factor receptor (EGFR)), ErbB-2 (also named as HER2 in humans and as neu in rodents), ErbB-3 (also named as HER3), and/or to ErbB-4 (also named as HER4). Examples of anti-EGFR family antibodies include, but are not limited to one or more of the following antibodies: C6.5, C6mL3-9, C6 MH3-B1, C6-B1D2, F5, HER3.A5, HER3.F4, HER3.H1, HER3.H3, HER3.E12, HER3.B12, EGFR.E12, EGFR.C10, EGFR.B11, EGFR.E8, HER4.B4, HER4.G4, HER4.F4, HER4.A8, HER4.B6, HER4.D4, HER4.D7, HER4.D11, HER4.D12, HER4.E3, HER4.E7, HER4.F8, HER4.C7, etc., also see, e.g., U.S. Patent publications US 2006/0099205 A1 and US 2004/0071696 A1, which are incorporated herein by reference.

The term "single chain Fv" ("sFv" or "scFv") polypeptide, as used herein, generally refers to a covalently linked VH (heavy chain variable region):VL (light chain variable region) heterodimer, which may be expressed from a nucleic acid including VH- and VL-encoding sequences either joined directly or joined by a peptide-encoding linker (see Huston, et al. Proc. Nat. Acad. Sci. USA, 85: 5879-5883 (1988)).

The term "inhibition of growth and/or proliferation," when used with cancer cells, generally refers to a decrease in the growth rate and/or proliferation rate of a cancer cell. For example, this may include death of a cancer cell (e.g., via apoptosis). In some embodiments, this term may also refer to inhibiting the growth and/or proliferation of a solid tumor and/or inducing tumor size reduction or elimination of the tumor.

The term "a cancer cell surface marker" or "a cancer cell associated marker," as used herein, generally refers to biomolecules such as proteins, carbohydrates, glycoproteins, and the like that are exclusively or preferentially or differentially expressed on a cancer cell and/or are found to be associated with a cancer cell and thereby provide targets preferential or specific to the cancer. In some embodiments, the preferential expression can be preferential expression as compared to any other cell in the organism, or preferential expression within a particular area of the organism (e.g., within a particular organ or tissue).

The term "member," as used herein, generally refers to a polypeptide, subunit, or moiety which is one component of the proteinaceous heterodimer.

The term "Fc region," as used herein, generally refers to the carboxy terminal portion of an immunoglobulin heavy chain constant region, or an analog or portion thereof capable of binding to an Fc receptor. As is known, each immunoglobulin heavy chain constant region comprises four or five domains. The domains are named sequentially as follows: CH1-hinge-CH2-CH3(-CH4). CH4 is present in IgM, which has no hinge region. The immunoglobulin heavy chain constant region useful in the present disclosure may comprise an immunoglobulin hinge region, and may also comprise a CH3 domain. For example, the immunoglobulin heavy chain constant region may comprise an immunoglobulin hinge region, a CH2 domain, and a CH3 domain. In some embodiments, the Fc region according to the present disclosure consists of the hinge-CH2-CH3 domain.

The term "complexed with," as used herein, generally refers to the association (e.g., binding) of one member/subunit with another member/subunit of a molecule (e.g., an antibody). For example, a light chain may be complexed with a heavy chain to form a targeting moiety.

The term "binding specificity," as used herein, generally refers to the ability to specifically bind to (e.g., immunoreact with) a given target (while not binding to or substantially not binding to a non-target). A targeting moiety of the present disclosure may be monospecific and contain one or more binding sites which specifically bind to a target, or may be multispecific (e.g., bispecific or trispecific) and contain two or more binding sites which specifically bind to the same or different targets.

The term "associates with" or "associated with," as used herein, generally refers to that one entity is in physical association or contact with another. For example, a first member of the proteinaceous heterodimer may "associate with" a second member covalently or non-covalently. In some embodiments, a first member of the proteinaceous heterodimer associates with a second member via an interface, and the interface is formed by amino acid residues (i.e., interface residues) from the first member and the second member, respectively.

The term "modification" as used herein, generally refers to any manipulation of the peptide backbone (e.g., amino acid sequence) or any post-translational modifications (e.g., glycosylation) of a polypeptide. For example, a modification is in comparison to the sequence of a corresponding wild-type polypeptide. A modification may be a substitution, an addition, and/or a deletion of one or more amino acids (e.g., 1, 2, 3, 4, 5, 6, 7, 8, 9, 10, or more).

The term "knob-and-hole modification," as used herein, generally refers to introducing a modification at the interface of a polypeptide to form a bulge (knob modification) and introducing a modification at a corresponding position of another polypeptide to form a cavity (hole-modification), and the size of the bulge is the same or similar to that of the cavity. For example, the knob-and-hole modification enables the formation of a heterodimer, while inhibiting the formation of a homodimer. See the reference of U.S. Pat. No. 5,731,168; U.S. Pat. No. 7,695,936; Ridgway et al., Prot Eng 9, 617-621 (1996) and Carter, J Immunol Meth 248, 7-15 (2001). Accordingly, the term "knob modification," as used herein, generally refers to a modification at the interface of a polypeptide to replace an amino acid having a smaller side chain (e.g., alanine or threonine) with an amino acid having a larger side chain (e.g., tyrosine or tryptophan) to form a bulge. The term "hole modification," as used herein, generally refers to a modification at a corresponding position of another polypeptide to replace an amino acid having a larger side chain (e.g., tyrosine or tryptophan) with an amino acid having a smaller side chain (e.g., alanine or threonine) to form a cavity. The knob modification and the hole modification can be made by altering the nucleic acid encoding the polypeptides, e.g., by site-specific mutagenesis, or by peptide synthesis. In a specific embodiment, the knob modification comprises the amino acid substitutions Y349C and T366W in one of the two subunits of the Fc region, and the hole modification comprises the amino acid substitutions D356C, T366S, L368A, and Y407V in the other one of the two subunits of the Fc region.

The term "HEK293 cell," as used herein, generally refers to clonal isolates derived from transformed human embryonal kidney (HEK) cells. The HEK293 strain is a variant of the 293 cell line that demonstrates better adherence in monolayer culture and ease of use for plaque assays and other anchorage-dependent applications. They have been adapted to suspension culture in serum-free media, e.g., 293 SFM II.

The term "CHO cell," as used herein, generally refers to Chinese hamster ovary cells, which are non-secretory, immortal fibroblasts. The CHO cells rarely secrete CHO endogenous protein, which is thus favorable to the separation and purification for a target protein.

The term "COS-1 cell," as used herein, generally refers to fibroblast-like cell lines derived from monkey kidney tissue. COS cells are obtained by immortalizing CV-1 cells with a version of the SV40 virus that can produce large T antigen but has a defect in genomic replication. One form of COS cell lines commonly used is COS-1.

The term "NS0 cell," as used herein, generally refers to a model cell line derived from the non-secreting murine myeloma. The cell line is a cholesterol-dependent cell line that was generated from a subline of NSI/1.

The term "fusion protein," as used herein, generally refers to a polypeptide that comprises, or consists of, an amino acid sequence of a polypeptide fused directly or indirectly (e.g., via a linker) to an amino acid sequence of a heterologous polypeptide (i.e., a polypeptide unrelated to the former polypeptide or the domain thereof).

The term "C-terminus," as used herein, generally refers to the carboxy terminus of a polypeptide. The term "N-terminus," as used herein, generally refers to the amino terminus of a polypeptide. The term "EGFR," as used herein, generally refers to epidermal growth factor receptor, for example, see Carpenter et al. Ann. Rev. Biochem. 56:881-914 (1987), including naturally occurring mutant forms thereof.

The term "EGFR mutant," as used herein, generally refers to a mutated form of EGFR (e.g., EGFR variant III (also known as EGFRvIII), which is the most common extracellular domain mutation of EGFR, this mutation leads to a deletion of exons 2-7 of the EGFR gene, which is characterized by a truncated extracellular domain with ligand-independent constitutive activity).

The term "HER2/neu," as used herein, generally refers to a human HER2 protein, for example, in Semba et al., PNAS (USA) 82:6497-6501 (1985) and Yamamoto et al. Nature 319:230-234 (1986) (GenBank accession number X03363).

The term "GPC3," as used herein, generally refers to a protein encoded by the gene glypican 3 (NCBI database Gene ID: 2719), which is an early marker of liver cancer. GPC3 is highly expressed in hepatocellular carcinoma and is detected in the tissues of patients with early hepatocellular carcinoma. The term "anti-GPC3 antibody," as used herein, generally refers to an antibody that specifically or preferentially binds to GPC3. For example, an anti-GPC3 antibody may be codrituzumab or antigen-binding fragments thereof.

The term "FAP," as used herein, generally refers to fibroblast activation protein (FAP). FAP exists in tumor matrix fibroblasts and plays a role in the cell surface. It is a membrane serine peptidase, which is a member of the type II serine protease family and has dipeptidyl peptidase and collagenase activity. The term "anti-FAP antibody," as used herein, generally refers to an antibody that specifically or preferentially binds to FAP. For example, an anti-FAP antibody may be antibody 28H1 or antigen-binding fragments thereof.

The term "Muc1," as used herein, generally refers to a glycoprotein encoded by the muc1 gene. Muc1 is mainly present in the epithelial tissues and organs of the mammary gland, pancreas, ovary, etc. It is highly expressed on the surface of cancer epithelial cells, and accordingly becomes the target of immune response.

The term "anti-Muc1 antibody," as used herein, generally refers to an antibody that specifically or preferentially binds to Muc1. For example, an anti-Muc1 antibody may be antibody 5E5, a humanized version of the antibody 5E5, or antigen-binding fragments thereof.

The term "MUC5AC," as used herein, generally refers to the mucin MUC5AC. MUC5AC is highly expressed in colorectal cancer, gastric signet ring cell carcinoma, colon cancer, rectal cancer, and pancreatic cancer.

The term "anti-MUC5AC antibody," as used herein, generally refers to an antibody that specifically or preferentially binds to MUC5AC. For example, an anti-MUC5AC antibody may be antibody ensituximab or antigen-binding fragments thereof.

The term "Mesothelin," as used herein, generally refers to a cell surface glycoprotein with a molecular weight of 40 KD. Mesothelin is highly expressed in a variety of tumor tissues, such as early pancreatic tumors, and it can be expressed in normal pleura, pericardium, and peritoneal mesothelial cells. The term "anti-Mesothelin antibody," as used herein, generally refers to an antibody that specifically or preferentially binds to Mesothelin. For example, an anti-Mesothelin antibody may be antibody amatuximab, a humanized version of the antibody amatuximab, or antigen-binding fragments thereof. The term "chemokine," as used herein, generally refers to some low molecular weight (mostly 8-10 KD) proteins capable of attracting white blood cells to the site of infection. For example, the common structural features of chemokine proteins may include a small molecular weight and four cysteine residues at the conserved positions ensuring the tertiary structure. Some chemokines are involved in promoting inflammatory responses, and some are involved in controlling cell migration during normal process of repair or development.

The term "lymphokine," as used herein, generally refers to a hormone-like polypeptide produced by activated lymphocytes, which can act on the corresponding target cells, causing changes in the characteristics or functions of the target cells. The lymphocytes act on adjacent or distant target cells via lymphokines to achieve immunomodulatory and immune effects. Common lymphokines include, but are not limited to, monocyte-macrophage migration inhibitory factor (MIF), leukocyte motility inhibitory factor (LIF), natural killer cell cytotoxin (NKCF), and lymphotoxin (LB).

The term "tumor necrosis factor," as used herein, generally refers to tumor necrosis factors produced by activated macrophages, NK cells, and T lymphocytes. Among them, TNF produced by macrophages is called TNF-α, and T lymphocytes produced by lymphotoxin (LT) are named TNF-β. The term "immunoglobulin," as used herein, generally refers to a protein consisting of one or more polypeptides substantially encoded by immunoglobulin genes. The recognized immunoglobulin genes include the kappa, lambda, alpha, gamma (IgG1, IgG2, IgG3, IgG4), delta, epsilon, and mu constant region genes, as well as myriad immunoglobulin variable region genes. One form of immunoglobulin constitutes the basic structural unit of an antibody. This form is a tetramer and consists of two identical pairs of immunoglobulin chains, each pair having one light chain and one heavy chain. In each pair, the light and heavy chain variable regions are together responsible for binding to an antigen, and the constant regions are responsible for the antibody effector functions. In addition to antibodies, immunoglobulins may exist in a variety of other forms including, for example, Fv, Fab, Fab', and (Fab')2.

The term "fused in frame," as used herein, generally refers to the joining of two or more open reading frames (ORFs) to form a continuous longer ORF, in a manner that maintains the correct reading frame of the original ORFs.

The term "linker," as used herein, generally refers to a synthetic amino acid sequence that connects or links two polypeptide sequences, e.g., that links two polypeptide domains. A linker may connect two amino acid sequences via peptide bonds. In some embodiments, a linker of the present disclosure connects an immunoregulator to the second Fc region in a linear sequence.

The term "located N-terminal to," as used herein, generally refers to locating at a position N-terminal to another molecule (e.g., another polypeptide). For example, according to the present disclosure, two or more immunoregulators may be located N-terminal to the second Fc region.

The term "amino acid substitution," as used herein, generally refers to that one amino acid at a specific position of a polypeptide is replaced by another amino acid.

The term "EU index of the KABAT number," as used herein, generally refers to the index of the EU number corresponding to the amino acid sequence according to Kabat et al. (1971) Ann. NY Acad, Sci. 190:382-391 and Kabat, E. A., et al. (1991) Sequences of proteins of immunological interest, Fifth Edition, U.S. Department of Health and Human Services, NIH Publication No. 91-3242.

The term "isolated polynucleotide," as used herein, generally refers to a polymeric form of nucleotides of any length, either deoxyribonucleotides or ribonucleotides, or analogs thereof, isolated from its native environment, or that is artificially synthesized.

The term "protein mixture," as used herein, generally refers to a mixture of two or more types of proteins.

The term "homodimer," as used herein, generally refers to a molecule formed by two identical monomers (e.g., two identical members or subunits). The two monomers may aggregate, complex, or associate with each other via covalent and/or non-covalent interactions. For example, the two monomers of a proteinaceous homodimer may associate with each other via interactions between interface amino acid residues from each of the two monomers.

The term "malignant effusion," as used herein, generally refers to effusions resulting from malignant tumors.

The term "malignant cell," as used herein, generally refers to cells proliferating malignantly. The malignant cell may comprise a tumor cell. The malignant cell may metastasize from the primary tissue to other parts of the body through the circulatory system or lymphatic system.

The term "malignant pleural effusion (MPE)," as used herein, generally refers to fluid that accumulates in the pleural space. For example, MPE may include effusions due to pleural metastases caused by direct invasion or hematogenous spread of tumor cells. MPE may further include inflammatory pleural effusions caused by a tumor combined with heart failure, tuberculosis, etc. Malignant pleural effusion is common in lung cancer, breast cancer, lymphoma, leukemia, etc., and is also found in ovarian cancer, gastrointestinal tumors, pleural mesothelioma, etc. Malignant pleural effusion can cause severe discomfort symptoms such as dyspnea, dysphagia, chest pain, cough, and fever, and in severe cases, cause respiratory and circulatory dysfunction, leading to death. Malignant pleural effusion can be considered a special tumor microenvironment and contains a large number of lymphocytes, macrophages, fibroblasts, and the like in addition to tumor cells. Symptomatic MPE can generally be treated by drainage, and ambulatory pleural catheter (Pleur-XTM) has been shown as a viable alternative therapy to the standard chest tube thoracostomy for this use.

The term "malignant ascites," as used herein, generally refers to symptoms caused by abdominal and peritoneal metastases of malignant tumors; and/or hepatic portal hypertension (such as). The malignant ascites may include malignant ascites caused by malignant tumors. For example, the malignant ascites may include transudative ascites caused by tumor cells blocking lymph vessels, vascular thrombosis, and inhibition of lymph and blood flow; ascites caused by increased vascular permeability and excessive water and other substances entering the abdominal cavity due to metastasis of tumor cells to the peritoneum, impaired vascular endothelial cells or even malignant changes; and/or ascites caused by nutritional consumption, cachexia and decreased plasma colloid osmotic pressure in patients. The malignant ascites may be treated with diuretics, chemotherapies (including systemic chemotherapy and intraperitoneal chemotherapy), targeted drug therapy and/or surgery combined with hyperthermic intraperitoneal perfusion (HIPEC).

The term "substantially comprises no," as used herein, generally refers that a composition (e.g., a mixture) comprises little or almost none of a substance. For example, the substance is present with a percentage of, e.g., less than 10%, less than 9%, less than 8%, less than 7%, less than 6%, less than 5%, less than 4%, less than 3%, less than 2%, less than 1%, less than 0.5%, less than 0.1%, or less than 0.01%.

The term "pharmaceutically acceptable excipient," as used herein, generally refers to any and all solvents, dispersion media, coatings, isotonic, absorption delaying agents, etc., that are compatible with pharmaceutical administration.

### Proteinaceous Heterodimers, Protein Mixtures, Isolated Polynucleotides, Vectors, and Host Cells

The proteinaceous heterodimer described herein may comprise a first member and a second member different from the first member. The first member may comprise a light chain and a heavy chain comprising a first Fc region, and the light chain may be complexed with the heavy chain to form a targeting moiety exhibiting binding specificity to a tumor antigen. The second member may comprise a polypeptide comprising an immunoregulator fused to a second Fc region. The first member may associate with the second member to form the heterodimer through complexation of the first Fc region with the second Fc region.

In some cases, the proteinaceous heterodimer of the present disclosure may be a proteinaceous complex. The complex may comprise at least three polypeptide chains, e.g., a first polypeptide chain, a second polypeptide chain, and a third polypeptide chain. In some embodiments, the complex consists of, or consists essentially of, three polypeptide chains. For example, the first polypeptide chain may comprise a heavy chain of an antibody specifically directed to a tumor antigen, and the second polypeptide chain may comprise a light chain of the antibody specifically directed to the tumor antigen. The heavy chain may comprise the first Fc region. The heavy chain (i.e., of the first polypeptide chain) and the light chain (i.e., of the second polypeptide chain) may be complexed to form the first member of the proteinaceous heterodimer. The third polypeptide chain may comprise (e.g., from N-terminus to C-terminus) one or more immunoregulators fused to the second Fc region, optionally via one or more linkers. The third polypeptide chain may be the second member of the proteinaceous heterodimer. The first member may associate with the second member to form the heterodimer through complexation of the first Fc region with the second Fc region.

For example, the proteinaceous heterodimer of the present disclosure may be a proteinaceous complex, and the complex may comprise (1) a heavy chain and a light chain of an antibody specific for a tumor antigen; and (2) a fusion protein comprising, from N-terminus to C-terminus, one or more immunoregulators fused to an antibody Fc region, optionally via one or more linkers.

The first Fc region may comprise a first modification and/or the second Fc region may comprise a second modification, wherein the first modification and/or the second modification may more effectively promote heterodimerization between the first member and the second member than a knob-and-hole modification comprising a knob modification and a hole modification. For example, the first modification may be in the CH3 domain of the first Fc region, and the second modification may be in the CH3 domain of the second Fc region. For example, the first modification and/or the second modification is in comparison to the sequence of its corresponding wild-type Fc region, respectively.

In some embodiments, the first Fc region comprises the first modification while the second Fc region does not comprise any modification, and the first modification more effectively promotes heterodimerization between the first member and the second member than the knob-and-hole modification comprising a knob modification and a hole modification.

In some embodiments, the second Fc region comprises the second modification while the first Fc region does not comprise any modification, and the second modification more effectively promotes heterodimerization between the first member and the second member than the knob-and-hole modification comprising a knob modification and a hole modification.

In some embodiments, the first Fc region comprises the first modification, and the second Fc region comprises the second modification, wherein the first modification and the second modification more effectively promote heterodimerization between the first member and the second member than the knob-and-hole modification comprising a knob modification and a hole modification.

In some embodiments, the first modification is different from the knob modification or the hole modification, and/or the second modification is different from the knob modification or the hole modification. For example, the first modification may be different from the knob modification or the hole modification, while the second modification is the same as the hole modification. In some cases, the first modification is the same as the knob modification, while the second modification is different from the knob modification or the hole modification. In some embodiments, the first Fc region comprises the first modification, the second Fc region comprises the second modification, and both the first modification and the second modification are the same as the knob modification or the hole modification.

In some embodiments, the first modification comprises an amino acid substitution at position T366, and an amino acid substitution at one or more positions selected from the group consisting of: Y349, F405, K409, D399, K360, Q347, K392, and S354, wherein the position of the amino acid is determined according to the EU index of the KABAT number.

In some embodiments, the first modification comprises an amino acid substitution selected from the group consisting of Y349C, Y349D, D399S, F405K, K360E, K409A, K409E, Q347E, Q347R, S354D, K392D, and T366W, wherein the position of the amino acid is determined according to the EU index of the KABAT number.

In some embodiments, the first modification comprises 2-5 amino acid substitutions.

In some embodiments, the first modification comprises an amino acid substitution at a group of positions selected from any of the following groups: 1) Y349 and T366; 2) Y349, T366, and F405; 3) Y349, T366, and K409; 4) Y349, T366, F405, K360, and Q347; 5) Y349, T366, F405, and Q347; 6) Y349, T366, K409, K360, and Q347; 7) Y349, T366, K409, and Q347; 8) T366, K409, and K392; 9) T366 and K409; 10) T366, K409, Y349, and S354; 11) T366 and F405; 12) T366, F405, and D399; and 13) T366, F405, Y349, and S354, wherein the position of the amino acid is determined according to the EU index of the KABAT number.

In some embodiments, the first modification comprises a group of amino acid substitutions selected from any of the following groups: 1) Y349C and T366W; 2) Y349C, T366W, and F405K; 3) Y349C, T366W, and K409E; 4) Y349C, T366W, and K409A; 5) Y349C, T366W, F405K, K360E, and Q347E; 6) Y349C, T366W, F405K, and Q347R; 7) Y349C, T366W, K409A, K360E, and Q347E; 8) Y349C, T366W, K409A, and Q347R; 9) T366W, K409A, and K392D; 10) T366W and K409A; 11) T366W, K409A, and Y349D; 12) T366W, K409A, Y349D, and S354D; 13) T366W and F405K; 14) T366W, F405K, and D399S; 15) T366W, F405K, and Y349D; and 16) T366W, F405K, Y349D, and S354D, wherein the position of the amino acid is determined according to the EU index of the KABAT number. In some embodiments, the second modification comprises amino acid substitutions at positions T366, L368, and Y407, as well as an amino acid substitution at one or more positions selected from the group consisting of D356, D399, E357, F405, K360, K392, K409, and Q347, wherein the position of the amino acid is determined according to the EU index of the KABAT number.

In some embodiments, the amino acid substitution comprised by the second modification is selected from the group consisting of D356C, D399S, E357A, F405K, K360E, K392D, K409A, L368A, L368G, Q347E, Q347R, T366S, Y407A, and Y407V, wherein the position of the amino acid is determined according to the EU index of the KABAT number.

In some embodiments, the second modification comprises amino acid substitutions at 4-6 positions. In some embodiments, the second modification comprises an amino acid substitution at a group of positions selected from any of the following groups: 1) D356, T366, L368, Y407, and F405; 2) D356, T366, L368, and Y407; 3) D356, T366, L368, Y407, and Q347; 4) D356, T366, L368, Y407, K360, and Q347; 5) D356, T366, L368, Y407, F405, and Q347; 6) D356, T366, L368, Y407, F405, K360, and Q347; 7) T366, L368, Y407, D399, and F405; 8) T366, L368, Y407, and F405; 9) T366, L368, Y407, F405, and E357; 10) T366, L368, Y407, and K409; 11) T366, L368, Y407, K409, and K392; and 12) T366, L368, Y407, K409, and E357, wherein the position of the amino acid is determined according to the EU index of the KABAT number.

In some embodiments, the second modification comprises a group of amino acid substitutions selected from any of the following groups: 1) D356C, T366S, L368A, Y407V, and F405K; 2) D356C, T366S, L368A, and Y407V; 3) D356C, T366S, L368A, Y407V, and Q347R; 4) D356C, T366S, L368A, Y407V, K360E, and Q347E; 5) D356C, T366S, L368A, Y407V, F405K, and Q347R; 6) D356C, T366S, L368A, Y407V, F405K, K360E, and Q347E; 7) T366S, L368A, Y407V, D399S, and F405K; 8) T366S, L368G, Y407A, and F405K; 9) T366S, L368A, Y407V, F405K, and E357A; 10) T366S, L368A, Y407V, and K409A; 11) T366S, L368A, Y407V, K409A, and K392D; 12) T366S, L368G, Y407A, and K409A; and 13) T366S, L368A, Y407V, K409A, and E357A, wherein the position of the amino acid is determined according to the EU index of the KABAT number.

In some embodiments, the first Fc region comprises the first modification, the second Fc region comprises the second modification, and the first modification and the second modification comprise an amino acid substitution at a group of positions selected from any of the following groups: 1) the first modification: Y349 and T366; and the second modification: D356, T366, L368, Y407, and F405; 2) the first modification: Y349, T366, and F405; and the second modification: D356, T366, L368, and Y407; 3) the first modification: Y349, T366, and K409; and the second modification: D356, T366, L368, Y407, and F405; 4) the first modification: Y349, T366, F405, K360, and Q347; and the second modification: D356, T366, L368, Y407, and Q347; 5) the first modification: Y349, T366, F405, and Q347; and the second modification: D356, T366, L368, Y407, K360, and Q347; 6) the first modification: Y349, T366, K409, K360, and Q347; and the second modification: D356, T366, L368, Y407, F405, and Q347; 7) the first modification: Y349, T366, K409, and Q347; and the second modification: D356, T366, L368, Y407, F405, K360, and Q347; 8) the first modification: T366, K409, and K392; and the second modification: T366, L368, Y407, D399, and F405; 9) the first modification: T366 and K409; and the second modification: T366, L368, Y407, and F405; 10) the first modification: T366, K409, and Y349; and the second modification: T366, L368, Y407, F405, and E357; 11) the first modification: T366, K409, Y349, and S354; and the second modification: T366, L368, Y407, F405, and E357; 12) the first modification: T366 and F405; and the second modification: T366, L368, Y407, and K409; 13) the first modification: T366, F405, and D399; and the second modification: T366, L368, Y407, K409, and K392; 14) the first modification: T366, F405, and Y349; and the second modification: T366, L368, Y407, K409, and E357; and 15) the first modification: T366, F405, Y349, and S354; and the second modification: T366, L368, Y407, K409, and E357; wherein the position of the amino acid is determined according to the EU index of the KABAT number.

In some embodiments, the first Fc region comprises the first modification, and the second Fc region comprises the second modification, wherein the first modification and the second modification comprise a group of amino acid substitutions selected from any of the following groups: 1) the first modification: Y349C and T366W; and the second modification: D356C, T366S, L368A, Y407V, and F405K; 2) the first modification: Y349C, T366W, and F405K; and the second modification: D356C, T366S, L368A, and Y407V; 3) the first modification: Y349C, T366W, and K409E; and the second modification: D356C, T366S, L368A, Y407V, and F405K; 4) the first modification: Y349C, T366W, and K409A; and the second modification: D356C, T366S, L368A, Y407V, and F405K; 5) the first modification: Y349C, T366W, F405K, K360E, and Q347E; and the second modification: D356C, T366S, L368A, Y407V, and Q347R; 6) the first modification: Y349C, T366W, F405K, and Q347R; and the second modification: D356C, T366S, L368A, Y407V, K360E, and Q347E; 7) the first modification: Y349C, T366W, K409A, K360E, and Q347E; and the second modification: D356C, T366S, L368A, Y407V, F405K, and Q347R; 8) the first modification: Y349C, T366W, K409A, and Q347R; and the second modification: D356C, T366S, L368A, Y407V, F405K, K360E, and Q347E; 9) the first modification: T366W, K409A, and K392D; and the second modification: T366S, L368A, Y407V, D399S, and F405K; 10) the first modification: T366W and K409A; and the second modification: T366S, L368G, Y407A, and F405K; 11) the first modification: T366W, K409A, and Y349D; and the second modification: T366S, L368A, Y407V, F405K, and E357A; 12) the first modification: T366W, K409A, Y349D, and S354D; and the second modification: T366S, L368A, Y407V, F405K, and E357A; 13) the first modification: T366W and F405K; and the second modification: T366S, L368A, Y407V, and K409A; 14) the first modification: T366W, F405K, and D399S; and the second modification: T366S, L368A, Y407V, K409A, and K392D; 15) the first modification: T366W and F405K; and the second modification: T366S, L368G, Y407A, and K409A; 16) the first modification: T366W, F405K, and Y349D; and the second modification: T366S, L368A, Y407V, K409A, and E357A; and 17) the first modification: T366W, F405K, Y349D, and S354D; and the second modification: T366S, L368A, Y407V, K409A, and E357A; wherein the position of the amino acid is determined according to the EU index of the KABAT number.

In some embodiments, the first Fc region comprises the first modification, the second Fc region comprises the second modification, the first modification comprises the amino acid substitutions T366W and K409A, and the second modification comprises the amino acid substitutions T366S, L368G, Y407A, and F405K, wherein the position of the amino acid is determined according to the EU index of the KABAT number.

In some embodiments, the knob-and-hole modification comprises a knob modification and a hole modification, wherein the knob modification comprises the amino acid substitutions Y349C and T366W, and the hole modification comprises the amino acid substitutions D356C, T366S, L368A, and Y407V, wherein the position of the amino acid is determined according to the EU index of the KABAT number.

In some embodiments, when expressed in a mammalian cell, a yield of the proteinaceous heterodimer of the present disclosure is at least 10% (e.g., at least 15%, at least 20%, at least 25%, at least 30%, at least 35%, at least 40%, at least 45%, at least 50%, at least 55%, at least 60%, at least 65%, at least 70%, at least 75%, at least 80%, at least 85% or more) higher than that of a reference protein. The reference protein differs from the proteinaceous heterodimer in that the reference protein: i) comprises the knob modification in the first Fc region, ii) comprises the hole modification in the second Fc region, and iii) does not comprise the first modification and the second modification simultaneously. The mammalian cell may be selected from the group consisting of a HEK293 cell, a CHO cell, a COS-1 cell, and an NS0 cell. In some embodiments, the knob modification comprises the amino acid substitutions Y349C and T366W, and the hole modification comprises the amino acid substitutions D356C, T366S, L368A, and Y407V, wherein the position of the amino acid is determined according to the EU index of the KABAT number.

In some embodiments, the polypeptide comprised in the second member is a fusion protein, and a C-terminus of the immunoregulator is directly or indirectly fused to an N-terminus of the second Fc region to form the fusion protein. In some embodiments, the C-terminus of the immunoregulator is indirectly fused to the N-terminus of the second Fc region. For example, the second Fc region may be fused in frame to the immunoregulator via a linker. The linker may be a synthetic amino acid sequence that connects or links two polypeptide sequences, e.g., via peptide bonds. In some embodiments, the linker is a peptide comprising 1, 2, 3, 4, 5, 6, 7, 8, 9, 10, 11, 12, 13, 14, 15, 16, 17, 18, 19, 20, 21, 22, 23, 24, 25, or more amino acids. For example, the linker may comprise 1-10 amino acids (e.g., 1, 2, 3, 4, 5, 6, 7, 8, 9, 10, or more amino acids), 1-15 amino acids (e.g., 1-11, 12, 13, 14, or 15 amino acids), 1-20 amino acids, 1-30 amino acids or more. In some embodiments, the linker comprises an amino acid sequence as set forth in SEQ ID NO: 79 or 88. In some embodiments, the linker is resistant to proteolysis or substantially resistant to proteolysis.

In some embodiments, the tumor antigen is selected from the group consisting of EGFR, an EGFR mutant, HER2/neu, GPC3, FAP, Muc1, MUC5AC, and Mesothelin.

The light chain of the targeting moiety may contain CDRs comprising amino acid sequences that are at least 60%, 70%, 75%, 80%, 85%, 90%, 95%, 96%, 97%, 98%, 99%, or 100% identical to those comprised in the corresponding CDRs of a light chain of an antibody specifically directed to a tumor antigen. In some embodiments, the light chain of the targeting moiety contains a variable region comprising an amino acid sequence that is at least 60%, 70%, 75%, 80%, 85%, 90%, 95%, 96%, 97%, 98%, 99%, or 100% identical to that comprised in the corresponding variable region of a light chain of an antibody specifically directed to a tumor antigen. In some embodiments, the light chain of the targeting moiety contains an amino acid sequence that is at least 60%, 70%, 75%, 80%, 85%, 90%, 95%, 96%, 97%, 98%, 99%, or 100% identical to that comprised in the corresponding amino acid sequence of a light chain of an antibody specifically directed to a tumor antigen.

The heavy chain of the targeting moiety may contain CDRs comprising amino acid sequences that are at least 60%, 70%, 75%, 80%, 85%, 90%, 95%, 96%, 97%, 98%, 99%, or 100% identical to those comprised in the corresponding CDRs of a heavy chain of an antibody specifically directed to a tumor antigen. In some embodiments, the heavy chain of the targeting moiety contains a variable region comprising an amino acid sequence that is at least 60%, 70%, 75%, 80%, 85%, 90%, 95%, 96%, 97%, 98%, 99%, or 100% identical to that comprised in the corresponding variable region of a heavy chain of an antibody specifically directed to a tumor antigen. In some embodiments, the heavy chain of the targeting moiety contains an amino acid sequence that is at least 60%, 70%, 75%, 80%, 85%, 90%, 95%, 96%, 97%, 98%, 99%, or 100% identical to that comprised in the corresponding amino acid sequence of a heavy chain of an antibody specifically directed to a tumor antigen.

In some embodiments, the light chain of the targeting moiety contains CDRs comprising amino acid sequences that are at least 60%, 70%, 75%, 80%, 85%, 90%, 95%, 96%, 97%, 98%, 99%, or 100% identical to those comprised in the corresponding CDRs of a light chain of an antibody specifically directed to a tumor antigen; and the heavy chain of the targeting moiety contains CDRs comprising amino acid sequences that are at least 60%, 70%, 75%, 80%, 85%, 90%, 95%, 96%, 97%, 98%, 99%, or 100% identical to those comprised in the corresponding CDRs of a heavy chain of an antibody specifically directed to a tumor antigen.

In some embodiments, the light chain of the targeting moiety contains a variable region comprising an amino acid sequence that is at least 60%, 70%, 75%, 80%, 85%, 90%, 95%, 96%, 97%, 98%, 99%, or 100% identical to that comprised in the corresponding variable region of a light chain of an antibody specifically directed to a tumor antigen; and the heavy chain of the targeting moiety contains a variable region comprising an amino acid sequence that is at least 60%, 70%, 75%, 80%, 85%, 90%, 95%, 96%, 97%, 98%, 99% or 100% identical to that comprised in the corresponding variable region of a heavy chain of an antibody specifically directed to a tumor antigen.

In some embodiments, the light chain of the targeting moiety contains an amino acid sequence comprising an amino acid sequence that is at least 60%, 70%, 75%, 80%, 85%, 90%, 95%, 96%, 97%, 98%, 99%, or 100% identical to that comprised in the corresponding amino acid sequence of a light chain of an antibody specifically directed to a tumor antigen; and the heavy chain of the targeting moiety contains an amino acid sequence comprising an amino acid sequence that is at least 60%, 70%, 75%, 80%, 85%, 90%, 95%, 96%, 97%, 98%, 99%, or 100% identical to that comprised in the corresponding amino acid sequence of a heavy chain of an antibody specifically directed to a tumor antigen.

The antibody specifically directed to a tumor antigen may be selected from the group consisting of anti-EGFR, anti-EGFR mutant, anti-HER2/neu, anti-GPC3, anti-FAP, anti-Muc1, anti-MUC5AC, and anti-Mesothelin. In some embodiments, an anti-EGFR antibody is Cetuximab. In some embodiments, an anti-EGFR mutant antibody is an anti-EGFR variant III antibody, such as Mab806. In some embodiments, an anti-HER2/neu antibody is Trastuzumab or Pertuzumab. In some embodiments, an anti-GPC3 antibody is antibody codrituzumab. In some embodiments, an anti-FAP antibody is antibody 28H1. In some embodiments, an anti-Muc1 antibody is antibody 5E5 or a humanized version of the antibody 5E5. In some embodiments, an anti-MUC5AC antibody is antibody ensituximab. In some embodiments, an anti-Mesothelin antibody is antibody amatuximab or a humanized version of the antibody amatuximab.

In some embodiments, the targeting moiety specifically binds to EGFR, the light chain of the first member comprises CDR1-3, the amino acid sequence of the CDR1 is as set forth in SEQ ID NO: 101, the amino acid sequence of the CDR2 is as set forth in SEQ ID NO: 102, and the amino acid sequence of the CDR3 is as set forth in SEQ ID NO: 103.

In some embodiments, the targeting moiety specifically binds to EGFR, the light chain of the first member comprises a light chain variable region, and the amino acid sequence of the light chain variable region is as set forth in SEQ ID NO: 104.

In some embodiments, the targeting moiety specifically binds to EGFR, and the amino acid sequence of the light chain of the first member is as set forth in SEQ ID NO: 37.

In some embodiments, the targeting moiety specifically binds to EGFR, the heavy chain of the first member comprises CDR1-3, the amino acid sequence of the CDR1 is as set forth in SEQ ID NO: 105, the amino acid sequence of the CDR2 is as set forth in SEQ ID NO: 106, and the amino acid sequence of the CDR3 is as set forth in SEQ ID NO: 107.

In some embodiments, the targeting moiety specifically binds to EGFR, the heavy chain of the first member comprises a heavy chain variable region, and the amino acid sequence of the heavy chain variable region is as set forth in SEQ ID NO: 108.

In some embodiments, the targeting moiety specifically binds to EGFR, and the amino acid sequence of the heavy chain of the first member is as set forth in SEQ ID NO: 39.

In some embodiments, the targeting moiety specifically binds to EGFR, the light chain of the first member comprises light chain CDR1-3, the amino acid sequence of the light chain CDR1 is as set forth in SEQ ID NO: 101, the amino acid sequence of the light chain CDR2 is as set forth in SEQ ID NO: 102, and the amino acid sequence of the light chain CDR3 is as set forth in SEQ ID NO: 103; and the heavy chain of the first member comprises heavy chain CDR1-3, the amino acid sequence of the heavy chain CDR1 is as set forth in SEQ ID NO: 105, the amino acid sequence of the heavy chain CDR2 is as set forth in SEQ ID NO: 106, and the amino acid sequence of the heavy chain CDR3 is as set forth in SEQ ID NO: 107.

In some embodiments, the targeting moiety specifically binds to EGFR, the light chain of the first member comprises a light chain variable region, and the amino acid sequence of the light chain variable region is as set forth in SEQ ID NO: 104; and the heavy chain of the first member comprises a heavy chain variable region, and the amino acid sequence of the heavy chain variable region is as set forth in SEQ ID NO: 108.

In some embodiments, the targeting moiety specifically binds to EGFR, and the amino acid sequence of the light chain of the first member is as set forth in SEQ ID NO: 37; and the amino acid sequence of the heavy chain of the first member is as set forth in SEQ ID NO: 39.

In some embodiments, the targeting moiety specifically binds to an EGFR mutant, the light chain of the first member comprises CDR1-3, the amino acid sequence of the CDR1 is as set forth in SEQ ID NO: 109, the amino acid sequence of the CDR2 is as set forth in SEQ ID NO: 110, and the amino acid sequence of the CDR3 is as set forth in SEQ ID NO: 111.

In some embodiments, the targeting moiety specifically binds to an EGFR mutant, the light chain of the first member comprises a light chain variable region, and the amino acid sequence of the light chain variable region is as set forth in SEQ ID NO: 112.

In some embodiments, the targeting moiety specifically binds to an EGFR mutant, and the amino acid sequence of the light chain of the first member is as set forth in SEQ ID NO: 53.

In some embodiments, the targeting moiety specifically binds to an EGFR mutant, the heavy chain of the first member comprises CDR1-3, the amino acid sequence of the CDR1 is as set forth in SEQ ID NO: 113, the amino acid sequence of the CDR2 is as set forth in SEQ ID NO: 114, and the amino acid sequence of the CDR3 is as set forth in SEQ ID NO: 115.

In some embodiments, the targeting moiety specifically binds to an EGFR mutant, the heavy chain of the first member comprises a heavy chain variable region, and the amino acid sequence of the heavy chain variable region is as set forth in SEQ ID NO: 116.

In some embodiments, the targeting moiety specifically binds to an EGFR mutant, and the amino acid sequence of the heavy chain of the first member is as set forth in SEQ ID NO: 55.

In some embodiments, the targeting moiety specifically binds to an EGFR mutant, the light chain of the first member comprises light chain CDR1-3, the amino acid sequence of the light chain CDR1 is as set forth in SEQ ID NO: 109, the amino acid sequence of the light chain CDR2 is as set forth in SEQ ID NO: 110, and the amino acid sequence of the light chain CDR3 is as set forth in SEQ ID NO: 111; and the heavy chain of the first member comprises heavy chain CDR1-3, the amino acid sequence of the heavy chain CDR1 is as set forth in SEQ ID NO: 113, the amino acid sequence of the heavy chain CDR2 is as set forth in SEQ ID NO: 114, and the amino acid sequence of the heavy chain CDR3 is as set forth in SEQ ID NO: 115.

In some embodiments, the targeting moiety specifically binds to an EGFR mutant, the light chain of the first member comprises a light chain variable region, and the amino acid sequence of the light chain variable region is as set forth in SEQ ID NO: 112; and the heavy chain of the first member comprises a heavy chain variable region, and the amino acid sequence of the heavy chain variable region is as set forth in SEQ ID NO: 116.

In some embodiments, the targeting moiety specifically binds to an EGFR mutant, and the amino acid sequence of the light chain of the first member is as set forth in SEQ ID NO: 53; and the amino acid sequence of the heavy chain of the first member is as set forth in SEQ ID NO: 55.

In some embodiments, the targeting moiety specifically binds to HER2/neu, the light chain of the first member comprises CDR1-3, the amino acid sequence of the CDR1 is selected from SEQ ID NOs: 117 and 125, the amino acid sequence of the CDR2 is selected from SEQ ID NOs: 118 and 126, and the amino acid sequence of the CDR3 is selected from SEQ ID NOs: 119 and 127.

In some embodiments, the targeting moiety specifically binds to HER2/neu, the light chain of the first member comprises a light chain variable region, and the amino acid sequence of the light chain variable region is selected from SEQ ID NOs: 120 and 128.

In some embodiments, the targeting moiety specifically binds to HER2/neu, and the amino acid sequence of the light chain of the first member is selected from SEQ ID NOs: 45 and 49.

In some embodiments, the targeting moiety specifically binds to HER2/neu, the heavy chain of the first member comprises CDR1-3, the amino acid sequence of the CDR1 is selected from SEQ ID NOs: 121 and 129, the amino acid sequence of the CDR2 is selected from SEQ ID NOs: 122 and 130, and the amino acid sequence of the CDR3 is selected from SEQ ID NOs: 123 and 131.

In some embodiments, the targeting moiety specifically binds to HER2/neu, the antibody heavy chain of the first member comprises a heavy chain variable region, and the amino acid sequence of the heavy chain variable region is selected from SEQ ID NOs: 124 and 132.

In some embodiments, the targeting moiety specifically binds to HER2/neu, and the amino acid sequence of the heavy chain of the first member is selected from SEQ ID NOs: 47 and 51.

In some embodiments, the targeting moiety specifically binds to HER2/neu, the light chain of the first member comprises light chain CDR1-3, the amino acid sequence of the light chain CDR1 is selected from SEQ ID NOs: 117 and 125, the amino acid sequence of the light chain CDR2 is selected from SEQ ID NOs: 118 and 126, and the amino acid sequence of the light chain CDR3 is selected from SEQ ID NOs: 119 and 127; and the heavy chain of the first member comprises heavy chain CDR1-3, the amino acid sequence of the heavy chain CDR1 is selected from SEQ ID NOs: 121 and 129, the amino acid sequence of the heavy chain CDR2 is selected from SEQ ID NOs: 122 and 130, and the amino acid sequence of the heavy chain CDR3 is selected from SEQ ID NOs: 123 and 131.

In some embodiments, the targeting moiety specifically binds to HER2/neu, the light chain of the first member comprises a light chain variable region, and the amino acid sequence of the light chain variable region is selected from SEQ ID NOs: 120 and 128; and the heavy chain of the first member comprises a heavy chain variable region, and the amino acid sequence of the heavy chain variable region is selected from SEQ ID NOs: 124 and 132.

In some embodiments, the targeting moiety specifically binds to HER2/neu, and the amino acid sequence of the light chain of the first member is selected from SEQ ID NOs: 45 and 49; and the amino acid sequence of the heavy chain of the first member is selected from SEQ ID NOs: 47 and 51. In some embodiments, the targeting moiety specifically binds to GPC3, the light chain of the first member comprises CDR1-3, the amino acid sequence of the CDR1 is as set forth in SEQ ID NO: 133, the amino acid sequence of the CDR2 is as set forth in SEQ ID NO: 134, and the amino acid sequence of the CDR3 is as set forth in SEQ ID NO: 135.

In some embodiments, the targeting moiety specifically binds to GPC3, the light chain of the first member comprises a light chain variable region, and the amino acid sequence of the light chain variable region is as set forth in SEQ ID NO: 136.

In some embodiments, the targeting moiety specifically binds to GPC3, and the amino acid sequence of the light chain of the first member is as set forth in SEQ ID NO: 57.

In some embodiments, the targeting moiety specifically binds to GPC3, the heavy chain of the first member comprises CDR1-3, the amino acid sequence of the CDR1 is as set forth in SEQ ID NO: 137, the amino acid sequence of the CDR2 is as set forth in SEQ ID NO: 138, and the amino acid sequence of the CDR3 is as set forth in SEQ ID NO: 139.

In some embodiments, the targeting moiety specifically binds to GPC3, the heavy chain of the first member comprises a heavy chain variable region, and the amino acid sequence of the heavy chain variable region is as set forth in SEQ ID NO: 140.

In some embodiments, the targeting moiety specifically binds to GPC3, and the amino acid sequence of the heavy chain of the first member is as set forth in SEQ ID NO: 59.

In some embodiments, the targeting moiety specifically binds to GPC3, the light chain of the first member comprises light chain CDR1-3, the amino acid sequence of the light chain CDR1 is as set forth in SEQ ID NO: 133, the amino acid sequence of the light chain CDR2 is as set forth in SEQ ID NO: 134, and the amino acid sequence of the light chain CDR3 is as set forth in SEQ ID NO: 135; and the heavy chain of the first member comprises heavy chain CDR1-3, the amino acid sequence of the heavy chain CDR1 is as set forth in SEQ ID NO: 137, the amino acid sequence of the heavy chain CDR2 is as set forth in SEQ ID NO: 138, and the amino acid sequence of the heavy chain CDR3 is as set forth in SEQ ID NO: 139.

In some embodiments, the targeting moiety specifically binds to GPC3, the light chain of the first member comprises a light chain variable region, and the amino acid sequence of the light chain variable region is as set forth in SEQ ID NO: 136; and the heavy chain of the first member comprises a heavy chain variable region, and the amino acid sequence of the heavy chain variable region is as set forth in SEQ ID NO: 140.

In some embodiments, the targeting moiety specifically binds to GPC3, and the amino acid sequence of the light chain of the first member is as set forth in SEQ ID NO: 57; and the amino acid sequence of the heavy chain of the first member is as set forth in SEQ ID NO: 59.

In some embodiments, the targeting moiety specifically binds to FAP, the light chain of the first member comprises CDR1-3, the amino acid sequence of the CDR1 is as set forth in SEQ ID NO: 141, the amino acid sequence of the CDR2 is as set forth in SEQ ID NO: 142, and the amino acid sequence of the CDR3 is as set forth in SEQ ID NO: 143.

In some embodiments, the targeting moiety specifically binds to FAP, the light chain of the first member comprises a light chain variable region, and the amino acid sequence of the light chain variable region is as set forth in SEQ ID NO: 144.

In some embodiments, the targeting moiety specifically binds to FAP, and the amino acid sequence of the light chain of the first member is as set forth in SEQ ID NO: 61.

In some embodiments, the targeting moiety specifically binds to FAP, the heavy chain of the first member comprises CDR1-3, the amino acid sequence of the CDR1 is as set forth in SEQ ID NO: 145, the amino acid sequence of the CDR2 is as set forth in SEQ ID NO: 146, and the amino acid sequence of the CDR3 is as set forth in SEQ ID NO: 147.

In some embodiments, the targeting moiety specifically binds to FAP, the heavy chain of the first member comprises a heavy chain variable region, and the amino acid sequence of the heavy chain variable region is as set forth in SEQ ID NO: 148.

In some embodiments, the targeting moiety specifically binds to FAP, and the amino acid sequence of the heavy chain of the first member is as set forth in SEQ ID NO: 63.

In some embodiments, the targeting moiety specifically binds to FAP, the light chain of the first member comprises light chain CDR1-3, the amino acid sequence of the light chain CDR1 is as set forth in SEQ ID NO: 141, the amino acid sequence of the light chain CDR2 is as set forth in SEQ ID NO: 142, and the amino acid sequence of the light chain CDR3 is as set forth in SEQ ID NO: 143; and the heavy chain of the first member comprises heavy chain CDR1-3, the amino acid sequence of the heavy chain CDR1 is as set forth in SEQ ID NO: 145, the amino acid sequence of the heavy chain CDR2 is as set forth in SEQ ID NO: 146, and the amino acid sequence of the heavy chain CDR3 is as set forth in SEQ ID NO: 147.

In some embodiments, the targeting moiety specifically binds to FAP, the light chain of the first member comprises a light chain variable region, and the amino acid sequence of the light chain variable region is as set forth in SEQ ID NO: 148; and the chain of the first member comprises a heavy chain variable region, and the amino acid sequence of the heavy chain variable region is as set forth in SEQ ID NO: 152.

In some embodiments, the targeting moiety specifically binds to FAP, and the amino acid sequence of the light chain of the first member is as set forth in SEQ ID NO: 61; and the amino acid sequence of the heavy chain of the first member is as set forth in SEQ ID NO: 63.

In some embodiments, the targeting moiety specifically binds to Muc1, the antibody light chain of the first member comprises CDR1-3, the amino acid sequence of the CDR1 is as set forth in SEQ ID NO: 149, the amino acid sequence of the CDR2 is as set forth in SEQ ID NO: 150, and the amino acid sequence of the CDR3 is as set forth in SEQ ID NO: 151.

In some embodiments, the targeting moiety specifically binds to Muc1, the antibody light chain of the first member comprises a light chain variable region, and the amino acid sequence of the light chain variable region is as set forth in SEQ ID NO: 152.

In some embodiments, the targeting moiety specifically binds to Muc1, and the amino acid sequence of the light chain of the first member is as set forth in SEQ ID NO: 65.

In some embodiments, the targeting moiety specifically binds to Muc1, the heavy chain of the first member comprises CDR1-3, the amino acid sequence of the CDR1 is as set forth in SEQ ID NO: 153, the amino acid sequence of the CDR2 is as set forth in SEQ ID NO: 154, and the amino acid sequence of the CDR3 is as set forth in SEQ ID NO: 155.

In some embodiments, the targeting moiety specifically binds to Muc1, the heavy chain of the first member comprises a heavy chain variable region, and the amino acid sequence of the heavy chain variable region is as set forth in SEQ ID NO: 156.

In some embodiments, the targeting moiety specifically binds to Muc1, and the amino acid sequence of the heavy chain of the first member is as set forth in SEQ ID NO: 67.

In some embodiments, the targeting moiety specifically binds to Muc1, the light chain of the first member comprises light chain CDR1-3, the amino acid sequence of the light chain CDR1 is as set forth in SEQ ID NO: 149, the amino acid sequence of the light chain CDR2 is as set forth in SEQ ID NO: 150, and the amino acid sequence of the light chain CDR3 is as set forth in SEQ ID NO: 151; and the heavy chain of the first member comprises heavy chain CDR1-3, the amino acid sequence of the heavy chain CDR1 is as set forth in SEQ ID NO: 153, the amino acid sequence of the heavy chain CDR2 is as set forth in SEQ ID NO: 154, and the amino acid sequence of the heavy chain CDR3 is as set forth in SEQ ID NO: 155.

In some embodiments, the targeting moiety specifically binds to Muc1, the light chain of the first member comprises a light chain variable region, and the amino acid sequence of the light chain variable region is as set forth in SEQ ID NO: 152; and the heavy chain of the first member comprises a heavy chain variable region, and the amino acid sequence of the heavy chain variable region is as set forth in SEQ ID NO: 156.

In some embodiments, the targeting moiety specifically binds to Muc1, and the amino acid sequence of the light chain of the first member is as set forth in SEQ ID NO: 65; and the amino acid sequence of the heavy chain of the first member is as set forth in SEQ ID NO: 67.

In some embodiments, the targeting moiety specifically binds to Mesothelin, the light chain of the first member comprises CDR1-3, the amino acid sequence of the CDR1 is as set forth in SEQ ID NO: 165, the amino acid sequence of the CDR2 is as set forth in SEQ ID NO: 166, and the amino acid sequence of the CDR3 is as set forth in SEQ ID NO: 167.

In some embodiments, the targeting moiety specifically binds to Mesothelin, the light chain of the first member comprises a light chain variable region, and the amino acid sequence of the light chain variable region is as set forth in SEQ ID NO: 168.

In some embodiments, the targeting moiety specifically binds to Mesothelin, and the amino acid sequence of the light chain of the first member is as set forth in SEQ ID NO: 73.

In some embodiments, the targeting moiety specifically binds to Mesothelin, the heavy chain of the first member comprises CDR1-3, the amino acid sequence of the CDR1 is as set forth in SEQ ID NO: 169, the amino acid sequence of the CDR2 is as set forth in SEQ ID NO: 170, and the amino acid sequence of the CDR3 is as set forth in SEQ ID NO: 171.

In some embodiments, the targeting moiety specifically binds to Mesothelin, the heavy chain of the first member comprises a heavy chain variable region, and the amino acid sequence of the heavy chain variable region is as set forth in SEQ ID NO: 172.

In some embodiments, the targeting moiety specifically binds to Mesothelin, and the amino acid sequence of the heavy chain of the first member is as set forth in SEQ ID NO: 75.

In some embodiments, the targeting moiety specifically binds to Mesothelin, the light chain of the first member comprises light chain CDR1-3, the amino acid sequence of the light chain CDR1 is as set forth in SEQ ID NO: 165, the amino acid sequence of the light chain CDR2 is as set forth in SEQ ID NO: 166, and the amino acid sequence of the light chain CDR3 is as set forth in SEQ ID NO: 167; and the heavy chain of the first member comprises heavy chain CDR1-3, the amino acid sequence of the heavy chain CDR1 is as set forth in SEQ ID NO: 169, the amino acid sequence of the heavy chain CDR2 is as set forth in SEQ ID NO: 170, and the amino acid sequence of the heavy chain CDR3 is as set forth in SEQ ID NO: 171.

In some embodiments, the targeting moiety specifically binds to Mesothelin, the light chain of the first member comprises a light chain variable region, and the amino acid sequence of the light chain variable region is as set forth in SEQ ID NO: 168; and the heavy chain of the first member comprises a heavy chain variable region, and the amino acid sequence of the heavy chain variable region is as set forth in SEQ ID NO: 172.

In some embodiments, the targeting moiety specifically binds to Mesothelin, and the amino acid sequence of the light chain of the first member is as set forth in SEQ ID NO: 73; and the amino acid sequence of the heavy chain of the first member is as set forth in SEQ ID NO: 75.

In some embodiments, the targeting moiety specifically binds to MUC5AC, the light chain of the first member comprises CDR1-3, the amino acid sequence of the CDR1 is as set forth in SEQ ID NO: 157, the amino acid sequence of the CDR2 is as set forth in SEQ ID NO: 158, and the amino acid sequence of the CDR3 is as set forth in SEQ ID NO: 159.

In some embodiments, the targeting moiety specifically binds to MUC5AC, the light chain of the first member comprises a light chain variable region, and the amino acid sequence of the light chain variable region is as set forth in SEQ ID NO: 160.

In some embodiments, the targeting moiety specifically binds to MUC5AC, and the amino acid sequence of the light chain of the first member is as set forth in SEQ ID NO: 69.

In some embodiments, the targeting moiety specifically binds to MUC5AC, the heavy chain of the first member comprises CDR1-3, the amino acid sequence of the CDR1 is as set forth in SEQ ID NO: 161, the amino acid sequence of the CDR2 is as set forth in SEQ ID NO: 162, and the amino acid sequence of the CDR3 is as set forth in SEQ ID NO: 163.

In some embodiments, the targeting moiety specifically binds to MUC5AC, the heavy chain of the first member comprises a heavy chain variable region, and the amino acid sequence of the heavy chain variable region is as set forth in SEQ ID NO: 164.

In some embodiments, the targeting moiety specifically binds to MUC5AC, and the amino acid sequence of the heavy chain of the first member is as set forth in SEQ ID NO: 71.

In some embodiments, the targeting moiety specifically binds to MUC5AC, the light chain of the first member comprises light chain CDR1-3, the amino acid sequence of the light chain CDR1 is as set forth in SEQ ID NO: 157, the amino acid sequence of the light chain CDR2 is as set forth in SEQ ID NO: 158, and the amino acid sequence of the light chain CDR3 is as set forth in SEQ ID NO: 159; and the heavy chain of the first member comprises heavy chain CDR1-3, the amino acid sequence of the heavy chain CDR1 is as set forth in SEQ ID NO: 161, the amino acid sequence of the heavy chain CDR2 is as set forth in SEQ ID NO: 162, and the amino acid sequence of the heavy chain CDR3 is as set forth in SEQ ID NO: 163.

In some embodiments, the targeting moiety specifically binds to MUC5AC, the light chain of the first member comprises a light chain variable region, and the amino acid sequence of the light chain variable region is as set forth in SEQ ID NO: 160; and the heavy chain of the first member comprises a heavy chain variable region, and the amino acid sequence of the heavy chain variable region is as set forth in SEQ ID NO: 164.

In some embodiments, the targeting moiety specifically binds to MUC5AC, and the amino acid sequence of the light chain of the first member is as set forth in SEQ ID NO: 69; and the amino acid sequence of the heavy chain of the first member is as set forth in SEQ ID NO: 71.

In some embodiments, the immunoregulator augments an immune response. Examples of immunoregulators capable of augmenting an immune response include, but are not limited to, IL-2, IFNα, IFNβ, IFNγ, IFNλ, tumor necrosis factor (TNF) α, IL-12, and IL-10.

In some embodiments, the immunoregulator reduces an immune response. Non-limiting examples of immunoregulators capable of reducing an immune response include IL-10, and transforming growth factor (TGF)-β.

In some embodiments, the immunoregulator is a cytokine. For example, the immunoregulator may be a cytokine selected from the group consisting of an interferon, an interleukin, a chemokine, a lymphokine, and a tumor necrosis factor.

In some embodiments, the immunoregulator is an interferon selected from the group consisting of interferon alpha, interferon lambda, and interferon beta.

In some embodiments, the immunoregulator is an interleukin, and the interleukin comprises interleukin 10, interleukin 2, and/or super interleukin 2.

In some embodiments, the first Fc region and the second Fc region are from an Fc region of an immunoglobulin. For example, the immunoglobulin may be selected from the group consisting of IgG1, IgG2, IgG3, and IgG4. In some embodiments, the first Fc region and the second Fc region are from an Fc region of an immunoglobulin, and the immunoglobulin is a human IgG1.

In some embodiments, the first modification and/or the second modification is in comparison to the wild-type amino acid sequence of the Fc region of human IgG1.

In some embodiments, the second Fc region is fused in frame to the immunoregulator.

In some embodiments, the polypeptide comprised in the second member comprises two or more immunoregulators, the two or more immunoregulators are fused in frame to each other and to the second Fc region, and wherein the two or more immunoregulators are located N-terminal to the second Fc region. In some embodiments, the two or more immunoregulators may be fused in frame to each other and/or to the second Fc region via a linker. The linker may be a synthetic amino acid sequence that connects or links two polypeptide sequences, e.g., via peptide bonds. In some embodiments, the linker is a peptide comprising, e.g., 1, 2, 3, 4, 5, 6, 7, 8, 9, 10, 11, 12, 13, 14, 15, 16, 17, 18, 19, 20, 21, 22, 23, 24, 25, or more amino acids. The two or more immunoregulators may be of the same type or may be of different types. For example, the two or more immunoregulators may be the same. In some embodiments, the two or more immunoregulators are interleukin 10.

In some embodiments, in the heavy chain of the first member, the amino acid sequence of the first Fc region is selected from SEQ ID NOs: 1, 4, 5, 6, 7, 9, 11, 13, 15, 17, 19, 21, 22, 24, 26, 27, and 29. In some embodiments, the amino acid sequence of the immunoregulator comprised in the second member is selected from SEQ ID NOs: 173-180.

In some embodiments, the amino acid sequence of the second Fc region comprised in the second member is selected from SEQ ID NOs: 2, 3, 8, 10, 12, 14, 16, 18, 20, 23, 25, and 28.

In some embodiments, the amino acid sequence of the polypeptide comprised in the second member is selected from SEQ ID NOs: 77, 80, 82, 84, 86, 89, 91, and 97.

In some embodiments, the amino acid sequence of the light chain comprised in the first member is selected from SEQ ID NOs: 37, 45, 49, 53, 57, 61, 65, 69, and 73. The amino acid sequence of the heavy chain comprised in the first member is selected from SEQ ID NOs: 39, 47, 51, 55, 59, 63, 67, 71, and 75, and the amino acid sequence of the polypeptide comprised in the second member is selected from SEQ ID NOs: 77, 80, 82, 84, 86, 89, 91, and 97.

In another aspect, the present disclosure provides a protein mixture, comprising: 1) the proteinaceous heterodimer according to the present disclosure; 2) a first homodimer formed by two of the first member of the proteinaceous heterodimer; and 3) a second homodimer formed by two of the second member of the proteinaceous heterodimer. The percentage of the proteinaceous heterodimer in the protein mixture may be at least 50% (e.g., at least 55%, at least 60%, at least 65%, at least 70%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, or more).

In some embodiments, in the protein mixture, the percentage of the second homodimer is less than the percentage of the first homodimer. For example, the percentage of the first homodimer may be at least 1.1 fold, at least 1.2 fold, at least 1.3 fold, at least 1.4 fold, at least 1.5 fold, at least 1.6 fold, at least 1.7 fold, at least 1.8 fold, at least 1.9 fold, at least 2.0 fold, at least 2.1 fold, at least 2.2 fold, at least 2.3 fold, at least 2.4 fold, at least 2.5 fold, at least 2.6 fold, at least 2.7 fold, at least 2.8 fold, at least 2.9 fold, at least 3.0 fold, at least 3.1 fold, at least 3.2 fold, at least 3.5 fold, at least 4.0 fold, at least 4.5 fold, at least 5.0 fold, at least 5.5 fold, at least 6.0 fold, at least 7.0 fold, at least 8.0 fold, or more than that of the second homodimer.

In some embodiments, the percentage of the second homodimer in the protein mixture is at most 10% (e.g., at most 0.0%, at most 0.01%, at most 0.1%, at most 0.5%, at most 1%, at most 1.5%, at most 2%, at most 3%, at most 4%, at most 5%, at most 6%, at most 7%, at most 8%, or at most 9%). In some embodiments, the protein mixture substantially comprises none of the second homodimer.

In another aspect, the present disclosure provides an isolated polynucleotide encoding the proteinaceous heterodimer according to the present disclosure. In some embodiments, the isolated polynucleotide encodes a subunit (e.g., a member) or a fragment of the proteinaceous heterodimer according to the present disclosure.

The polynucleotide may be synthesized using recombinant techniques well known in the art. For example, the polynucleotide may be synthesized by use of an automated DNA synthesizer.

Standard recombinant DNA and molecular cloning techniques include those described by Sambrook, J., Fritsch, E. F. and Maniatis, T. Molecular Cloning: A Laboratory Manual; Cold Spring Harbor Laboratory Press: Cold Spring Harbor, (1989) (Maniat*is)* and by T. J. Silhavy, M. L. Bennan, and L. W. Enquist, Experiments with Gene Fusions, Cold Spring Harbor Laboratory, Cold Spring Harbor, N.Y. (1984*)* and by Ausubel, F. M. et al., Current Protocols in Molecular Biology, pub. by Greene Publishing Assoc. and Wiley-Interscience (1987). Briefly, the nucleic acids of the present disclosure can be prepared from genomic DNA fragments, cDNAs, and RNAs, all of which can be extracted directly from a cell or recombinantly produced by various amplification processes including but not limited to PCR and RT-PCR.

Direct chemical synthesis of nucleic acids typically involves sequential addition of 3'-blocked and 5'-blocked nucleotide monomers to the terminal 5'-hydroxyl group of a growing nucleotide polymer chain, wherein each addition is effected by nucleophilic attack of the terminal 5'-hydroxyl group of the growing chain on the 3'-position of the added monomer, which is typically a phosphorus derivative, such as a phosphotriester, phosphoramidite, or the like. See, for example, Matteuci et al., Tet. Lett. 521:719 (1980*);* U.S. Pat. No. 4,500,707 to Caruthers et al.; and U.S. Pat. Nos. 5,436,327 and 5,700,637 to Southern et al.

In another aspect, the present disclosure provides a vector comprising the isolated polynucleotide of the present disclosure.

The vector may be any linear nucleic acids, plasmids, phagemids, cosmids, RNA vectors, viral vectors, and the like. Non-limiting examples of a viral vector may include a retrovirus, an adenovirus, and an adeno-associated virus. In some embodiments, the vector is an expression vector, e.g., a phage display vector.

An expression vector may be suitable for use in particular types of host cells and not others. For example, the expression vector can be introduced into the host organism, which is then monitored for viability and expression of any genes/polynucleotides contained in the vector.

The expression vector may also contain one or more selectable marker genes that, upon expression, confer one or more phenotypic traits useful for selecting or otherwise identifying host cells that carry the expression vector. Non-limiting examples of suitable selectable markers for eukaryotic cells include dihydrofolate reductase and neomycin resistance.

The subject vectors can be introduced into a host cell stably or transiently by a variety of established techniques. For example, one method involves a calcium chloride treatment, wherein the expression vector is introduced via a calcium precipitate. Other salts, for example, calcium phosphate, may also be used following a similar procedure. In addition, electroporation (that is, the application of current to increase the permeability of cells to nucleic acids) may be used. Other examples of transformation methods include microinjection, DEAE dextran mediated transformation, and heat shock in the presence of lithium acetate. Lipid complexes, liposomes, and dendrimers may also be employed to transfect the host cells.

Upon introduction of the heterologous sequence into a host cell, a variety of methods can be practiced to identify the host cells into which the subject vectors have been introduced. One exemplary selection method involves subculturing individual cells to form individual colonies, followed by testing for expression of the desired protein product. Another method entails selecting host cells containing the heterologous sequence based upon phenotypic traits conferred through the expression of selectable marker genes contained within the expression vector.

For example, the introduction of various heterologous sequences of the present disclosure into a host cell can be confirmed by methods such as PCR, Southern blot, or Northern blot hybridization. For example, nucleic acids can be prepared from the resultant host cells, and the specific sequences of interest can be amplified by PCR using primers specific for the sequences of interest. The amplified product is subjected to agarose gel electrophoresis, polyacrylamide gel electrophoresis, or capillary electrophoresis, followed by staining with ethidium bromide, SYBR Green solution or the like, or detection of DNA with UV detection. Alternatively, nucleic acid probes specific for the sequences of interest can be employed in a hybridization reaction. The expression of a specific gene sequence can be ascertained by detecting the corresponding mRNA via reverse-transcription coupled with PCR, Northern blot hybridization, or by immunoassays using antibodies reactive with the encoded gene product. Exemplary immunoassays include, but are not limited to ELISA, radioimmunoassays, and sandwich immunoassays.

Furthermore, the introduction of various heterologous sequences of the present disclosure into a host cell can be confirmed by the enzymatic activity of an enzyme (e.g., an enzymatic marker) that the heterologous sequence encodes. The enzyme can be assayed by a variety of methods known in the art. In general, the enzymatic activity can be ascertained by the formation of the product or conversion of a substrate of an enzymatic reaction that is under investigation. The reaction can take place *in vitro* or *in vivo.*

In another aspect, the present disclosure provides an isolated host cell, comprising the isolated polynucleotide or the vector of the present disclosure, and/or capable of expressing the proteinaceous heterodimer, and/or the isolated polynucleotide encoding the proteinaceous heterodimer, and/or the protein mixture of the present disclosure.

In some embodiments, the cell expresses the heterodimer protein of the present disclosure, the isolated polynucleotide encoding the heterodimer protein and/or the protein mixture of the present disclosure. The cell may be a eukaryotic cell or a prokaryotic cell. An appropriate cell may be transformed or transfected with the polynucleotide or vector of the present disclosure, and utilized for the expression and/or secretion of the heterodimer protein and/or the protein mixture. For example, the cell may be E. coli cells, other bacterial host cells, yeast cells, or various higher eukaryotic cells (e.g., immortal hybridoma cells, NS0 myeloma cells, HEK293 cells, Chinese hamster ovary cells, HeLa cells, COS cells, etc.). In some embodiments, polynucleotides encoding the proteinaceous heterodimer (e.g., a heterodimer protein) are operably connected to an expression control sequence suitable for expression in specific host cells.

### Medicament

The present disclosure provides use of the proteinaceous heterodimer described herein in the manufacture of a medicament for preventing and/or treating a malignant effusion.

In the present disclosure, the medicament may be a pharmaceutical composition, which may further comprise a pharmaceutically acceptable excipient.

Examples of pharmaceutically acceptable excipients include, but are not limited to inert solid diluents and fillers, diluents, sterile aqueous solution and various organic solvents, permeation enhancers, solubilizers and adjuvants.

In some embodiments, the medicament may be formulated for oral administration, intravenous administration, intramuscular administration, in-situ administration at the site of a tumor, inhalation, rectal administration, vaginal administration, transdermal administration, or administration via subcutaneous repository.

The medicament may be used for the prevention and/or treatment of a malignant effusion.

For example, the medicament may inhibit or delay the development or progression of a disease, may reduce tumor size (and even substantially eliminate tumors), and may alleviate and/or stabilize a disease condition.

Described below are non-limiting exemplary medicaments and methods for preparing same.

The medicament may, for example, be in a form suitable for oral administration as a tablet, capsule, pill, powder, sustained release formulation, solution, suspension, for parenteral injection as a sterile solution, suspension or emulsion, for topical administration as an ointment or cream, or for rectal administration as a suppository. The medicament may be in unit dosage forms suitable for single administration of precise dosages. The medicament may further comprise a proteinaceous heterodimer (e.g., a heterodimer protein) or a protein mixture according to the present disclosure as an active ingredient and may comprise a conventional pharmaceutical carrier or excipient. Further, it may include other medicinal or pharmaceutical agents, carriers, adjuvants, etc.

Exemplary parenteral administration forms include, but are not limited to, solutions or suspensions of an active proteinaceous heterodimer (e.g., a heterodimer protein) in sterile aqueous solutions, for example, aqueous propylene glycol or dextrose solutions. Such dosage forms can be suitably buffered with salts such as histidine and/or phosphate, if desired.

In some embodiments, the present disclosure provides a medicament for injection containing a proteinaceous heterodimer (e.g., a heterodimer protein) or a protein mixture of the present disclosure and a pharmaceutical excipient suitable for injection. Components and amounts of agents in the compositions are as described herein.

The forms in which the medicaments of the present disclosure may be incorporated for administration by injection include aqueous or oil suspensions, or emulsions, with sesame oil, corn oil, cottonseed oil, or peanut oil, as well as elixirs, mannitol, dextrose, or a sterile aqueous solution, and similar pharmaceutical vehicles.

Aqueous solutions in saline may also be used for injection. Ethanol, glycerol, propylene glycol, liquid polyethylene glycol, and the like (and suitable mixtures thereof), cyclodextrin derivatives, and vegetable oils may also be employed. The proper fluidity can be maintained, for example, by the use of a coating, such as lecithin, for the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like.

Sterile injectable solutions can be prepared by incorporating the proteinaceous heterodimer (e.g., heterodimer protein) or a protein mixture of the present disclosure in a suitable amount in the appropriate solvent with various other ingredients as enumerated above, as needed, followed by filtered sterilization. Generally, dispersions are prepared by incorporating the various sterilized active ingredients into a sterile vehicle which contains the basic dispersion medium and other ingredients from those enumerated above, as needed or desired. In the case of sterile powders for the preparation of sterile injectable solutions, certain desirable methods of preparation are vacuum-drying and freeze-drying techniques which yield a powder of the active ingredient plus any additional desired ingredient from a previously sterile-filtered solution thereof.

In some embodiments, the present disclosure provides a medicament for oral administration containing a proteinaceous heterodimer (e.g., a heterodimer protein) or a protein mixture of the present disclosure, and a pharmaceutical excipient suitable for oral administration.

In some embodiments, the present disclosure provides a solid medicament for oral administration containing: (i) an amount of a proteinaceous heterodimer (e.g., a heterodimer protein) or a protein mixture of the present disclosure; optionally (ii) an amount of a second agent; and (iii) a pharmaceutical excipient suitable for oral administration. In some embodiments, the composition further contains: (iv) an amount of a third agent. In some embodiments, amounts of the proteinaceous heterodimer or the protein mixture, second agent, and optional third agent are amounts that, alone or in combination, are effective in treating a condition of a subject.

In some embodiments, the pharmaceutical composition may be a liquid medicament suitable for oral consumption. Medicaments of the present disclosure suitable for oral administration can be presented as discrete dosage forms, such as capsules, cachets, or tablets, or liquids or aerosol sprays each containing a predetermined amount of an active ingredient as a powder or in granules, a solution, or a suspension in an aqueous or non-aqueous liquid, an oil-in-water emulsion, or a water-in-oil liquid emulsion. Such dosage forms can be prepared by any of the methods of pharmacy, but all methods typically include the step of bringing the active ingredient into association with the carrier, which constitutes one or more other ingredients. In general, the compositions are prepared by uniformly and intimately admixing the active ingredient with liquid carriers or finely divided solid carriers or both, and then, if necessary, shaping the product into the desired presentation.

The present disclosure further encompasses anhydrous medicaments and dosage forms comprising an active ingredient (e.g., a proteinaceous heterodimer or a heterodimer protein of the present disclosure), since water can facilitate the degradation of some polypeptides. For example, water may be added (e.g., 5%) in the pharmaceutical arts as a means of simulating long-term storage in order to determine characteristics such as shelf-life or the stability of formulations over time. An anhydrous pharmaceutical composition may be prepared and stored such that its anhydrous nature is maintained. Accordingly, anhydrous compositions may be packaged using materials known to prevent exposure to water such that they can be included in suitable formulary kits. Examples of suitable packaging include, but are not limited to, hermetically sealed foils, plastic or the like, unit dose containers, blister packs, and strip packs.

A proteinaceous heterodimer (e.g., a heterodimer protein or complex) or a protein mixture of the present disclosure can be combined in an intimate admixture with a pharmaceutical carrier according to conventional pharmaceutical compounding techniques. The carrier can take a wide variety of forms depending on the form of formulation desired for administration. In preparing the compositions for an oral dosage form, any of the usual pharmaceutical media can be employed as carriers, such as, for example, water, glycols, oils, alcohols, flavoring agents, preservatives, coloring agents, and the like in the case of oral liquid formulations (such as suspensions, solutions, and elixirs) or aerosols; or carriers such as starches, sugars, micro-crystalline cellulose, diluents, granulating agents, lubricants, binders, and disintegrating agents can be used in the case of oral solid formulations, in some embodiments without employing the use of lactose. For example, suitable carriers include powders, capsules, and tablets, as well as the solid oral formulations. If desired, tablets can be coated by standard aqueous or non-aqueous techniques.

When aqueous suspensions and/or elixirs are desired for oral administration, the active ingredient therein may be combined with various sweetening or flavoring agents, coloring matter or dyes and, if so desired, emulsifying and/or suspending agents, together with such diluents as water, ethanol, propylene glycol, glycerin and various combinations thereof.

The tablets can be uncoated or coated by known techniques to delay disintegration and absorption in the gastrointestinal tract and thereby provide a sustained action over a longer period.

Surfactants which can be used to form pharmaceutical compositions and dosage forms of the present disclosure include, but are not limited to, hydrophilic surfactants, lipophilic surfactants, and mixtures thereof. A mixture of hydrophilic surfactants may be employed, a mixture of lipophilic surfactants may be employed, or a mixture of at least one hydrophilic surfactant and at least one lipophilic surfactant may be employed.

In some embodiments, the medicament may include a solubilizer to ensure good solubilization and/or dissolution of the proteinaceous heterodimer or the protein mixture of the present disclosure and to minimize precipitation of the proteinaceous heterodimer or protein mixture of the present disclosure. This can be especially important for compositions for non-oral use, e.g., compositions for injection. A solubilizer may also be added to increase the solubility of the hydrophilic drug and/or other components, such as surfactants, or to maintain the composition as a stable or homogeneous solution or dispersion.

The composition can further include one or more pharmaceutically acceptable additives and excipients. Such additives and excipients include, but are not limited to, detackifiers, anti-foaming agents, buffering agents, polymers, antioxidants, preservatives, chelating agents, viscomodulators, tonicifiers, flavorants, colorants, odorants, opacifiers, suspending agents, binders, fillers, plasticizers, lubricants, and mixtures thereof.

In addition, an acid or a base may be incorporated into the composition to facilitate processing, to enhance stability, or for other reasons.

The pharmaceutical compositions of the present disclosure may comprise a therapeutically effective amount of the active agent (e.g., the proteinaceous heterodimer or the protein mixture of the present disclosure). A therapeutically effective amount is an amount of the pharmaceutical composition of the present disclosure capable of preventing and/or curing (at least partially) a condition or disorder (e.g., cancer) and/or any complications thereof in a subject suffering from or having a risk of developing the condition or disorder. The specific amount/concentration of the active agent comprised may vary according to the method of administration and the need of a patient, and can be determined based on, e.g., volume, viscosity, and/or body weight of a patient, etc. For example, an appropriate dose may be about 0.1 mg or 1 mg/kg/day to about 50 mg/kg/day; sometimes, the dose can be even higher. In some embodiments, the dose applied may be from about 3 mg/kg/day to about 3.5 mg/kg/day, from 3.5 mg/kg/day to about 7.2 mg/kg/day, from about 7.2 mg/kg/day to about 11.0 mg/kg/day, or from about 11.0 mg/kg/day to about 15.0 mg/kg/day. In some embodiments, the dose applied is from about 10 mg/kg/day to about 50 mg/kg/day, for example, from about 20 mg to about 50 mg per day, administered twice/day. It shall be understood that these specific doses may be conveniently adjusted by a skilled person in the art (e.g., a doctor or a pharmacist) based on conditions of a specific patient, formulation, and/or disease.

The proteinaceous heterodimer or the pharmaceutical composition of the present disclosure may also comprise one or more additional therapeutically active components. Such an additional therapeutically active component may be present separately in the composition, or may be attached to, conjugated to or associated with the proteinaceous heterodimer of the present disclosure.

### Medical Use and Methods of Treatment

In another aspect, the present disclosure provides use of the proteinaceous heterodimer or the protein mixture of the present disclosure in the manufacture of a medicament and/or a kit for preventing and/or treating a malignant effusion.

In the present disclosure, the medicament may further be used in combination with a chemotherapeutic agent, surgery, a catheter device for completing pleural drainage, and/or radiotherapy.

In some embodiments, the medicament and/or kit is used for specifically and/or preferentially inhibiting growth or differentiation of target cells (e.g., cancer cells) or killing target cells (e.g., cancer cells).

In another aspect, the present disclosure provides a method for inhibiting growth of a tumor or a tumor cell. The method may comprise contacting the tumor or tumor cell with an effective amount of the proteinaceous heterodimer according to the present disclosure, or the protein mixture according to the present disclosure. In some embodiments, the contacting occurs *in vitro.* In some embodiments, the contacting occurs *in vivo.*

In some embodiments, the contacting includes systemically or locally administering the proteinaceous heterodimer (e.g., a heterodimer protein), the protein mixture, the pharmaceutical composition, or the medicament of the present disclosure to a subject (e.g., a mammal). In some embodiments, the contacting includes administering the proteinaceous heterodimer (e.g., a heterodimer protein), the protein mixture, the pharmaceutical composition, or the medicament of the present disclosure directly at the site of a tumor. In some embodiments, the administering is conducted by oral administration, intravenous administration, intramuscular administration, in-situ administration at the site of a tumor, inhalation, rectal administration, vaginal administration, transdermal administration, or administration via subcutaneous repository.

In some embodiments, the tumor (e.g., cancer) or tumor cell (e.g., a cancer cell) is or is from a solid tumor. For example, the cancer may be selected from the group consisting of B cell lymphoma, lung cancer, bronchus cancer, colorectal cancer, prostate cancer, breast cancer, pancreatic cancer, stomach cancer, ovarian cancer, urinary bladder cancer, brain or central nervous system cancer, peripheral nervous system cancer, esophageal cancer, cervical cancer, melanoma, uterine or endometrial cancer, a cancer of the oral cavity or pharynx, liver cancer, kidney cancer, biliary tract cancer, small bowel or appendix cancer, salivary gland cancer, thyroid cancer, adrenal gland cancer, osteosarcoma, chondrosarcoma, liposarcoma, testicular cancer, and malignant fibrous histiocytoma.

In some embodiments, the cancer or cancer cell is within the body of a subject, e.g., a cancer or cancer cell within a human or in a non-human animal (e.g., a mammal).

In some embodiments, the mammal is a human. In some embodiments, the mammal is a mouse, a rat, a cat, a dog, a rabbit, a pig, a sheep, a horse, a cow, a goat, a gerbil, a hamster, a guinea pig, a monkey, or any other mammal. Many such mammals may be subjects that are known to the art as preclinical models for certain diseases or disorders, including solid tumors and/or other cancers (e.g., Talmadge et al., 2007 Am. J. Pathol. 170:793; Kerbel, 2003 Canc. Biol. Therap. 2(4 Suppl 1):S134; Man et al., 2007 Canc. Met. Rev. 26:737; Cespedes et al., 2006 Clin. TransL Oncol. 8:318).

### Method for Preparing Proteinaceous Heterodimers or Protein Mixtures

In another aspect, the present disclosure provides a method for producing a proteinaceous heterodimer or a protein mixture comprising the proteinaceous heterodimer, comprising (i) culturing the host cell of the present disclosure under conditions to effect expression of the proteinaceous heterodimer, and (ii) harvesting the expressed proteinaceous heterodimer or a protein mixture (such as the protein mixture of the present disclosure) comprising the expressed proteinaceous heterodimer.

In some embodiments, the method for producing a proteinaceous heterodimer comprises the following steps:
(1) providing a first member of the heterodimer, wherein the first member comprises a light chain and a heavy chain comprising a first Fc region, wherein the light chain is complexed with the heavy chain to form a targeting moiety exhibiting binding specificity to a tumor antigen;
(2) providing a second member of the heterodimer, and the second member is different from the first member, wherein the second member comprises a polypeptide comprising an immunoregulator fused to a second Fc region; the first member associates with the second member to form the heterodimer through complexation of the first Fc region with the second Fc region; and the first Fc region comprises a first modification and/or the second Fc region comprises a second modification, wherein the first modification and/or the second modification more effectively promotes heterodimerization between the first member and the second member than a knob-and-hole modification comprising a knob modification and a hole modification; and
3) obtaining the proteinaceous heterodimer.

In some embodiments, the method further comprises the steps of isolating and/or purifying the proteinaceous heterodimer or the protein mixture.

In some embodiments, the method further comprises the steps of transfecting/transforming host cells with polynucleotides/vectors encoding/expressing the heterodimer of the present disclosure, one or more members thereof, or fragments thereof.

In some embodiments, the proteinaceous heterodimer or the protein mixture of the present disclosure is produced by expressing a vector in a cell under conditions suitable for protein expression. In some embodiments, the proteinaceous heterodimer or the protein mixture of the present disclosure is produced in a single cell clone.

Factors that may vary among suitable conditions for protein expression include factors such as incubation time, temperature, and medium, and may depend on cell type and will be readily determined by one of ordinary skill in the art.

In some embodiments, during the process of producing the proteinaceous heterodimer or the protein mixture of the present disclosure, the host cells are grown in cultures, and in any apparatus that may be used to grow cultures, including fermenters. The cells may be grown as monolayers or attached to a surface. Alternatively, the host cells may be grown in suspension. The cells may be grown in a serum-free culture medium. The medium can be a commercially available medium, such as, but not limited to, Opti-CHO (Invitrogen, Catalogue #12681) supplemented with glutamine, such as 8 mM L-glutamine; RPMI 1640 medium, supplemented with 10% bovine calf serum, 10.5 ng/ml mIL⁻³ and L-glutamine; or 5% FCS medium.

### Animal Model and Progeny Thereof

In another aspect, the present disclosure further provides a method for constructing an animal model with a malignant effusion, which may comprise injecting a tumor cell into the thoracic cavity and/or the abdominal cavity of an animal.

In the present disclosure, a dose for the injection may be about 1E5 to about 2E6 cells injected per animal (where 1E5 is 1 × 10⁵, and 2E6 is 2 × 10⁶).

In the present disclosure, the dose for the injection may be about 1E5 to about 1.5E5 cells, about 1.5E5 to about 2E5 cells, about 2E5 to about 2.5E5 cells, about 2.5E5 to about 3E5 cells, about 3E5 to about 3.5E5 cells, about 3.5E5 to about 4E5 cells, about 4E5 to about 4.5E5 cells, about 4.5E5 to about 5E5 cells, about 5E5 to about 5.5E5 cells, about 5.5E5 to about 6E5 cells, about 6E5 to about 6.5E5 cells, about 6.5E5 to about 7E5 cells, about 7E5 to about 7.5E5 cells, about 7.5E5 to about 8E5 cells, about 8E5 to about 8.5E5 cells, about 8.5E5 to about 9E5 cells, about 9E5 to about 9.5E5 cells, about 9.5E5 to about 1E6 cells, about 1E6 to about 1.1E6 cells, about 1.1E6 to about 1.2E6 cells, about 1.2E6 to about 1.3E6 cells, about 1.3E6 to about 1.4E6 cells, about 1.4E6 to about 1.5E6 cells, about 1.5E6 to about 1.6E6 cells, about 1.6E6 to about 1.7E6 cells, about 1.7E6 to about 1.8E6 cells, about 1.8E6 to about 1.9E6 cells, or about 1.9E6 to about 2E6 cells injected per animal.

In the present disclosure, the animal may comprise a mammal. The animal may comprise a rodent. The animal may comprise a mouse. The mouse may be an inbred mouse or an outbred mouse. The mouse may be a normal mouse or an immunodeficient mouse. The mouse may include, but is not limited to, one or more of the following groups: C57BL/6 (including C57BL/6N and C57BL/6J), Balb/c, 615 mice, Chinese No. 1 (C-1) mice, Kunming mice (KM mice), NIH mice, LACA mice, ICR mice, nude mice, CBA/N mice, Beige mice, severe combined immunodeficient mice (SCID mice), non-obese diabetic/severe combined immunodeficient mice (NOD/SCID mice), NPG mice, NRG mice, BRG mice and NOD mice.

In the present disclosure, the injection may comprise intraperitoneal injection, intra-abdominal injection, and/or intrathoracic injection.

For example, the injection may comprise intraperitoneal injection, intra-abdominal injection, or intrathoracic injection into the animal.

For example, the injection may comprise intra-abdominal injection into the animal. In some embodiments, the injection may comprise intrathoracic injection into the animal described above.

In the present disclosure, the tumor cell may be derived from a solid tumor and/or a non-solid tumor. For example, the tumor may include, but is not limited to, Hodgkin's lymphoma, lung cancer, breast cancer, colon cancer, rectal cancer, liver cancer, pancreatic cancer, esophageal cancer, stomach cancer, prostate cancer, sarcomas (including osteosarcoma, chondrosarcoma, etc.), lymphoma, leukemia, glioma, melanoma, lipoma and/or thyroid cancer. For example, the tumor may comprise lung cancer, lymph cancer, liver cancer, stomach cancer, intestinal cancer, breast cancer, pancreatic cancer, sarcoma, ovarian cancer and/or mesothelioma.

For example, the tumor cell may comprise an H22-Luc cell, a B16-EGFR cell, an MC38-EGFR cell, a Pan02 mouse pancreatic cancer cell, an S180 mouse sarcoma cell and/or a Lewis-luc cell.

In the present disclosure, the tumor cell may cause a tumor in the animal.

The tumor cell may cause a tumor in C57BL/6 (including C57BL/6N and C57BL/6J), Balb/c, 615 mice, Chinese No. 1 (C-1) mice, Kunming mice (KM mice), NIH mice, LACA mice, ICR mice, nude mice, CBA/N mice, Beige mice, severe combined immunodeficient mice (SCID mice), non-obese diabetic/severe combined immunodeficient mice (NOD/SCID mice), NPG mice, NRG mice, BRG mice and/or NOD mice.

In the present disclosure, the tumor cell may cause a corresponding tumor in the animal. For example, BALB/c mice injected with H22-Luc cells may develop liver cancer, BALB/c mice injected with B16-EGFR cells may develop colon cancer, BALB/c mice injected with Pan02 mouse pancreatic cancer cells may develop pancreatic cancer, and C57BL/6 mice injected with Lewis-luc cells may develop lung cancer.

In the present disclosure, the tumor may metastasize to the pleura and/or the peritoneum of the animal and produce the malignant effusion. The malignant effusion may comprise malignant cells, blood and/or exudative fluid. The malignant effusion comprises malignant pleural effusion (MPE) and/or malignant ascites.

In the present disclosure, when the metastasized tumor belongs to a thoracic tumor, the malignant effusion may be produced in the pleura of the animal; for example, when the metastasized tumor is lung cancer, the malignant effusion, such as malignant pleural effusion (MPE), may be produced in the pleura of the animal. For example, the malignant pleural effusion is associated with non-small cell lung cancer.

In the present disclosure, when the metastasized tumor belongs to an abdominal tumor, the malignant effusion may be produced in the peritoneum of the animal; for example, when the metastasized tumor is colon cancer, pancreatic cancer, and/or liver cancer, the malignant effusion, such as malignant ascites, may be produced in the peritoneum of the animal.

In the present disclosure, the malignant cell may comprise a tumor cell. The malignant cell may metastasize from the primary tissue to other parts of the body through the circulatory system or lymphatic system.

The present disclosure further provides a tissue or organ, or a culture thereof, in an animal model with a malignant effusion prepared by the method described above.

The animal model with a malignant effusion or the progeny thereof prepared according to the method described above may specifically include: tissues or organs, or cultures thereof, from C57BL/6 (including C57BL/6N and C57BL/6J), Balb/c, 615 mice, Chinese No. 1 (C-1) mice, Kunming mice (KM mice), NIH mice, LACA mice, ICR mice, nude mice, CBA/N mice, Beige mice, severe combined immunodeficient mice (SCID mice), non-obese diabetic/severe combined immunodeficient mice (NOD/SCID mice), NPG mice, NRG mice, BRG mice and NOD mice with a malignant effusion.

In the present disclosure, the animal model with a malignant effusion or the progeny thereof prepared according to the method described above may specifically include: tissues or organs, or cultures thereof, from liver cancer BALB/c mice with a malignant effusion, colon cancer BALB/c mice with a malignant effusion, pancreatic cancer BALB/c mice with a malignant effusion, and lung cancer C57BL/6 mice with a malignant effusion.

For example, a tissue or organ, or a culture thereof, from liver cancer BALB/c mice with a malignant effusion may be obtained by the preparation according to the method described above. For example, a tissue or organ, or a culture thereof, from colon cancer BALB/c mice with a malignant effusion may be obtained by the preparation according to the method described above. For example, a tissue or organ, or a culture thereof, from pancreatic cancer BALB/c mice with a malignant effusion may be obtained by the preparation according to the method described above. For example, a tissue or organ, or a culture thereof, from lung cancer C57BL/6 mice with a malignant effusion may be obtained by the preparation according to the method described above.

The present disclosure further provides an animal model with a malignant effusion or progeny thereof prepared by the method described above.

The animal model with a malignant effusion or the progeny thereof prepared according to the method described above may specifically include: animal models or progeny thereof of C57BL/6 (including C57BL/6N and C57BL/6J), Balb/c, 615 mice, Chinese No.1 (C-1) mice, Kunming mice (KM mice), NIH mice, LACA mice, ICR mice, nude mice, CBA/N mice, Beige mice, severe combined immunodeficient mice (SCID mice), non-obese diabetic/severe combined immunodeficient mice (NOD/SCID mice), NPG mice, NRG mice, BRG mice and NOD mice with a malignant effusion. For example, the animal model with a malignant effusion or the progeny thereof prepared according to the method described above may specifically include: animal models or progeny thereof of liver cancer BALB/c mice with a malignant effusion, colon cancer BALB/c mice with a malignant effusion, pancreatic cancer BALB/c mice with a malignant effusion, and lung cancer C57BL/6 mice with a malignant effusion. For example, an animal model or progeny thereof of liver cancer BALB/c mice with a malignant effusion may be obtained by the preparation according to the method described above. For example, an animal model or progeny thereof of colon cancer BALB/c mice with a malignant effusion may be obtained by the preparation according to the method described above. For example, an animal model or progeny thereof of pancreatic cancer BALB/c mice with a malignant effusion may be obtained by the preparation according to the method described above. For example, an animal model or progeny thereof of lung cancer C57BL/6 mice with a malignant effusion may be obtained by the preparation according to the method described above.

The present disclosure further provides use of the tissue or organ, or the culture thereof described above, or the animal model with a malignant effusion or the progeny thereof in the screening, validation, evaluation, and/or research of an anti-tumor drug or as a model system for pharmacological, immunological, microbiological, and medical research.

The present disclosure includes the following embodiments:
1. Use of a proteinaceous heterodimer in the manufacture of a medicament for preventing and/or treating a malignant effusion, wherein the proteinaceous heterodimer comprises a first member and a second member different from the first member, wherein: the first member comprises a light chain and a heavy chain comprising a first Fc region, and the light chain is complexed with the heavy chain to form a targeting moiety exhibiting binding specificity to a tumor antigen; the second member comprises a polypeptide comprising an immunoregulator fused to a second Fc region; the first member associates with the second member to form the heterodimer through complexation of the first Fc region with the second Fc region; and the first Fc region comprises a first modification and/or the second Fc region comprises a second modification, wherein the first modification and/or the second modification more effectively promotes heterodimerization between the first member and the second member than a knob-and-hole modification comprising a knob modification and a hole modification.
2. The use according to embodiment 1, wherein the first modification is different from the knob modification or the hole modification, and/or the second modification is different from the knob modification or the hole modification.
3. The use according to any one of the preceding embodiments, wherein when expressed in a mammalian cell, a yield of the proteinaceous heterodimer is at least 10% higher than that of a reference protein, and the reference protein differs from the proteinaceous heterodimer in that the reference protein: i) comprises the knob modification in the first Fc region, ii) comprises the hole modification in the second Fc region, and iii) does not comprise the first modification and the second modification simultaneously.
4. The use according to embodiment 3, wherein the mammalian cell is selected from the group consisting of a HEK293 cell, a CHO cell, a COS-1 cell, and an NS0 cell.
5. The use according to any one of the preceding embodiments, wherein the first Fc region comprises the first modification, the second Fc region comprises the second modification, and neither the first modification nor the second modification is the same as the knob modification or the hole modification.
6. The use according to any one of the preceding embodiments, wherein the polypeptide comprised in the second member is a fusion protein, and a C-terminus of the immunoregulator is directly or indirectly fused to an N-terminus of the second Fc region to form the fusion protein.
7. The use according to any one of the preceding embodiments, wherein the tumor antigen is selected from the group consisting of EGFR, an EGFR mutant, HER2/neu, GPC3, FAP, Muc1, MUC5AC, and Mesothelin.
8. The use according to any one of the preceding embodiments, wherein the light chain of the targeting moiety contains CDRs comprising amino acid sequences that are at least 60%, 70%, 75%, 80%, 85%, 90%, 95%, 96%, 97%, 98%, 99%, or 100% identical to those comprised in the corresponding CDRs of a light chain of an antibody specifically directed to a tumor antigen.
9. The use according to any one of the preceding embodiments, wherein the light chain of the targeting moiety contains a variable region comprising an amino acid sequence that is at least 60%, 70%, 75%, 80%, 85%, 90%, 95%, 96%, 97%, 98%, 99%, or 100% identical to that comprised in the corresponding variable region of a light chain of an antibody specifically directed to a tumor antigen.
10. The use according to any one of the preceding embodiments, wherein the light chain of the targeting moiety contains an amino acid sequence that is at least 60%, 70%, 75%, 80%, 85%, 90%, 95%, 96%, 97%, 98%, 99%, or 100% identical to that comprised in the corresponding amino acid sequence of a light chain of an antibody specifically directed to a tumor antigen.
11. The use according to any one of the preceding embodiments, wherein the heavy chain of the targeting moiety contains CDRs comprising amino acid sequences that are at least 60%, 70%, 75%, 80%, 85%, 90%, 95%, 96%, 97%, 98%, 99%, or 100% identical to those comprised in the corresponding CDRs of a heavy chain of an antibody specifically directed to a tumor antigen.
12. The use according to any one of the preceding embodiments, wherein the heavy chain of the targeting moiety contains a variable region comprising an amino acid sequence that is at least 60%, 70%, 75%, 80%, 85%, 90%, 95%, 96%, 97%, 98%, 99%, or 100% identical to that comprised in the corresponding variable region of a heavy chain of an antibody specifically directed to a tumor antigen.
13. The use according to any one of the preceding embodiments, wherein the heavy chain of the targeting moiety contains an amino acid sequence that is at least 60%, 70%, 75%, 80%, 85%, 90%, 95%, 96%, 97%, 98%, 99%, or 100% identical to that comprised in the corresponding amino acid sequence of a heavy chain of an antibody specifically directed to a tumor antigen.
14. The use according to any one of the preceding embodiments, wherein the light chain of the targeting moiety contains CDRs comprising amino acid sequences that are at least 60%, 70%, 75%, 80%, 85%, 90%, 95%, 96%, 97%, 98%, 99%, or 100% identical to those comprised in the corresponding CDRs of a light chain of an antibody specifically directed to a tumor antigen; and the heavy chain of the targeting moiety contains CDRs comprising amino acid sequences that are at least 60%, 70%, 75%, 80%, 85%, 90%, 95%, 96%, 97%, 98%, 99%, or 100% identical to those comprised in the corresponding CDRs of a heavy chain of an antibody specifically directed to a tumor antigen.
15. The use according to any one of the preceding embodiments, wherein the light chain of the targeting moiety contains a variable region comprising an amino acid sequence that is at least 60%, 70%, 75%, 80%, 85%, 90%, 95%, 96%, 97%, 98%, 99%, or 100% identical to that comprised in the corresponding variable region of a light chain of an antibody specifically directed to a tumor antigen; and the heavy chain of the targeting moiety contains a variable region comprising an amino acid sequence that is at least 60%, 70%, 75%, 80%, 85%, 90%, 95%, 96%, 97%, 98%, 99%, or 100% identical to that comprised in the corresponding variable region of a heavy chain of an antibody specifically directed to a tumor antigen.
16. The use according to any one of the preceding embodiments, wherein the light chain of the targeting moiety contains an amino acid sequence that is at least 60%, 70%, 75%, 80%, 85%, 90%, 95%, 96%, 97%, 98%, 99%, or 100% identical to that comprised in the corresponding amino acid sequence of a light chain of an antibody specifically directed to a tumor antigen; and the heavy chain of the targeting moiety contains an amino acid sequence that is at least 60%, 70%, 75%, 80%, 85%, 90%, 95%, 96%, 97%, 98%, 99%, or 100% identical to that comprised in the corresponding amino acid sequence of a heavy chain of an antibody specifically directed to a tumor antigen.
17. The use according to any one of embodiments 8-16, wherein the antibody specifically directed to a tumor antigen is selected from the group consisting of anti-EGFR, anti-EGFR mutant, anti-HER2/neu, anti-GPC3, anti-FAP, anti-Muc1, anti-MUC5AC, and anti-Mesothelin.
18. The use according to any one of the preceding embodiments, wherein the immunoregulator augments an immune response.
19. The use according to any one of embodiments 1-17, wherein the immunoregulator reduces an immune response.
20. The use according to any one of the preceding embodiments, wherein the immunoregulator is a cytokine.
21. The use according to embodiment 20, wherein the immunoregulator is a cytokine selected from the group consisting of an interferon, an interleukin, a chemokine, a lymphokine, and a tumor necrosis factor.
22. The use according to embodiment 21, wherein the immunoregulator is an interferon selected from the group consisting of interferon alpha, interferon lambda, and interferon beta.
23. The use according to embodiment 21, wherein the immunoregulator is an interleukin, and the interleukin comprises interleukin 10, interleukin 2, and/or super interleukin 2.
24. The use according to any one of the preceding embodiments, wherein the first Fc region and the second Fc region are from an Fc region of an immunoglobulin, and the immunoglobulin is selected from the group consisting of IgG1, IgG2, IgG3, and IgG4.
25. The use according to embodiment 24, wherein the first Fc region and the second Fc region are from an Fc region of an immunoglobulin, and the immunoglobulin is a human IgG1.
26. The use according to any one of the preceding embodiments, wherein the second Fc region is fused in frame to the immunoregulator.
27. The use according to any one of the preceding embodiments, wherein the second Fc region is fused in frame to the immunoregulator via a linker.
28. The use according to any one of the preceding embodiments, wherein the polypeptide comprised in the second member comprises two or more immunoregulators, the two or more immunoregulators are fused in frame to each other and to the second Fc region, and wherein the two or more immunoregulators are located N-terminal to the second Fc region.
29. The use according to embodiment 28, wherein the two or more immunoregulators are the same.
30. The use according to any one of the preceding embodiments, wherein the first modification comprises an amino acid substitution at position T366, and an amino acid substitution at one or more positions selected from the group consisting of: Y349, F405, K409, D399, K360, Q347, K392, and S354, wherein the position of the amino acid is determined according to the EU index of the KABAT number.
31. The use according to embodiment 30, wherein the first modification comprises an amino acid substitution selected from the group consisting of Y349C, Y349D, D399S, F405K, K360E, K409A, K409E, Q347E, Q347R, S354D, K392D, and T366W.
32. The use according to embodiment 30 or 31, wherein the first modification comprises 2-5 amino acid substitutions.
33. The use according to any one of embodiments 30-32, wherein the first modification comprises an amino acid substitution at a group of positions selected from any of the following groups: 1) Y349 and T366; 2) Y349, T366, and F405; 3) Y349, T366, and K409; 4) Y349, T366, F405, K360, and Q347; 5) Y349, T366, F405, and Q347; 6) Y349, T366, K409, K360, and Q347; 7) Y349, T366, K409, and Q347; 8) T366, K409, and K392; 9) T366 and K409; 10) T366, K409, Y349, and S354; 11) T366 and F405; 12) T366, F405, and D399; and 13) T366, F405, Y349, and S354.
34. The use according to any one of embodiments 30-33, wherein the first modification comprises a group of amino acid substitutions selected from any of the following groups: 1) Y349C and T366W; 2) Y349C, T366W, and F405K; 3) Y349C, T366W, and K409E; 4) Y349C, T366W, and K409A; 5) Y349C, T366W, F405K, K360E, and Q347E; 6) Y349C, T366W, F405K, and Q347R; 7) Y349C, T366W, K409A, K360E, and Q347E; 8) Y349C, T366W, K409A, and Q347R; 9) T366W, K409A, and K392D; 10) T366W and K409A; 11) T366W, K409A, and Y349D; 12) T366W, K409A, Y349D, and S354D; 13) T366W and F405K; 14) T366W, F405K, and D399S; 15) T366W, F405K, and Y349D; and 16) T366W, F405K, Y349D, and S354D.
35. The use according to any one of embodiments 30-34, wherein the second modification comprises amino acid substitutions at positions T366, L368, and Y407, as well as an amino acid substitution at one or more positions selected from the group consisting of D356, D399, E357, F405, K360, K392, K409, and Q347, wherein the position of the amino acid is determined according to the EU index of the KABAT number.
36. The use according to embodiment 35, wherein the amino acid substitution comprised by the second modification is selected from the group consisting of D356C, D399S, E357A, F405K, K360E, K392D, K409A, L368A, L368G, Q347E, Q347R, T366S, Y407A, and Y407V.
37. The use according to embodiment 35 or 36, wherein the second modification comprises 4-6 amino acid substitutions.
38. The use according to any one of embodiments 35-37, wherein the second modification comprises an amino acid substitution at a group of positions selected from any of the following groups: 1) D356, T366, L368, Y407, and F405; 2) D356, T366, L368, and Y407; 3) D356, T366, L368, Y407, and Q347; 4) D356, T366, L368, Y407, K360, and Q347; 5) D356, T366, L368, Y407, F405, and Q347; 6) D356, T366, L368, Y407, F405, K360, and Q347; 7) T366, L368, Y407, D399, and F405; 8) T366, L368, Y407, and F405; 9) T366, L368, Y407, F405, and E357; 10) T366, L368, Y407, and K409; 11) T366, L368, Y407, K409, and K392; and 12) T366, L368, Y407, K409, and E357.
39. The use according to any one of embodiments 35-38, wherein the second modification comprises a group of amino acid substitutions selected from any of the following groups: 1) D356C, T366S, L368A, Y407V, and F405K; 2) D356C, T366S, L368A, and Y407V; 3) D356C, T366S, L368A, Y407V, and Q347R; 4) D356C, T366S, L368A, Y407V, 360E, and Q347E; 5) D356C, T366S, L368A, Y407V, F405K, and Q347R; 6) D356C, T366S, L368A, Y407V, F405K, K360E, and Q347E; 7) T366S, L368A, Y407V, D399S, and F405K; 8) T366S, L368G, Y407A, and F405K; 9) T366S, L368A, Y407V, F405K, and E357A; 10) T366S, L368A, Y407V, and K409A; 11) T366S, L368A, Y407V, K409A, and K392D; 12) T366S, L368G, Y407A, and K409A; and 13) T366S, L368A, Y407V, K409A, and E357A.
40. The use according to any one of the preceding embodiments, wherein the first Fc region comprises the first modification, the second Fc region comprises the second modification, and the first modification and the second modification comprise an amino acid substitution at a group of positions selected from any of the following groups: 1) the first modification: Y349 and T366; and the second modification: D356, T366, L368, Y407, and F405; 2) the first modification: Y349, T366, and F405; and the second modification: D356, T366, L368, and Y407; 3) the first modification: Y349, T366, and K409; and the second modification: D356, T366, L368, Y407, and F405; 4) the first modification: Y349, T366, F405, K360, and Q347; and the second modification: D356, T366, L368, Y407, and Q347; 5) the first modification: Y349, T366, F405, and Q347; and the second modification: D356, T366, L368, Y407, K360, and Q347; 6) the first modification: Y349, T366, K409, K360, and Q347; and the second modification: D356, T366, L368, Y407, F405, and Q347; 7) the first modification: Y349, T366, K409, and Q347; and the second modification: D356, T366, L368, Y407, F405, K360, and Q347; 8) the first modification: T366, K409, and K392; and the second modification: T366, L368, Y407, D399, and F405; 9) the first modification: T366 and K409; and the second modification: T366, L368, Y407, and F405; 10) the first modification: T366, K409, and Y349; and the second modification: T366, L368, Y407, F405, and E357; 11) the first modification: T366, K409, Y349, and S354; and the second modification: T366, L368, Y407, F405, and E357; 12) the first modification: T366 and F405; and the second modification: T366, L368, Y407, and K409; 13) the first modification: T366, F405, and D399; and the second modification: T366, L368, Y407, K409, and K392; 14) the first modification: T366, F405, and Y349; and the second modification: T366, L368, Y407, K409, and E357; and 15) the first modification: T366, F405, Y349, and S354; and the second modification: T366, L368, Y407, K409, and E357; wherein the position of the amino acid is determined according to the EU index of the KABAT number.
41. The use according to any one of the preceding embodiments, wherein the first Fc region comprises the first modification, and the second Fc region comprises the second modification, wherein the first modification and the second modification comprise a group of amino acid substitutions selected from any of the following groups: 1) the first modification: Y349C and T366W; and the second modification: D356C, T366S, L368A, Y407V, and F405K; 2) the first modification: Y349C, T366W, and F405K; and the second modification: D356C, T366S, L368A, and Y407V; 3) the first modification: Y349C, T366W, and K409E; and the second modification: D356C, T366S, L368A, Y407V, and F405K; 4) the first modification: Y349C, T366W, and K409A; and the second modification: D356C, T366S, L368A, Y407V, and F405K; 5) the first modification: Y349C, T366W, F405K, K360E, and Q347E; and the second modification: D356C, T366S, L368A, Y407V, and Q347R; 6) the first modification: Y349C, T366W, F405K, and Q347R; and the second modification: D356C, T366S, L368A, Y407V, K360E, and Q347E; 7) the first modification: Y349C, T366W, K409A, K360E, and Q347E; and the second modification: D356C, T366S, L368A, Y407V, F405K, and Q347R; 8) the first modification: Y349C, T366W, K409A, and Q347R; and the second modification: D356C, T366S, L368A, Y407V, F405K, K360E, and Q347E; 9) the first modification: T366W, K409A, and K392D; and the second modification: T366S, L368A, Y407V, D399S, and F405K; 10) the first modification: T366W and K409A; and the second modification: T366S, L368G, Y407A, and F405K; 11) the first modification: T366W, K409A, and Y349D; and the second modification: T366S, L368A, Y407V, F405K, and E357A; 12) the first modification: T366W, K409A, Y349D, and S354D; and the second modification: T366S, L368A, Y407V, F405K, and E357A; 13) the first modification: T366W and F405K; and the second modification: T366S, L368A, Y407V, and K409A; 14) the first modification: T366W, F405K, and D399S; and the second modification: T366S, L368A, Y407V, K409A, and K392D; 15) the first modification: T366W and F405K; and the second modification: T366S, L368G, Y407A, and K409A; 16) the first modification: T366W, F405K, and Y349D; and the second modification: T366S, L368A, Y407V, K409A, and E357A; and 17) the first modification: T366W, F405K, Y349D, and S354D; and the second modification: T366S, L368A, Y407V, K409A, and E357A; wherein the position of the amino acid is determined according to the EU index of the KABAT number.
42. The use according to embodiment 41, wherein the first Fc region comprises the first modification, the second Fc region comprises the second modification, the first modification comprises the amino acid substitutions T366W and K409A, and the second modification comprises the amino acid substitutions T366S, L368G, Y407A, and F405K, wherein the position of the amino acid is determined according to the EU index of the KABAT number.
43. The use according to any one of the preceding embodiments, wherein the targeting moiety specifically binds to EGFR, the light chain of the first member comprises CDR1-3, the amino acid sequence of the CDR1 is selected from SEQ ID NO: 101, the amino acid sequence of the CDR2 is selected from SEQ ID NO: 102, and the amino acid sequence of the CDR3 is selected from SEQ ID NO: 103.
44. The use according to any one of the preceding embodiments, wherein the targeting moiety specifically binds to EGFR, the light chain of the first member comprises a light chain variable region, and the amino acid sequence of the light chain variable region is selected from SEQ ID NO: 104.
45. The use according to any one of the preceding embodiments, wherein the targeting moiety specifically binds to EGFR, and the amino acid sequence of the light chain of the first member is selected from SEQ ID NO: 37.
46. The use according to any one of embodiments 43-45, wherein the targeting moiety specifically binds to EGFR, the heavy chain of the first member comprises CDR1-3, the amino acid sequence of the CDR1 is selected from SEQ ID NO: 105, the amino acid sequence of the CDR2 is selected from SEQ ID NO: 106, and the amino acid sequence of the CDR3 is selected from SEQ ID NO: 107.
47. The use according to any one of embodiments 43-45, wherein the targeting moiety specifically binds to EGFR, the heavy chain of the first member comprises a heavy chain variable region, and the amino acid sequence of the heavy chain variable region is selected from SEQ ID NO: 108.
48. The use according to any one of embodiments 43-45, wherein the targeting moiety specifically binds to EGFR, and the amino acid sequence of the heavy chain of the first member is selected from SEQ ID NO: 39.
49. The use according to any one of embodiments 1-42, wherein the targeting moiety specifically binds to an EGFR mutant, the light chain of the first member comprises CDR1-3, the amino acid sequence of the CDR1 is selected from SEQ ID NO: 109, the amino acid sequence of the CDR2 is selected from SEQ ID NO: 110, and the amino acid sequence of the CDR3 is selected from SEQ ID NO: 111.
50. The use according to any one of embodiments 1-42, wherein the targeting moiety specifically binds to an EGFR mutant, the light chain of the first member comprises a light chain variable region, and the amino acid sequence of the light chain variable region is selected from SEQ ID NO: 112.
51. The use according to any one of embodiments 1-42, wherein the targeting moiety specifically binds to an EGFR mutant, and the amino acid sequence of the light chain of the first member is selected from SEQ ID NO: 53.
52. The use according to any one of embodiments 49-51, wherein the targeting moiety specifically binds to an EGFR mutant, the heavy chain of the first member comprises CDR1-3, the amino acid sequence of the CDR1 is selected from SEQ ID NO: 113, the amino acid sequence of the CDR2 is selected from SEQ ID NO: 114, and the amino acid sequence of the CDR3 is selected from SEQ ID NO: 115.
53. The use according to any one of embodiments 49-51, wherein the targeting moiety specifically binds to an EGFR mutant, the heavy chain of the first member comprises a heavy chain variable region, and the amino acid sequence of the heavy chain variable region is selected from SEQ ID NO: 116.
54. The use according to any one of embodiments 49-51, wherein the targeting moiety specifically binds to an EGFR mutant, and the amino acid sequence of the heavy chain of the first member is selected from SEQ ID NO: 55.
55. The use according to any one of embodiments 1-42, wherein the targeting moiety specifically binds to HER2/neu, the light chain of the first member comprises CDR1-3, the amino acid sequence of the CDR1 is selected from SEQ ID NOs: 117 and 125, the amino acid sequence of the CDR2 is selected from SEQ ID NOs: 118 and 126, and the amino acid sequence of the CDR3 is selected from SEQ ID NOs: 119 and 127.
56. The use according to any one of embodiments 1-42, wherein the targeting moiety specifically binds to HER2/neu, the light chain of the first member comprises a light chain variable region, and the amino acid sequence of the light chain variable region is selected from SEQ ID NOs: 120 and 128.
57. The use according to any one of embodiments 1-42, wherein the targeting moiety specifically binds to HER2/neu, and the amino acid sequence of the light chain of the first member is selected from SEQ ID NOs: 45 and 49.
58. The use according to any one of embodiments 55-57, wherein the targeting moiety specifically binds to HER2/neu, the heavy chain of the first member comprises CDR1-3, the amino acid sequence of the CDR1 is selected from SEQ ID NOs: 121 and 129, the amino acid sequence of the CDR2 is selected from SEQ ID NOs: 122 and 130, and the amino acid sequence of the CDR3 is selected from SEQ ID NOs: 123 and 131.
59. The use according to any one of embodiments 55-57, wherein the targeting moiety specifically binds to HER2/neu, the antibody heavy chain of the first member comprises a heavy chain variable region, and the amino acid sequence of the heavy chain variable region is selected from SEQ ID NOs: 124 and 132.
60. The use according to any one of embodiments 55-57, wherein the targeting moiety specifically binds to HER2/neu, and the amino acid sequence of the heavy chain of the first member is selected from SEQ ID NOs: 47 and 51.
61. The use according to any one of embodiments 1-42, wherein the targeting moiety specifically binds to GPC3, the antibody light chain of the first member comprises CDR1-3, the amino acid sequence of the CDR1 is selected from SEQ ID NO: 133, the amino acid sequence of the CDR2 is selected from SEQ ID NO: 134, and the amino acid sequence of the CDR3 is selected from SEQ ID NO: 135.
62. The use according to any one of embodiments 1-42, wherein the targeting moiety specifically binds to GPC3, the antibody light chain of the first member comprises a light chain variable region, and the amino acid sequence of the light chain variable region is selected from SEQ ID NO: 136.
63. The use according to any one of embodiments 1-42, wherein the targeting moiety specifically binds to GPC3, and the amino acid sequence of the light chain of the first member is selected from SEQ ID NO: 57.
64. The use according to any one of embodiments 61-63, wherein the targeting moiety specifically binds to GPC3, the antibody heavy chain of the first member comprises CDR1-3, the amino acid sequence of the CDR1 is selected from SEQ ID NO: 137, the amino acid sequence of the CDR2 is selected from SEQ ID NO: 138, and the amino acid sequence of the CDR3 is selected from SEQ ID NO: 139.
65. The use according to any one of embodiments 61-63, wherein the targeting moiety specifically binds to GPC3, the antibody heavy chain of the first member comprises a heavy chain variable region, and the amino acid sequence of the heavy chain variable region is selected from SEQ ID NO: 140.
66. The use according to any one of embodiments 61-63, wherein the targeting moiety specifically binds to GPC3, and the amino acid sequence of the heavy chain of the first member is selected from SEQ ID NO: 59.
67. The use according to any one of embodiments 1-42, wherein the targeting moiety specifically binds to FAP, the antibody light chain of the first member comprises CDR1-3, the amino acid sequence of the CDR1 is selected from SEQ ID NO: 141, the amino acid sequence of the CDR2 is selected from SEQ ID NO: 142, and the amino acid sequence of the CDR3 is selected from SEQ ID NO: 143.
68. The use according to any one of embodiments 1-42, wherein the targeting moiety specifically binds to FAP, the antibody light chain of the first member comprises a light chain variable region, and the amino acid sequence of the light chain variable region is selected from SEQ ID NO: 144.
69. The use according to any one of embodiments 1-42, wherein the targeting moiety specifically binds to FAP, and the amino acid sequence of the light chain of the first member is selected from SEQ ID NO: 61.
70. The use according to any one of embodiments 67-69, wherein the targeting moiety specifically binds to FAP, the antibody heavy chain of the first member comprises CDR1-3, the amino acid sequence of the CDR1 is selected from SEQ ID NO: 145, the amino acid sequence of the CDR2 is selected from SEQ ID NO: 146, and the amino acid sequence of the CDR3 is selected from SEQ ID NO: 147.
71. The use according to any one of embodiments 67-69, wherein the targeting moiety specifically binds to FAP, the antibody heavy chain of the first member comprises a heavy chain variable region, and the amino acid sequence of the heavy chain variable region is selected from SEQ ID NO: 148.
72. The use according to any one of embodiments 67-69, wherein the targeting moiety specifically binds to FAP, and the amino acid sequence of the heavy chain of the first member is selected from SEQ ID NO: 63.
73. The use according to any one of embodiments 1-42, wherein the targeting moiety specifically binds to Muc1, the antibody light chain of the first member comprises CDR1-3, the amino acid sequence of the CDR1 is selected from SEQ ID NO: 149, the amino acid sequence of the CDR2 is selected from SEQ ID NO: 150, and the amino acid sequence of the CDR3 is selected from SEQ ID NO: 151.
74. The use according to any one of embodiments 1-42, wherein the targeting moiety specifically binds to Mucl, the antibody light chain of the first member comprises a light chain variable region, and the amino acid sequence of the light chain variable region is selected from SEQ ID NO: 152.
75. The use according to any one of embodiments 1-42, wherein the targeting moiety specifically binds to Mucl, and the amino acid sequence of the light chain of the first member is selected from SEQ ID NO: 65.
76. The use according to any one of embodiments 73-75, wherein the targeting moiety specifically binds to Muc1, the antibody heavy chain of the first member comprises CDR1-3, the amino acid sequence of the CDR1 is selected from SEQ ID NO: 153, the amino acid sequence of the CDR2 is selected from SEQ ID NO: 154, and the amino acid sequence of the CDR3 is selected from SEQ ID NO: 155.
77. The use according to any one of embodiments 73-75, wherein the targeting moiety specifically binds to Muc1, the antibody heavy chain of the first member comprises a heavy chain variable region, and the amino acid sequence of the heavy chain variable region is selected from SEQ ID NO: 156.
78. The use according to any one of embodiments 73-75, wherein the targeting moiety specifically binds to Muc1, and the amino acid sequence of the heavy chain of the first member is selected from SEQ ID NO: 67.
79. The use according to any one of embodiments 1-42, wherein the targeting moiety specifically binds to Mesothelin, the antibody light chain of the first member comprises CDR1-3, the amino acid sequence of the CDR1 is selected from SEQ ID NO: 165, the amino acid sequence of the CDR2 is selected from SEQ ID NO: 166, and the amino acid sequence of the CDR3 is selected from SEQ ID NO: 167.
80. The use according to any one of embodiments 1-42, wherein the targeting moiety specifically binds to Mesothelin, the antibody light chain of the first member comprises a light chain variable region, and the amino acid sequence of the light chain variable region is selected from SEQ ID NO: 168.
81. The use according to any one of embodiments 1-42, wherein the targeting moiety specifically binds to Mesothelin, and the amino acid sequence of the light chain of the first member is selected from SEQ ID NO: 73.
82. The use according to any one of embodiments 79-81, wherein the targeting moiety specifically binds to Mesothelin, the antibody heavy chain of the first member comprises CDR 1-3, the amino acid sequence of the CDR1 is selected from SEQ ID NO: 169, the amino acid sequence of the CDR2 is selected from SEQ ID NO: 170, and the amino acid sequence of the CDR3 is selected from SEQ ID NO: 171.
83. The use according to any one of embodiments 79-81, wherein the targeting moiety specifically binds to Mesothelin, the antibody heavy chain of the first member comprises a heavy chain variable region, and the amino acid sequence of the heavy chain variable region is selected from SEQ ID NO: 172.
84. The use according to any one of embodiments 79-81, wherein the targeting moiety specifically binds to Mesothelin, and the amino acid sequence of the heavy chain of the first member is selected from SEQ ID NO: 75.
85. The use according to any one of embodiments 1-42, wherein the targeting moiety specifically binds to MUC5AC, the antibody light chain of the first member comprises CDR1-3, the amino acid sequence of the CDR1 is selected from SEQ ID NO: 157, the amino acid sequence of the CDR2 is selected from SEQ ID NO: 158, and the amino acid sequence of the CDR3 is selected from SEQ ID NO: 159.
86. The use according to any one of embodiments 1-42, wherein the targeting moiety specifically binds to MUC5AC, the antibody light chain of the first member comprises a light chain variable region, and the amino acid sequence of the light chain variable region is selected from SEQ ID NO: 160.
87. The use according to any one of embodiments 1-42, wherein the targeting moiety specifically binds to MUC5AC, and the amino acid sequence of the light chain of the first member is selected from SEQ ID NO: 69.
88. The use according to any one of embodiments 85-87, wherein the targeting moiety specifically binds to MUC5AC, the antibody heavy chain of the first member comprises CDR1-3, the amino acid sequence of the CDR1 is selected from SEQ ID NO: 161, the amino acid sequence of the CDR2 is selected from SEQ ID NO: 162, and the amino acid sequence of the CDR3 is selected from SEQ ID NO: 163.
89. The use according to any one of embodiments 85-87, wherein the targeting moiety specifically binds to MUC5AC, the antibody heavy chain of the first member comprises a heavy chain variable region, and the amino acid sequence of the heavy chain variable region is selected from SEQ ID NO: 164.
90. The use according to any one of embodiments 85-87, wherein the targeting moiety specifically binds to MUC5AC, and the amino acid sequence of the heavy chain of the first member is selected from SEQ ID NO: 71.
91. The use according to any one of the preceding embodiments, wherein in the heavy chain of the first member, the amino acid sequence of the first Fc region is selected from SEQ ID NOs: 1, 4, 5, 6, 7, 9, 11, 13, 15, 17, 19, 21, 22, 24, 26, 27, and 29.
92. The use according to any one of the preceding embodiments, wherein the amino acid sequence of the immunoregulator comprised in the second member is selected from SEQ ID NOs: 173-180.
93. The use according to any one of the preceding embodiments, wherein the amino acid sequence of the second Fc region comprised in the second member is selected from SEQ ID NOs: 2, 3, 8, 10, 12, 14, 16, 18, 20, 23, 25, and 28.
94. The use according to any one of the preceding embodiments, wherein the amino acid sequence of the polypeptide comprised in the second member is selected from SEQ ID NOs: 77, 80, 82, 84, 86, 89, 91, and 97.
95. The use according to any one of the preceding embodiments, wherein the amino acid sequence of the light chain comprised in the first member is SEQ ID NOs: 37, 45, 49, 53, 57, 61, 65, 69, and 73, the amino acid sequence of the heavy chain comprised in the first member is SEQ ID NOs: 39, 47, 51, 55, 59, 63, 67, 71, and 75, and the amino acid sequence of the polypeptide comprised in the second member is SEQ ID NOs: 77, 80, 82, 84, 86, 89, 91, and 97.
96. The use according to any one of the preceding embodiments, wherein the knob-and-hole modification comprises a knob modification and a hole modification, wherein the knob modification comprises the amino acid substitutions Y349C and T366W, and the hole modification comprises the amino acid substitutions D356C, T366S, L368A, and Y407V, wherein the position of the amino acid is determined according to the EU index of the KABAT number.
97. The use according to any one of the preceding embodiments, wherein the medicament comprises a pharmaceutical composition comprising the proteinaceous heterodimer according to any one of the preceding embodiments and a pharmaceutically acceptable carrier.
98. The use according to any one of the preceding embodiments, wherein the medicament comprises a nucleic acid molecule encoding the proteinaceous heterodimer according to any one of the preceding embodiments.
99. The use according to the preceding embodiment 98, wherein the medicament comprises a vector comprising the nucleic acid molecule.
100. The use according to any one of the preceding embodiments 98-99, wherein the medicament comprises a cell comprising the nucleic acid molecule or the vector.
101. The use according to any one of the preceding embodiments 98-100, wherein the medicament comprises a protein mixture comprising: 1) the proteinaceous heterodimer according to any one of embodiments 1-96; 2) a first homodimer formed by two of the first member of the proteinaceous heterodimer; and 3) a second homodimer formed by two of the second member of the proteinaceous heterodimer; wherein the percentage of the proteinaceous heterodimer in the protein mixture is at least 50%.
102. The use according to any one of the preceding embodiments, wherein a patient suffering from the malignant effusion comprises a tumor patient.
103. The use according to the preceding embodiment 102, wherein the patient has symptoms comprising fatigue, vomiting, edema in the dorsum of the foot, loss of appetite, dyspnea, cough, chest pain, and/or abdominal pain.
104. The use according to any one of the preceding embodiments 102-103, wherein the tumor comprises a solid tumor and/or a non-solid tumor.
105. The use according to any one of the preceding embodiments 102-104, wherein the tumor comprises lung cancer, lymph cancer, liver cancer, stomach cancer, intestinal cancer, breast cancer, pancreatic cancer, ovarian cancer, and/or mesothelioma.
106. The use according to any one of the preceding embodiments, wherein the malignant effusion comprises malignant cells.
107. The use according to any one of the preceding embodiments, wherein the malignant effusion comprises blood and/or exudative fluid.
108. The use according to any one of the preceding embodiments 102-107, wherein the malignant effusion is associated with metastasis of the tumor to the pleura.
109. The use according to any one of the preceding embodiments 102-108, wherein the malignant effusion is associated with metastasis of the tumor to the peritoneum.
110. The use according to any one of the preceding embodiments, wherein the malignant effusion is caused by a tumor comprising a primary tumor of the lung, lymph, liver, stomach, colon, breast, ovarian cancer, pancreatic cancer, bile duct, esophagus, brain, nose, and/or prostate.
111. The use according to any one of the preceding embodiments, wherein the malignant effusion is caused by metastasis to the thoracic cavity of a tumor comprising a primary cancer of the lung, lymph, liver, stomach, intestine, breast, ovarian cancer, and/or mesothelioma.
112. The use according to any one of the preceding embodiments, wherein the malignant effusion is caused by metastasis to the abdominal cavity of a tumor comprising ovarian cancer, pancreatic cancer, and a primary cancer of the bile duct, esophagus, sarcoma, and/or liver.
113. The use according to any one of the preceding embodiments, wherein the malignant effusion comprises malignant pleural effusion (MPE) and/or malignant ascites.
114. The use according to any one of the preceding embodiments, wherein the malignant pleural effusion is associated with non-small cell lung cancer.
115. The use according to any one of the preceding embodiments, wherein the medicament is formulated for oral administration, intravenous administration, intramuscular administration, in-situ administration at the site of a tumor, inhalation, rectal administration, vaginal administration, transdermal administration, or administration via subcutaneous repository.
116. The use according to any one of the preceding embodiments, wherein the medicament is configured to comprise a drainage device.
117. The use according to any one of the preceding embodiments, wherein the medicament is capable of inhibiting growth of a tumor or a tumor cell.
118. A method for constructing an animal model with a malignant effusion, comprising injecting a tumor cell into the thoracic cavity and/or the abdominal cavity of an animal.
119. The method according to embodiment 118, wherein a dose for the injection is about 1 × 10⁵ to about 2 × 10⁶ cells injected per animal.
120. The method according to any one of embodiments 118-119, wherein the animal comprises a mouse.
121. The method according to any one of embodiments 118-120, wherein the injection comprises intraperitoneal injection, intra-abdominal injection, and/or intrathoracic injection.
122. The method according to any one of embodiments 118-121, wherein the tumor cell is derived from a solid tumor and/or a non-solid tumor.
123. The method according to embodiment 122, wherein the tumor comprises lung cancer, lymph cancer, liver cancer, stomach cancer, intestinal cancer, breast cancer, pancreatic cancer, sarcoma, ovarian cancer, and/or mesothelioma.
124. The method according to any one of embodiments 118-123, wherein the tumor cell comprises an H22-Luc cell, a B16-EGFR cell, an MC38-EGFR cell, a Pan02 mouse pancreatic cancer cell, an S180 mouse sarcoma cell, and/or a Lewis-luc cell.
125. The method according to any one of embodiments 118-124, wherein the tumor cell is capable of causing a tumor in the animal.
126. The method according to embodiment 125, wherein the tumor metastasizes to the pleura and/or the peritoneum of the animal and produces the malignant effusion.
127. The method according to any one of embodiments 118-126, wherein the malignant effusion comprises malignant cells.
128. The method according to any one of embodiments 118-127, wherein the malignant effusion comprises blood and/or exudative fluid.
129. The method according to any one of embodiments 118-128, wherein the malignant effusion comprises malignant pleural effusion (MPE) and/or malignant ascites.
130. The method according to any one of embodiments 118-129, wherein the malignant pleural effusion is associated with non-small cell lung cancer.
131. A tissue or organ, or a culture thereof, in an animal model with a malignant effusion prepared by the method according to any one of embodiments 118-120.
132. An animal model with a malignant effusion or progeny thereof prepared by the method according to any one of embodiments 118-130.
133. Use of the tissue or organ, or the culture thereof, or the animal model with a malignant effusion or the progeny thereof according to any one of embodiments 131-132 in the screening, validation, evaluation, and/or research of an anti-tumor drug or as a model system for pharmacological, immunological, microbiological, and medical research.

While preferred embodiments of the present disclosure have been shown and described herein, it will be obvious to those skilled in the art that such embodiments are provided by way of example only. Numerous variations, changes, and substitutions will now occur to those skilled in the art without departing from the present disclosure. It should be understood that various alternatives to the embodiments of the present disclosure described herein may be employed in practicing the present disclosure. It is intended that the following claims define the scope of the present disclosure and that methods and structures within the scope of these claims and their equivalents be covered thereby.

### EXAMPLES

The examples and formulations provided below further illustrate and exemplify the proteinaceous heterodimer of the present disclosure and methods for using and preparing same. It is to be understood that the scope of the present disclosure is not limited in any way by the scope of the following examples and preparations.

### Example 1: Modification and Preparation of Polypeptides

### 1.1 Determining amino acid modifications in the Fc regions

CH3-CH3 domain interface amino acid residues were determined for human IgG1. Wild-type human IgG1 comprises a first heavy chain (chain A) and a second heavy chain (chain B), the interface amino acids in each of chain A and chain B are shown in Table 1 below, and the position of the amino acid is determined according to the EU index of the KABAT number:

**Table 1. CH3-CH3 interface residues in wild-type human IgG1 antibody Fc regions (PDB No. 1DN2)**

| **Interface residues in chain A** | **Interface residues in chain B** |
|---|---|
| Gln347 | Lys360 |
| Val348 | Glu356 |
| Tyr349 | Ser354, Glu356, Glu357, Lys360 |
| Thr350 | Ser354, Glu356 |
| Leu351 | Leu351, Pro352, Pro353, Ser354, Thr366 |
| Pro352 | Leu351, Pro352 |
| Pro353 | Leu351 |
| Ser354 | Tyr349, Thr350, Leu351 |
| Glu356 | Val348, Tyr349, Thr350, Lys439 |
| Glu357 | Tyr349, Leu368, Lys370 |
| Lys360 | Gln347, Tyr349, Lys370 |
| Gln362 | Lys370 |
| Val363 | Lys370 |
| Ser364 | Leu368, Lys370, Tyr407 |
| Leu365 | Tyr407 |
| Thr366 | Leu351, Leu368, Tyr407 |
| Leu368 | Glu357, Ser364, Thr366, Lys409 |
| Lys370 | Glu357, Lys360, Gln362, Ser364, Lys409, Thr411 |
| Asn390 | Ser400 |
| Lys392 | Val397, Leu398, Asp399, Ser400, Phe405 |
| Thr393 | Val397 |
| Thr394 | Thr394, Val397, Phe405, Tyr407 |
| Pro395 | Pro395, Val397 |
| Val397 | Lys392, Thr393, Thr394, Pro395 |
| Leu398 | Lys392 |
| Asp399 | Lys392, Lys409, Thr411 |
| Ser400 | Asn390, Lys392 |
| Phe405 | Lys392, Thr394, Tyr407, Lys409 |
| Leu406 | Thr394 |
| Tyr407 | Thr366, Thr394, Phe405, Tyr407, Lys409 |
| Ser408 | Tyr407 |
| Lys409 | Leu368, Lys370, Asp399, Phe405, Tyr407 |
| Thr411 | Lys370, Asp399 |
| Lys439A | Glu356B |

Then, amino acid modifications (e.g., amino acid substitutions) were made to the interface residues to obtain the following groups of modifications (as shown in Table 2 below, the reference KH refers to the knob-and-hole modifications). In the present disclosure, chain A is also referred to as Fc9 or the first Fc region, and chain B is also referred to as Fc6 or the second Fc region:

**Table 2. Groups of amino acid modifications**

| **Group** | **Fc Chain** | **Modifications** | **SEQ ID NO** |
|---|---|---|---|
| Reference (KH) | A | Y349C+T366W | 1 |
| | B | D356C+T366S+L368A+Y407V | 2 |
| 1 | A | Y349C+T366W | 1 |
| | B | D356C+T366S+L368A+Y407V+F405K | 3 |
| 2 | A | Y349C+T366W+F405K | 4 |
| | B | D356C+T366S+L368A+Y407V | 2 |
| 3 | A | Y349C+T366W+K409E | 5 |
| | B | D356C+T366S+L368A+Y407V+F405K | 3 |
| 4 | A | Y349C+T366W+K409A | 6 |
| | B | D356C+T366S+L368A+Y407V+F405K | 3 |
| 5 | A | Y349C+T366W+F405K+K360E+Q347E | 7 |
| | B | D356C+T366S+L368A+Y407V+Q347R | 8 |
| 6 | A | Y349C+T366W+F405K+Q347R | 9 |
| | B | D356C+T366S+L368A+Y407V+K360E+Q347E | 10 |
| 7 | A | Y349C+T366W+K409A+K360E+Q347E | 11 |
| | B | D356C+T366S+L368A+Y407V+F405K+Q347R | 12 |
| 8 | A | Y349C+T366W+K409A+Q347R | 13 |
| | B | D356C+T366S+L368A+Y407V+F405K+K360E+ Q347E | 14 |
| 9 | A | T366W+K409A+K392D | 15 |
| | B | T366S+L368A+Y407V+D399S+F405K | 16 |
| 10 | A | T366W+K409A | 17 |
| | B | T366S+L368G+Y407A+F405K | 18 |
| 11 | A | T366W+K409A+Y349D | 19 |
| | B | T366S+L368A+Y407V+F405K+E357A | 20 |
| 12 | A | T366W+K409A+Y349D+S354D | 21 |
| | B | T366S+L368A+Y407V+F405K+E357A | 20 |
| 13 | A | T366W+F405K | 22 |
| | B | T366S+L368A+Y407V+K409A | 23 |
| 14 | A | T366W+F405K+D399S | 24 |
| | B | T366S+L368A+Y407V+K409A+K392D | 25 |
| 15 | A | T366W+F405K | 22 |
| | B | T366S+L368G+Y407A+K409A | 26 |
| 16 | A | T366W+F405K+Y349D | 27 |
| | B | T366S+L368A+Y407V+K409A+E357A | 28 |
| 17 | A | T366W+F405K+Y349D+S354D | 29 |
| | B | T366S+L368A+Y407V+K409A+E357A | 28 |

Subsequently, formation of heterodimer proteins comprising the groups of modifications listed in Table 2 above was examined using a ScFv-Fc/Fc system, as explained in detail below.

First of all, human immunoglobulin gamma1 (IgG1) constant region amino acid sequence was obtained from the database Uniprot (P01857), to get wild-type human IgG1-Fc region amino acid sequence (SEQ ID NO: 30). The polynucleotide fragment encoding wild-type human IgG1-Fc was obtained by RT-PCR from human PBMC total RNA (SEQ ID NO: 31, named as the Fc gene fragment). A polynucleotide fragment encoding a mouse kappaIII signal peptide (SEQ ID NO: 32) was added to the 5' end of the Fc gene by overlapping PCR, and then subcloned into the vector pcDNA4 (Invitrogen, Cat V86220), to obtain a recombinant expression vector for expressing human IgG1-Fc in mammalian cells.

In some cases, a polypeptide encoding a variable region of a camel single domain antibody (VhH) was fused to the N terminal of the Fc gene fragment to obtain a fusion gene fragment (as set forth in SEQ ID NO: 33) encoding the fusion protein VhH-Fc (as set forth in SEQ ID NO: 34). It was then subcloned into the vector pcDNA4 (Invitrogen, Cat V86220), to obtain a recombinant expression vector for expressing the fusion protein VhH-Fc in mammalian cells.

A nucleic acid molecule encoding a ScFv-Fc fusion protein (SEQ ID NO: 35) was synthesized, wherein the ScFv refers to an anti-Her2 single chain antibody, and the amino acid sequence of the ScFv-Fc fusion protein is as set forth in SEQ ID NO: 36. The ScFv-Fc gene fragment was then subcloned into the vector pcDNA4 (Invitrogen, Cat V86220), to obtain a recombinant expression vector for expressing the ScFv-Fc fusion protein in mammalian cells.

Then, the amino acid modifications as listed in Table 2 above were respectively introduced into the ScFv-Fc (groups KH and 1-17), the VhH-Fc (groups 9-12, 14, 15 and 17), and the Fc gene fragment (groups KH and 1-8) by overlapping PCR, wherein chain A refers to the Fc region in ScFv-Fc and chain B refers to the independent Fc region or the Fc region in VhH-Fc. The gene fragments with amino acid modifications were respectively subcloned into the vector pcDNA4 (Invitrogen, Cat V86220), to obtain recombinant expression vectors for expressing the modified ScFv-Fc fusion proteins, the modified Fc proteins, and the modified VhH-Fc fusion proteins in mammalian cells. Then, HEK293 cells (ATCC CRL-1573TM) in suspension culture were transfected with the constructed expression vectors with PEI. For each group, the expression vector expressing the A chain (ScFv-Fc fusion protein) and that expressing the B chain (Fc protein or VhH-Fc fusion protein) were co-transfected at a ratio of 1:1. After culturing for 5-6 days, the supernatant of the transient expression products was collected, and the expression products comprising corresponding proteinaceous heterodimers were preliminarily purified using Protein A affinity chromatography. Each of the preliminarily purified expression products comprises the homodimer protein ScFv-Fc/ScFv-Fc, the homodimer protein Fc/Fc (or the homodimer protein VhH-Fc/VhH-Fc) and the heterodimer protein ScFv-Fc/Fc (or the heterodimer protein ScFv-Fc/VhH-Fc), present in various percentages, respectively. Since the molecular weights of these proteins (i.e., the homodimers and the heterodimers) are different, their corresponding percentage could be determined according to corresponding band intensities reflected on non-reduced SDS-PAGE gels. The intensities were quantified and the results are summarized in Tables 3-6 below.

**Table 3. Percentage of homodimer proteins and heterodimer proteins in expression products**

| Group | ScFv-Fc homodimer (%) | ScFv-Fc/Fc heterodimer (%) | Fc homodimer (%) |
|---|---|---|---|
| KH | 29 | 51 | 20 |
| 1 | 24 | 58 | 18 |
| 2 | 10 | 70 | 20 |
| 3 | 25 | 57 | 18 |
| 4 | 10 | 77 | 13 |

**Table 4. Percentage of homodimer proteins and heterodimer proteins in expression products**

| Group | ScFv-Fc homodimer (%) | ScFv-Fc/Fc heterodimer (%) | Fc homodimer (%) |
|---|---|---|---|
| 2 | 17 | 60 | 23 |
| 5 | 14 | 72 | 14 |
| 6 | 14 | 62 | 24 |
| 4 | 21 | 69 | 10 |
| 7 | 24 | 64 | 12 |
| 8 | 21 | 71 | 8 |

**Table 5. Percentage of homodimer proteins and heterodimer proteins in expression products**

| Group | ScFv-Fc homodimer (%) | ScFv-Fc/VhH-Fc heterodimer (%) | VhH-Fc homodimer (%) |
|---|---|---|---|
| 4 | 13 | 68 | 19 |
| 9 | 7 | 80 | 13 |
| 10 | 15 | 85 | 0 |
| 11 | 14 | 83 | 3 |
| 12 | 10 | 84 | 6 |

**Table 6. Percentage of homodimer proteins and heterodimer proteins in expression products**

| Group | ScFv-Fc homodimer (%) | ScFv-Fc/VhH-Fc heterodimer (%) | VhH-Fc homodimer (%) |
|---|---|---|---|
| 2 | 9 | 64 | 27 |
| 14 | 6 | 81 | 13 |
| 15 | 5 | 88 | 7 |
| 17 | 9 | 84 | 7 |

As can be seen from Tables 3-6 above, all groups of modifications promoted heterodimer formation more effectively than the reference modification knob-and-hole. For illustrative purposes, the modifications in group 10(modifications in chain A: T366W+K409A; modifications in chain B: T366S+L368G+Y407A+F405K) were used in the following examples to generate the proteinaceous heterodimers or the protein mixtures of the present disclosure.

### 1.2 Preparation of Anti-EGFR (Cetuximab)

Full length amino acid sequences of the heavy chain and light chain of Cetuximab (also known as Erbitux or Erb, which is an antibody against epidermal growth factor receptor EGFR) were obtained, and corresponding DNA sequences encoding these amino acid sequences were obtained using online tool DNAworks (helixweb.nih.gov/dnaworks/). Then, nucleic acid molecules encoding the light chain of Cetuximab (Erb-LC) were synthesized. The amino acid sequence of Erb-LC is as set forth in SEQ ID NO: 37, and the corresponding polynucleotide sequence encoding it is as set forth in SEQ ID NO: 38. Then, point mutations (T366W and K409A) were introduced into the polynucleotide sequences encoding the Fc region of Cetuximab heavy chain gene, and nucleic acid molecules encoding the modified Cetuximab heavy chain were synthesized (referred to herein as erb-Fc9). The corresponding polypeptide encoding it was named as Erb-Fc9. The amino acid sequence of Erb-Fc9 is as set forth in SEQ ID NO: 39, and the polynucleotide sequence encoding it is as set forth in SEQ ID NO: 40. In another experiment, point mutations (T366S, L368G, Y407A and F405K) were introduced into the polynucleotide sequences encoding the Fc region of Cetuximab heavy chain gene, and nucleic acid molecules encoding the modified Cetuximab heavy chain were synthesized (referred to herein as erb-Fc6), the corresponding polypeptide encoding it was named as Erb-Fc6. The amino acid sequence of Erb-Fc6 is as set forth in SEQ ID NO: 41, and the polynucleotide sequence encoding it is as set forth in SEQ ID NO: 42.

In another experiment, to prepare the reference protein, point mutations (Y349C and T366W) were introduced into the polynucleotide sequences encoding the Fc region of Cetuximab heavy chain gene, and nucleic acid molecules encoding the modified Cetuximab heavy chain were synthesized (referred to herein as erb-knob). The corresponding polypeptide encoding it was named as Erb-Knob. The amino acid sequence of Erb-Knob is as set forth in SEQ ID NO: 43, and the polynucleotide sequence encoding it is as set forth in SEQ ID NO: 44.

### 1.3 Preparation of Anti-HER2 (Trastuzumab)

Full length amino acid sequences of the heavy chain and light chain of Trastuzumab were obtained according to US patent US7879325B2 (incorporated herein by reference). Then, corresponding DNA sequences encoding these amino acid sequences were obtained using online tool DNAworks (helixweb.nih.gov/dnaworks/). Nucleic acid molecules encoding the light chain of Trastuzumab (T-LC) were then synthesized. The amino acid sequence of T-LC is as set forth in SEQ ID NO: 45, and the corresponding polynucleotide sequence encoding it is as set forth in SEQ ID NO: 46. Then, point mutations (T366W and K409A) were introduced into the polynucleotide sequences encoding the Fc region of Trastuzumab heavy chain gene, and nucleic acid molecules encoding the modified Trastuzumab heavy chain were synthesized (referred to herein as t-Fc9). The corresponding polypeptide encoding it was named as T-Fc9. The amino acid sequence of T-Fc9 is as set forth in SEQ ID NO: 47, and the polynucleotide sequence encoding it is as set forth in SEQ ID NO: 48.

### 1.4 Preparation of Anti-HER2 (Pertuzumab)

Full length amino acid sequences of the heavy chain and light chain of Pertuzumab were obtained according to US7879325B2 (incorporated herein by reference). Then, corresponding DNA sequences encoding these amino acid sequences were obtained using online tool DNAworks (helixweb.nih.gov/dnaworks/). Nucleic acid molecules encoding the light chain of Pertuzumab (P-LC) were then synthesized. The amino acid sequence of P-LC is as set forth in SEQ ID NO: 49, and the corresponding polynucleotide sequence encoding it is as set forth in SEQ ID NO: 50. Then, point mutations (T366W and K409A) were introduced into the polynucleotide sequences encoding the Fc region of Pertuzumab heavy chain gene, and nucleic acid molecules encoding the modified Pertuzumab heavy chain were synthesized (referred to herein as p-Fc9). The corresponding polypeptide encoding it was named as P-Fc9. The amino acid sequence of P-Fc9 is as set forth in SEQ ID NO: 51, and the polynucleotide sequence encoding it is as set forth in SEQ ID NO: 52.

### 1.5 Preparation of Anti-EGFR mutant (Mab806)

Full length amino acid sequences of the heavy chain and light chain of Mab806 were obtained according to US7589180B2 (incorporated herein by reference). Then, corresponding DNA sequences encoding these amino acid sequences were obtained using online tool DNAworks (helixweb.nih.gov/dnaworks/). Nucleic acid molecules encoding the light chain of Mab806 (Mab806-LC) were then synthesized. The amino acid sequence of Mab806-LC is as set forth in SEQ ID NO: 53, and the corresponding polynucleotide sequence encoding it is as set forth in SEQ ID NO: 54. Then, point mutations (T366W and K409A) were introduced into the polynucleotide sequences encoding the Fc region of Mab806 heavy chain gene, and nucleic acid molecules encoding the modified Mab806 heavy chain were synthesized (referred to herein as mab806-Fc9), the corresponding polypeptide encoding it was named as Mab806-Fc9. The amino acid sequence of Mab806-Fc9 is as set forth in SEQ ID NO: 55, and the polynucleotide sequence encoding it is as set forth in SEQ ID NO: 56.

### 1.6 Preparation of Anti-GPC3 (codrituzumab)

Full length amino acid sequences of the heavy chain and light chain of codrituzumab were obtained according to US7919086B2 (incorporated herein by reference). Then, corresponding DNA sequences encoding these amino acid sequences were obtained using online tool DNAworks (helixweb.nih.gov/dnaworks/). Nucleic acid molecules encoding the light chain of codrituzumab (C-mab-LC) were then synthesized. The amino acid sequence of C-mab-LC is as set forth in SEQ ID NO: 57, and the corresponding polynucleotide sequence encoding it is as set forth in SEQ ID NO: 58. Then, point mutations (T366W and K409A) were introduced into the polynucleotide sequences encoding the Fc region of codrituzumab heavy chain gene, and nucleic acid molecules encoding the modified codrituzumab heavy chain were synthesized (referred to herein as C-mab-Fc9). The corresponding polypeptide encoding it was named as C-mab-Fc9. The amino acid sequence of C-mab-Fc9 is as set forth in SEQ ID NO: 59, and the polynucleotide sequence encoding it is as set forth in SEQ ID NO: 60.

### 1.7 Preparation of Anti-FAP(28H1)

Full length amino acid sequences of the heavy chain and light chain of 28H1 were obtained according to US20120128591A1 (incorporated herein by reference). Then, corresponding DNA sequences encoding these amino acid sequences were obtained using online tool DNAworks (helixweb.nih.gov/dnaworks/). Nucleic acid molecules encoding the light chain of 28H1 (28H1-LC) were then synthesized. The amino acid sequence of 28H1-LC is as set forth in SEQ ID NO: 61, and the corresponding polynucleotide sequence encoding it is as set forth in SEQ ID NO: 62. Then, point mutations (T366W and K409A) were introduced into the polynucleotide sequences encoding the Fc region of 28H1 heavy chain gene, and nucleic acid molecules encoding the modified 28H1 heavy chain were synthesized (referred to herein as 28H1-Fc9). The corresponding polypeptide encoding it was named as 28H1-Fc9. The amino acid sequence of 28H1-Fc9 is as set forth in SEQ ID NO: 63, and the polynucleotide sequence encoding it is as set forth in SEQ ID NO: 64.

### 1.8 Preparation of Anti-Muc1(5E5)

Full length amino acid sequences of the heavy chain and light chain of 5E5 were obtained according to US8440798B2 (incorporated herein by reference). Then, corresponding DNA sequences encoding these amino acid sequences were obtained using online tool DNAworks (helixweb.nih.gov/dnaworks/). Nucleic acid molecules encoding the light chain of 5E5 (5E5-LC) were then synthesized. The amino acid sequence of 5E5-LC is as set forth in SEQ ID NO: 65, and the corresponding polynucleotide sequence encoding it is as set forth in SEQ ID NO: 66. Then, point mutations (T366W and K409A) were introduced into the polynucleotide sequences encoding the Fc region of 5E5 heavy chain gene, and nucleic acid molecules encoding the modified 5E5 heavy chain were synthesized (referred to herein as 5E5-Fc9). The corresponding polypeptide encoding it was named as 5E5-Fc9. The amino acid sequence of 5E5-Fc9 is as set forth in SEQ ID NO: 67, and the polynucleotide sequence encoding it is as set forth in SEQ ID NO: 68.

### 1.9 Preparation of Anti-MUC5AC(ensituximab)

Full length amino acid sequences of the heavy chain and light chain of ensituximab were obtained according to WO2006113546A2 (incorporated herein by reference). Then, corresponding DNA sequences encoding these amino acid sequences were obtained using online tool DNAworks (helixweb.nih.gov/dnaworks/). Nucleic acid molecules encoding the light chain of ensituximab(E-mab-LC) were then synthesized. The amino acid sequence of E-mab-LC is as set forth in SEQ ID NO: 69, and the corresponding polynucleotide sequence encoding it is as set forth in SEQ ID NO: 70. Then, point mutations (T366W and K409A) were introduced into the polynucleotide sequences encoding the Fc region of ensituximab heavy chain gene, and nucleic acid molecules encoding the modified ensituximab heavy chain were synthesized (referred to herein as E-mab-Fc9), the corresponding polypeptide encoding it was named as E-mab-FC9. The amino acid sequence of E-mab-Fc9 is as set forth in SEQ ID NO: 71, and the polynucleotide sequence encoding it is as set forth in SEQ ID NO: 72.

### 1.10 Preparation of Anti-Mesothelin(amatuximab)

Full length amino acid sequences of the heavy chain and light chain of amatuximab were obtained from http://www.imgt.org/mAb-DB/index. Then, corresponding DNA sequences encoding these amino acid sequences were obtained using online tool DNAworks (helixweb.nih.gov/dnaworks/). Nucleic acid molecules encoding the light chain of amatuximab(A-mab-LC) were then synthesized. The amino acid sequence of A-mab-LC is as set forth in SEQ ID NO: 73, and the corresponding polynucleotide sequence encoding it is as set forth in SEQ ID NO: 74. Then, point mutations (T366W and K409A) were introduced into the polynucleotide sequences encoding the Fc region of amatuximab heavy chain gene, and nucleic acid molecules encoding the modified xxx heavy chain were synthesized (referred to herein as A-mab-Fc9), the corresponding polypeptide encoding it was named as A-mab-FC9. The amino acid sequence of A-mab-Fc9 is as set forth in SEQ ID NO: 75, and the polynucleotide sequence encoding it is as set forth in SEQ ID NO: 76.

### 1.11 Preparation of muIFNa4-Fc6

First of all, sequence information of mouse interferon α4(IFNa4) (NM_010504.2) was obtained from the National Center for Biotechnology Information (NCBI), and the full length polynucleotide sequences encoding it were obtained. Then, amino acid sequences of human IgG1-Fc (i.e., residue 104 to residue 330 of P01857) were obtained according to the amino acid sequences of human immunoglobulin gamma1 (IgG1) constant region (P01857) from the protein database Uniprot. Afterwards, point mutations (T366S, L368G, Y407A and F405K) were introduced into the IgG1-Fc fragment, and the polypeptide obtained thereby is referred to as Fc6. Then, a linker sequence "GSGGG" (SEQ ID NO: 79) was added to the N-terminus of the Fc6, to obtain linker-Fc6. The corresponding DNA sequence encoding it was then designed using online tool DNAworks (helixweb.nih.gov/dnaworks/). Polynucleotide sequences encoding mouse IFNa4 were added to the 5' end of the polynucleotide sequences encoding the linker-Fc6, thereby obtaining and synthesizing a polynucleotide sequence encoding the fusion protein muIFNa4-Fc6. The amino acid sequence of muIFNa4-Fc6 is as set forth in SEQ ID NO: 77, and the polynucleotide sequence encoding it is as set forth in SEQ ID NO: 78.

### 1.12 Preparation of huIFNa2-Fc6

First of all, sequence information of human interferon α2 (IFNa2) (NM_000605.3) was obtained from the National Center for Biotechnology Information (NCBI), and the full length polynucleotide sequences encoding it were obtained. Then, amino acid sequences of human IgG1-Fc (i.e., residue 104 to residue 330 of P01857) were obtained according to the amino acid sequences of human immunoglobulin gamma1 (IgG1) constant region (P01857) from the protein database Uniprot. Afterwards, point mutations (T366S, L368G, Y407A and F405K) were introduced into the IgG1-Fc fragment, and the polypeptide obtained thereby is referred to as Fc6. Then, a linker sequence "GSGGG" (SEQ ID NO: 79) was added to the N-terminus of the Fc6, to obtain linker-Fc6. The corresponding DNA sequence encoding it was then designed using online tool DNAworks (helixweb.nih.gov/dnaworks/). Polynucleotide sequences encoding human IFNa2 were added to the 5' end of the polynucleotide sequences encoding the linker-Fc6, thereby obtaining and synthesizing a polynucleotide sequence encoding the fusion protein huIFNa2-Fc6. The amino acid sequence of huIFNa2-Fc6 is as set forth in SEQ ID NO: 80, and the polynucleotide sequence encoding it is as set forth in SEQ ID NO: 81.

### 1.13 Preparation of muIFNb-Fc6

First of all, sequence information of mouse interferon β (IFNβ) (NM_005018.2) was obtained from the National Center for Biotechnology Information (NCBI), and the full length polynucleotide sequences encoding it were obtained. Then, amino acid sequences of human IgG1-Fc (i.e., residue 104 to residue 330 of P01857) were obtained according to the amino acid sequences of human immunoglobulin gamma1 (IgG1) constant region (P01857) from the protein database Uniprot. Afterwards, point mutations (T366S, L368G, Y407A and F405K) were introduced into the IgG1-Fc fragment, and the polypeptide obtained thereby is referred to as Fc6. Then, a linker sequence "GSGGG" (SEQ ID NO: 79) was added to the N-terminus of the Fc6, to obtain linker-Fc6. The corresponding DNA sequence encoding it was then designed using online tool DNAworks (helixweb.nih.gov/dnaworks/). Polynucleotide sequences encoding mouse IFNβ were added to the 5' end of the polynucleotide sequences encoding the linker-Fc6, thereby obtaining and synthesizing a polynucleotide sequence encoding the fusion protein muIFNb-Fc6. The amino acid sequence of muIFNb-Fc6 is as set forth in SEQ ID NO: 82, and the polynucleotide sequence encoding it is as set forth in SEQ ID NO: 83.

### 1.14 Preparation of huIFNb-Fc6

First of all, sequence information of human interferon β (IFNβ) (EF064725.1) was obtained from the National Center for Biotechnology Information (NCBI), and the full length polynucleotide sequences encoding it were obtained. Then, amino acid sequences of human IgG1-Fc (i.e., residue 104 to residue 330 of P01857) were obtained according to the amino acid sequences of human immunoglobulin gamma1 (IgG1) constant region (P01857) from the protein database Uniprot. Afterwards, point mutations (T366S, L368G, Y407A and F405K) were introduced into the IgG1-Fc fragment, and the polypeptide obtained thereby is referred to as Fc6. Then, a linker sequence "GSGGG" (SEQ ID NO: 79) was added to the N-terminus of the Fc6, to obtain linker-Fc6. The corresponding DNA sequence encoding it was then designed using online tool DNAworks (helixweb.nih.gov/dnaworks/). Polynucleotide sequences encoding human IFNβ were added to the 5' end of the polynucleotide sequences encoding the linker-Fc6, thereby obtaining and synthesizing a polynucleotide sequence encoding the fusion protein huIFNb-Fc6. The amino acid sequence of huIFNb-Fc6 is as set forth in SEQ ID NO: 84, and the polynucleotide sequence encoding it is as set forth in SEQ ID NO: 85.

### 1.15 Preparation of huIFNL-Fc6

First of all, sequence information of human interferon λ (IFNL) (BC117482.1) was obtained from the National Center for Biotechnology Information (NCBI), and the full length polynucleotide sequences encoding it were obtained. Then, amino acid sequences of human IgG1-Fc (i.e., residue 104 to residue 330 of P01857) were obtained according to the amino acid sequences of human immunoglobulin gamma1 (IgG1) constant region (P01857) from the protein database Uniprot. Afterwards, point mutations (T366S, L368G, Y407A and F405K) were introduced into the IgG1-Fc fragment, and the polypeptide obtained thereby is referred to as Fc6. Then, a linker sequence "GSGGG" (SEQ ID NO: 79) was added to the N-terminus of the Fc6, to obtain linker-Fc6. The corresponding DNA sequence encoding it was then designed using online tool DNAworks (helixweb.nih.gov/dnaworks/). Polynucleotide sequences encoding human IFNL were added to the 5' end of the polynucleotide sequences encoding the linker-Fc6, thereby obtaining and synthesizing a polynucleotide sequence encoding the fusion protein huIFNL-Fc6. The amino acid sequence of huIFNL-Fc6 is as set forth in SEQ ID NO: 86, and the polynucleotide sequence encoding it is as set forth in SEQ ID NO: 87.

### 1.16 Preparation of huIL10-Fc6

First of all, sequence information of human interleukin 10 (huIL10) (P22301) was obtained from the National Center for Biotechnology Information (NCBI), and the full length polynucleotide sequences encoding it were obtained. Then, amino acid sequences of human IgG1-Fc (i.e., residue 104 to residue 330 of P01857) were obtained according to the amino acid sequences of human immunoglobulin gamma1 (IgG1) constant region (P01857) from the protein database Uniprot. Afterwards, point mutations (T366S, L368G, Y407A and F405K) were introduced into the IgG1-Fc fragment, and the polypeptide obtained thereby is referred to as Fc6. Then, a linker sequence "(GGGGS)₃" (SEQ ID NO: 88) was added to the N-terminus of the Fc6, to obtain linker-Fc6. The corresponding DNA sequence encoding it was then designed using online tool DNAworks (helixweb.nih.gov/dnaworks/). Polynucleotide sequences encoding huIL10 were added to the 5' end of the polynucleotide sequences encoding the linker-Fc6, thereby obtaining and synthesizing a polynucleotide sequence encoding the fusion protein huIL10-Fc6. The amino acid sequence of huIL10-Fc6 is as set forth in SEQ ID NO: 89, and the polynucleotide sequence encoding it is as set forth in SEQ ID NO: 90.

### 1.17 Preparation of (huIL10)₂-Fc6

First of all, sequence information of human interleukin 10 (huIL10) (P22301) was obtained from the National Center for Biotechnology Information (NCBI), and the full length polynucleotide sequences encoding it were obtained. Then, amino acid sequences of human IgG1-Fc (i.e., residue 104 to residue 330 of P01857) were obtained according to the amino acid sequences of human immunoglobulin gamma1 (IgG1) constant region (P01857) from the protein database Uniprot. Afterwards, point mutations (T366S, L368G, Y407A and F405K) were introduced into the IgG1-Fc fragment, and the polypeptide obtained thereby is referred to as Fc6. Then, a linker sequence "(GGGGS)₃" (SEQ ID NO: 88) was added to the N-terminus of the Fc6, to obtain linker-Fc6. The corresponding DNA sequence encoding it was then designed using online tool DNAworks (helixweb.nih.gov/dnaworks/). Then, a linker sequence "(GGGGS)₃" (SEQ ID NO: 88) was added between two copies of huIL10, to obtain (huIL10)₂. Polynucleotide sequences encoding (huIL10)₂ were then added to the 5' end of the polynucleotide sequences encoding the linker-Fc6, thereby obtaining and synthesizing a polynucleotide sequence encoding the fusion protein (huIL10)₂-Fc6. The amino acid sequence of (huIL10)₂-Fc6 is as set forth in SEQ ID NO: 91, and the polynucleotide sequence encoding it is as set forth in SEQ ID NO: 92.

### 1.18 Preparation of (huIL10)₂-Fc9

First of all, sequence information of human interleukin 10 (huIL10) (P22301) was obtained from the National Center for Biotechnology Information (NCBI), and the full length polynucleotide sequences encoding it were obtained. Then, amino acid sequences of human IgG1-Fc (i.e., residue 104 to residue 330 of P01857) were obtained according to the amino acid sequences of human immunoglobulin gamma1 (IgG1) constant region (P01857) from the protein database Uniprot. Afterwards, point mutations (T366W and K409A) were introduced into the IgG1-Fc fragment, and the polypeptide obtained thereby is referred to as Fc9. Then, a linker sequence "(GGGGS)₃" (SEQ ID NO: 88) was added to the N-terminus of the Fc9, to obtain linker-Fc9. The corresponding DNA sequence encoding it was then designed using online tool DNAworks (helixweb.nih.gov/dnaworks/). Then, a linker sequence "(GGGGS)₃" (SEQ ID NO: 88) was added between two copies of huIL10, to obtain (huIL10)₂. Polynucleotide sequences encoding (huIL10)₂ were then added to the 5' end of the polynucleotide sequences encoding the linker-Fc9, thereby obtaining and synthesizing a polynucleotide sequence encoding the fusion protein (huIL10)₂-Fc9. The amino acid sequence of (huIL10)₂-Fc9 is as set forth in SEQ ID NO: 93, and the polynucleotide sequence encoding it is as set forth in SEQ ID NO: 94.

### 1.19 Preparation of (huIL10)₂-Fc-hole

First of all, sequence information of human interleukin 10 (huIL10) (P22301) was obtained from the National Center for Biotechnology Information (NCBI), and the full length polynucleotide sequences encoding it were obtained. Then, amino acid sequences of human IgG1-Fc (i.e., residue 104 to residue 330 of P01857) were obtained according to the amino acid sequences of human immunoglobulin gamma1 (IgG1) constant region (P01857) from the protein database Uniprot. Afterwards, point mutations (T366S, L368G, Y407A and F405K) were introduced into the IgG1-Fc fragment, and the polypeptide obtained thereby is referred to as Fc-hole. Then, a linker sequence "(GGGGS)₃" (SEQ ID NO: 88) was added to the N-terminus of the Fc-hole, to obtain linker-Fc-hole. The corresponding DNA sequence encoding it was then designed using online tool DNAworks (helixweb.nih.gov/dnaworks/). Then, a linker sequence "(GGGGS)₃" (SEQ ID NO: 88) was added between two copies of huIL10, to obtain (huIL10)₂. Polynucleotide sequences encoding (huIL10)₂ were then added to the 5' end of the polynucleotide sequences encoding the linker-Fc-hole, thereby obtaining and synthesizing a polynucleotide sequence encoding the fusion protein (huIL10)₂-Fc-hole. The amino acid sequence of (huIL10)₂-Fc-hole is as set forth in SEQ ID NO: 95, and the polynucleotide sequence encoding it is as set forth in SEQ ID NO: 96.

### 1.20 Preparation of husIL2-Fc6

First of all, sequence information of human super interleukin 2 (husIL2) was obtained according to Nature 484, 529-533 (26 April 2012) (incorporated herein by reference), and the full length polynucleotide sequences encoding it were obtained. Then, amino acid sequences of human IgG1-Fc (i.e., residue 104 to residue 330 of P01857) were obtained according to the amino acid sequences of human immunoglobulin gamma1 (IgG1) constant region (P01857) from the protein database Uniprot. Afterwards, point mutations (T366S, L368G, Y407A and F405K) were introduced into the IgG1-Fc fragment, and the polypeptide obtained thereby is referred to as Fc6. Then, a linker sequence "GGGGS" (SEQ ID NO: 79) was added to the N-terminus of the Fc6, to obtain linker-Fc6. The corresponding DNA sequence encoding it was then designed using online tool DNAworks (helixweb.nih.gov/dnaworks/). Polynucleotide sequences encoding husIL2 were added to the 5' end of the polynucleotide sequences encoding the linker-Fc6, thereby obtaining and synthesizing a polynucleotide sequence encoding the fusion protein husIL2-Fc6. The amino acid sequence of husIL2-Fc6 is as set forth in SEQ ID NO: 97, and the polynucleotide sequence encoding it is as set forth in SEQ ID NO: 98.

### 1.21 Preparation of husIL2-hole

First of all, sequence information of human super interleukin 2 (husIL2) was obtained according to Nature 484, 529-533 (26 April 2012) (incorporated herein by reference), and the full length polynucleotide sequences encoding it were obtained. Then, amino acid sequences of human IgG1-Fc (i.e., residue 104 to residue 330 of P01857) were obtained according to the amino acid sequences of human immunoglobulin gamma1 (IgG1) constant region (P01857) from the protein database Uniprot. Afterwards, point mutations (D356C, T366S, L368A and Y407V) were introduced into the IgG 1-Fc fragment, and the polypeptide obtained thereby is referred to as Fc-hole. Then, a linker sequence "GGGGS" (SEQ ID NO: 79) was added to the N-terminus of the Fc-hole, to obtain linker-Fc-hole. The corresponding DNA sequence encoding it was then designed using online tool DNAworks (helixweb.nih.gov/dnaworks/). Polynucleotide sequences encoding husIL2 were added to the 5' end of the polynucleotide sequences encoding the linker-Fc-hole, thereby obtaining and synthesizing a polynucleotide sequence encoding the fusion protein husIL2-Fc-hole. The amino acid sequence of husIL2-Fc-hole is as set forth in SEQ ID NO: 99, and the polynucleotide sequence encoding it is as set forth in SEQ ID NO: 100.

### Example 2: Construction of Recombinant Plasmids

The nucleic acid molecules (encoding Erb-Fc9, Erb-Fc6, Erb-Knob, T-Fc9, P-Fc9, Mab806-Fc9, C-mab-Fc9, 28H1-Fc9, 5E5-Fc9, E-mab-Fc9, A-mab-Fc9, T-LC (Trastuzumab light chain), P-LC (Pertuzumab light chain), Erb-LC (Cetuximab light chain), Mab806-LC (Mab806 light chain), C-mab-LC (codrituzumab light chain), 28H1-LC (28H1 light chain), 5E5-LC (5E5 light chain), E-mab-LC (ensituximab light chain), A-mab-LC (amatuximab light chain), muIFNa4-Fc6, huIFNa2-Fc6, muIFNb-Fc6, huIFNb-Fc6, huIFNL-Fc6, huIL10-Fc6, (huIL10)₂-Fc6, (huIL10)₂-Fc9, (huIL10)₂-Fc-hole, husIL2-Fc6, and husIL2-hole, respectively) obtained according to Example 1 were digested with HindIII and EcoRI (Takara), and then subcloned into the pcDNA4/myc-HisA vectors (Invitrogen, V863-20), respectively. The plasmids obtained were verified by sequencing, and the correct recombinant plasmids were named as: pcDNA4-Erb-Fc9, pcDNA4-Erb-Fc6, pcDNA4-Erb-Knob, pcDNA4-T-Fc9, pcDNA4-P-Fc9, pcDNA4-Mab806-Fc9, pcDNA4-C-mab-Fc9, pcDNA4-28H1-Fc9, pcDNA4-5E5-Fc9, pcDNA4-E-mab-Fc9, pcDNA4-A-mab-Fc9, pcDNA4-T-LC, pcDNA4-P-LC, pcDNA4-Erb-LC, pcDNA4-Mab806-LC, pcDNA4-C-mab-LC, pcDNA4-28H1-LC, pcDNA4-5E5-LC, pcDNA4-E-mab-LC, pcDNA4-A-mab-LC, pcDNA4-muIFNa4-Fc6, pcDNA4-huIFNa2-Fc6, pcDNA4-muIFNb-Fc6, pcDNA4-huIFNb-Fc6, pcDNA4-huIFNL-Fc6, pcDNA4-huIL10-Fc6, pcDNA4-(huIL10)₂-Fc6, pcDNA4-(huIL10)₂-Fc9, pcDNA4-(huIL10)₂-Fc-hole, pcDNA4-husIL2-Fc6, and pcDNA4-husIL2-hole, respectively.

### Example 3: Expression and Purification of Proteinaceous Heterodimers

Two days before transfection, 12 × 600 mL suspension-domesticated HEK293 (ATCC, CRL-1573^{™}) cells were prepared for transient transfection, and the cells were seeded at a density of 0.8 × 10⁶ cells/mL. Two days later, three aliquots of cell suspension were centrifuged, and then resuspended in 600 mL Freestyle293 culture medium.

The recombinant expression vectors obtained from Example 2 were divided into the following groups:
Group 1: pcDNA4-Erb-Knob (200 µg)+pcDNA4-Erb-LC (200 µg)+pcDNA4-(huIL10)₂-Fc-hole (200 µg)
Group 2: pcDNA4-Erb-Fc6 (200 µg)+pcDNA4-Erb-LC (200 µg)+pcDNA4-(huIL10)₂-Fc9 (200 µg)
Group 3: pcDNA4-Erb-Fc9 (200 µg)+pcDNA4-Erb-LC (200 µg)+pcDNA4-(huIL10)₂-Fc6 (200 µg)
Group 4: pcDNA4-Erb-Fc9 (200 µg)+pcDNA4-Erb-LC (200 µg)+pcDNA4-muIFNa4-Fc6 (200 µg)
Group 5: pcDNA4-Erb-Fc9 (200 µg)+pcDNA4-Erb-LC (200 µg)+pcDNA4-huIFNa2-Fc6 (200 µg)
Group 6: pcDNA4-Erb-Fc9 (200 µg)+pcDNA4-Erb-LC (200 µg)+pcDNA4-muIFNb-Fc6 (200 µg)
Group 7: pcDNA4-Erb-Fc9 (200 µg)+pcDNA4-Erb-LC (200 µg)+pcDNA4-huIFNb-Fc6 (200 µg)
Group 8: pcDNA4-Erb-Fc9 (200 µg)+pcDNA4-Erb-LC (200 µg)+pcDNA4-huIFNL-Fc6 (200 µg)
Group 9: pcDNA4-Erb-Fc9 (200 µg)+pcDNA4-Erb-LC (200 µg)+pcDNA4-huIL10-Fc6 (200 µg)
Group 10: pcDNA4-Erb-Knob (200 µg)+pcDNA4-Erb-LC (200 µg)+pcDNA4-husIL2-hole (200 µg)
Group 11: pcDNA4-Erb-Fc9 (200 µg)+pcDNA4-Erb-LC (200 µg)+pcDNA4-husIL2-Fc6 (200 µg)
Group 12: pcDNA4-Mab806-Fc9 (200 µg)+pcDNA4-Mab806-LC (200 µg)+pcDNA4-muIFNa4-Fc6 (200 µg)
Group 13: pcDNA4-Mab806-Fc9 (200 µg)+pcDNA4-Mab806-LC (200 µg)+pcDNA4-huIFNa2-Fc6 (200 µg)
Group 14: pcDNA4-Mab806-Fc9 (200 µg)+pcDNA4-Mab806-LC (200 µg)+pcDNA4-muIFNb-Fc6 (200 µg)
Group 15: pcDNA4-Mab806-Fc9 (200 µg)+pcDNA4-Mab806-LC (200 µg)+pcDNA4-huIFNb-Fc6 (200 µg)
Group 16: pcDNA4-Mab806-Fc9 (200 µg)+pcDNA4-Mab806-LC (200 µg)+pcDNA4-huIFNL-Fc6 (200 µg)
Group 17: pcDNA4-Mab806-Fc9 (200 µg)+pcDNA4-Mab806-LC (200 µg)+pcDNA4-huIL10-Fc6 (200 µg)
Group 18: pcDNA4-Mab806-Fc9 (200 µg)+pcDNA4-Mab806-LC (200 µg)+pcDNA4-(huIL10)₂-Fc6 (200 µg)
Group 19: pcDNA4-Mab806-Fc9 (200 µg)+pcDNA4-Mab806-LC (200 µg)+pcDNA4-husIL2-Fc6 (200 µg)
Group 20: pcDNA4-T-Fc9 (200 µg)+pcDNA4-T-LC (200 µg)+pcDNA4-muIFNa4-Fc6 (200 µg)
Group 21: pcDNA4-T-Fc9 (200 µg)+pcDNA4-T-LC (200 µg)+pcDNA4-huIFNa2-Fc6 (200 µg)
Group 22: pcDNA4-T-Fc9 (200 µg)+pcDNA4-T-LC (200 µg)+pcDNA4-muIFNb-Fc6 (200 µg)
Group 23: pcDNA4-T-Fc9 (200 µg)+pcDNA4-T-LC (200 µg)+pcDNA4-huIFNb-Fc6 (200 µg)
Group 24: pcDNA4-T-Fc9 (200 µg)+pcDNA4-T-LC (200 µg)+pcDNA4-huIFNL-Fc6 (200 µg)
Group 25: pcDNA4-T-Fc9 (200 µg)+pcDNA4-T-LC (200 µg)+pcDNA4-huIL10-Fc6 (200 µg)
Group 26: pcDNA4-T-Fc9 (200 µg)+pcDNA4-T-LC (200 µg)+pcDNA4-(huIL10)₂-Fc6 (200 µg)
Group 27: pcDNA4-T-Fc9 (200 µg)+pcDNA4-T-LC (200 µg)+pcDNA4-husIL2-Fc6 (200 µg)
Group 28: pcDNA4-P-Fc9 (200 µg)+pcDNA4-P-LC (200 µg)+pcDNA4-muIFNa4-Fc6 (200 µg)
Group 29: pcDNA4-P-Fc9 (200 µg)+pcDNA4-P-LC (200 µg)+pcDNA4-huIFNa2-Fc6 (200 µg)
Group 30: pcDNA4-P-Fc9 (200 µg)+pcDNA4-P-LC (200 µg)+pcDNA4-muIFNb-Fc6 (200 µg)
Group 31: pcDNA4-P-Fc9 (200 µg)+pcDNA4-P-LC (200 µg)+pcDNA4-huIFNb-Fc6 (200 µg)
Group 32: pcDNA4-P-Fc9 (200 µg)+pcDNA4-P-LC (200 µg)+pcDNA4-huIFNL-Fc6 (200 µg)
Group 33: pcDNA4-P-Fc9 (200 µg)+pcDNA4-P-LC (200 µg)+pcDNA4-huIL10-Fc6 (200 µg)
Group 34: pcDNA4-P-Fc9 (200 µg)+pcDNA4-P-LC (200 µg)+pcDNA4-(huIL10)₂-Fc6 (200 µg)
Group 35: pcDNA4-P-Fc9 (200 µg)+pcDNA4-P-LC (200 µg)+pcDNA4-husIL2-Fc6 (200 µg)
Group 36: pcDNA4-C-mab-Fc9 (200 µg)+pcDNA4-C-mab-LC (200 µg)+pcDNA4-muIFNa4-Fc6 (200 µg)
Group 37: pcDNA4-C-mab-Fc9 (200 µg)+pcDNA4-C-mab-LC (200 µg)+pcDNA4-huIFNa2-Fc6 (200 µg)
Group 38: pcDNA4-C-mab-Fc9 (200 µg)+pcDNA4-C-mab-LC (200 µg)+pcDNA4-muIFNb-Fc6 (200 µg)
Group 39: pcDNA4-C-mab-Fc9 (200 µg)+pcDNA4-C-mab-LC (200 µg)+pcDNA4-huIFNb-Fc6 (200 µg)
Group 40: pcDNA4-C-mab-Fc9 (200 µg)+pcDNA4-C-mab-LC (200 µg)+pcDNA4-huIFNL-Fc6 (200 µg)
Group 41: pcDNA4-C-mab-Fc9 (200 µg)+pcDNA4-C-mab-LC (200 µg)+pcDNA4-huIL10-Fc6 (200 µg)
Group 42: pcDNA4-C-mab-Fc9 (200 µg)+pcDNA4-C-mab-LC (200 µg)+pcDNA4-(huIL10)₂-Fe6 (200 µg)
Group 43: pcDNA4-C-mab-Fc9 (200 µg)+pcDNA4-C-mab-LC (200 µg)+pcDNA4-husIL2-Fc6 (200 µg)
Group 44: pcDNA4-28H1-Fc9 (200 µg)+pcDNA4-28H1-LC (200 µg)+pcDNA4-muIFNa4-Fc6(200 µg)
Group 45: pcDNA4-28H1-Fc9 (200 µg)+pcDNA4-28H1-LC (200 µg)+pcDNA4-huIFNa2-Fc6 (200 µg)
Group 46: pcDNA4-28H1-Fc9 (200 µg)+pcDNA4-28H1-LC (200 µg)+pcDNA4-muIFNb-Fc6 (200 µg)
Group 47: pcDNA4-28H1-Fc9 (200 µg)+pcDNA4-28H1-LC (200 µg)+pcDNA4-huIFNb-Fc6 (200 µg)
Group 48: pcDNA4-28H1-Fc9 (200 µg)+pcDNA4-28H1-LC (200 µg)+pcDNA4-huIFNL-Fc6 (200 µg)
Group 49: pcDNA4-28H1-Fc9 (200 µg)+pcDNA4-28H1-LC (200 µg)+pcDNA4-huIL10-Fc6 (200 µg)
Group 50: pcDNA4-28H1-Fc9 (200 µg)+pcDNA4-28H1-LC (200 µg)+pcDNA4-(huIL10)₂-Fc6 (200 µg)
Group 51: pcDNA4-28H1-Fc9 (200 µg)+pcDNA4-28H1-LC (200 µg)+pcDNA4-husIL2-Fc6 (200 µg)
Group 52: pcDNA4-5E5-Fc9 (200 µg)+pcDNA4-5E5-LC (200 µg)+pcDNA4-muIFNa4-Fc6 (200 µg)
Group 53: pcDNA4-5E5-Fc9 (200 µg)+pcDNA4-5E5-LC (200 µg)+pcDNA4-huIFNa2-Fc6 (200 µg)
Group 54: pcDNA4-5E5-Fc9 (200 µg)+pcDNA4-5E5-LC (200 µg)+pcDNA4-muIFNb-Fc6 (200 µg)
Group 55: pcDNA4-5E5-Fc9 (200 µg)+pcDNA4-5E5-LC (200 µg)+pcDNA4-huIFNb-Fc6 (200 µg)
Group 56: pcDNA4-5E5-Fc9 (200 µg)+pcDNA4-5E5-LC (200 µg)+pcDNA4-huIFNL-Fc6 (200 µg)
Group 57: pcDNA4-5E5-Fc9 (200 µg)+pcDNA4-5E5-LC (200 µg)+pcDNA4-huIL10-Fc6 (200 µg)
Group 58: pcDNA4-5E5-Fc9 (200 µg)+pcDNA4-5E5-LC (200 µg)+pcDNA4-(huIL10)₂-Fc6 (200 µg)
Group 59: pcDNA4-5E5-Fc9 (200 µg)+pcDNA4-5E5-LC (200 µg)+pcDNA4-husIL2-Fc6 (200 µg)
Group 60: pcDNA4-E-mab-Fc9 (200 µg)+pcDNA4-E-mab-LC (200 µg)+pcDNA4-muIFNa4-Fc6 (200 µg)
Group 61: pcDNA4-E-mab-Fc9 (200 µg)+pcDNA4-E-mab-LC (200 µg)+pcDNA4-huIFNa2-Fc6 (200 µg)
Group 62: pcDNA4-E-mab-Fc9 (200 µg)+pcDNA4-E-mab-LC (200 µg)+pcDNA4-muIFNb-Fc6 (200 µg)
Group 63: pcDNA4-E-mab-Fc9 (200 µg)+pcDNA4-E-mab-LC (200 µg)+pcDNA4-huIFNb-Fc6 (200 µg)
Group 64: pcDNA4-E-mab-Fc9 (200 µg)+pcDNA4-E-mab-LC (200 µg)+pcDNA4-huIFNL-Fc6 (200 µg)
Group 65: pcDNA4-E-mab-Fc9 (200 µg)+pcDNA4-E-mab-LC (200 µg)+pcDNA4-huIL10-Fc6 (200 µg)
Group 66: pcDNA4-E-mab-Fc9 (200 µg)+pcDNA4-E-mab-LC (200 µg)+pcDNA4-(huIL10)₂-Fc6 (200 µg)
Group 67: pcDNA4-E-mab-Fc9 (200 µg)+pcDNA4-E-mab-LC (200 µg)+pcDNA4-husIL2-Fc6 (200 µg)
Group 68: pcDNA4-A-mab-Fc9 (200 µg)+pcDNA4-A-mab-LC (200 µg)+pcDNA4-muIFNa4-Fe6 (200 µg)
Group 69: pcDNA4-A-mab-Fc9 (200 µg)+pcDNA4-A-mab-LC (200 gg)+pcDNA4-huIFNa2-Fc6 (200 µg)
Group 70: pcDNA4-A-mab-Fc9 (200 µg)+pcDNA4-A-mab-LC (200 µg)+pcDNA4-muIFNb-Fe6 (200 µg)
Group 71: pcDNA4-A-mab-Fc9 (200 µg)+pcDNA4-A-mab-LC (200 µg)+pcDNA4-huIFNb-Fc6 (200 µg)
Group 72: pcDNA4-A-mab-Fc9 (200 µg)+pcDNA4-A-mab-LC (200 µg)+pcDNA4-huIFNL-Fc6 (200 µg)
Group 73: pcDNA4-A-mab-Fc9 (200 µg)+pcDNA4-A-mab-LC (200 µg)+pcDNA4-huIL10-Fc6 (200 µg)
Group 74: pcDNA4-A-mab-Fc9 (200 µg)+pcDNA4-A-mab-LC (200 µg)+pcDNA4-(huIL10)₂-Fc6 (200 µg)
Group 75: pcDNA4-A-mab-Fc9 (200 µg)+pcDNA4-A-mab-LC (200 µg)+pcDNA4-husIL2-Fc6 (200 µg)

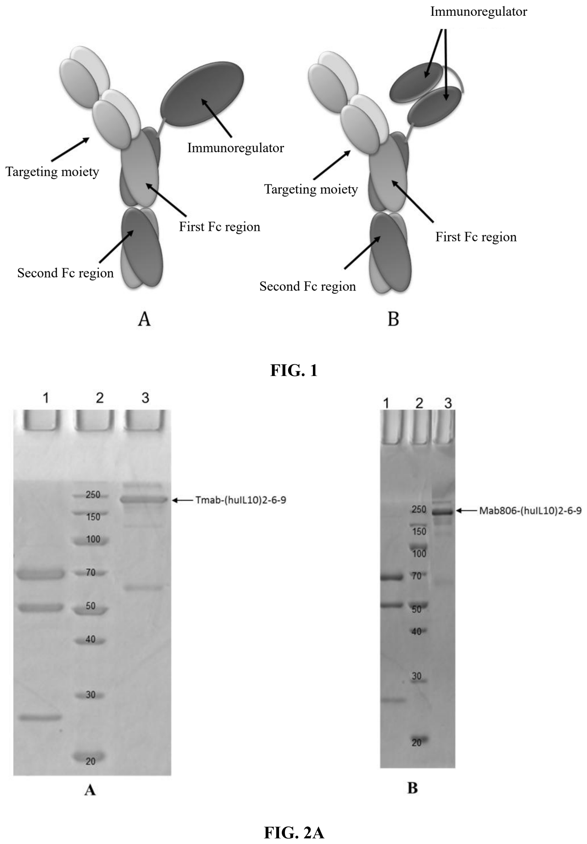

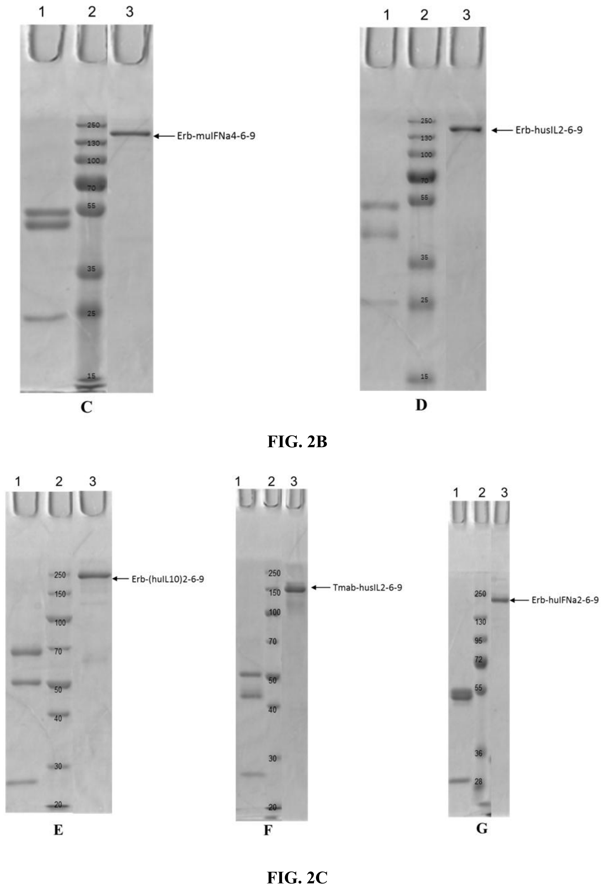

Each group of plasmid mixtures was diluted with 6 mL Freestyle293 medium, and PEI (polyethylenimine) solution was added to perform transfection. Each group of plasmid/PEI mixtures was added into 600 mL cell suspension, which was then cultured at 37 °C, 10% CO₂, 90 rpm, and the medium was supplemented with 50 µg/L IGF-1 (insulin-like growth factor I). Four hours later, the culture was supplemented with 600 mL EX293 medium, 2 mM glutamine and 50 µg/L IGF-1, and cultured at 135 rpm. After 24 hours, 3.8 mM VPA was added. After 5-6 days, the supernatant of 5 × 1200 mL cells was collected, and crude proteinaceous heterodimer samples were purified by Protein A affinity chromatography. The samples obtained were examined first with SDS-PAGE, and the target bands were clearly seen. Examples are shown in FIG. 2.

FIG. 2A shows the SDS-PAGE assay result for Tmab-(huIL 10)2-6-9, lane 2 was loaded with a protein marker (MW marker 26619), lane 1 was loaded with the reduced Tmab-(huIL10)2-6-9 heterodimer protein, and lane 3 was loaded with the non-reduced Tmab-(huIL10)2-6-9 heterodimer protein; T-LC has a MW of about 25 KD, T-Fc9 has a MW of about 50 KD, (huIL10)2-Fc6 has a MW of about 65 KD, and the Tmab-(huIL10)2-6-9 heterodimer protein has a MW of about 140 KD.

FIG. 2B shows the SDS-PAGE assay result for Mab806-(huIL10)2-6-9, lane 2 was loaded with a protein marker (MW marker 26619), lane 1 was loaded with the reduced Mab806-(huIL10)2-6-9 heterodimer protein, and lane 3 was loaded with the non-reduced Mab806-(huIL 10)2-6-9 heterodimer protein; Mab806-LC has a MW of about 25 KD, Mab806-Fc9 has a MW of about 50 KD, (huIL10)2-Fc6 has a MW of about 65 KD, and the Mab806-(huIL10)2-6-9 heterodimer protein has a MW of about 140 KD.

FIG. 2C shows the SDS-PAGE assay result for Erb-muIFNa4-6-9, lane 2 was loaded with a protein marker (MW marker 26619), lane 1 was loaded with the reduced Erb-muIFNa4-6-9 heterodimer protein, and lane 3 was loaded with the non-reduced Erb-muIFNa4-6-9 heterodimer protein; Erb-LC has a MW of about 25 KD, Erb-Fc9 has a MW of about 50 KD, muIFNa4-Fc6 has a MW of about 45 KD, and the Erb-muIFNa4-6-9 heterodimer protein has a MW of about 120 KD.

FIG. 2D shows the SDS-PAGE assay result for Erb-husIL2-6-9, lane 2 was loaded with a protein marker (MW marker 26619), lane 1 was loaded with the reduced Erb-husIL2-6-9 heterodimer protein, and lane 3 was loaded with the non-reduced Erb-husIL2-6-9 heterodimer protein; Erb-LC has a MW of about 25 KD, Erb-Fc9 has a MW of about 50 KD, husIL2-Fc6 has a MW of about 41 KD, and the Erb-husIL2-6-9 heterodimer protein has a MW of about 120 KD.

FIG. 2E shows the SDS-PAGE assay result for Erb-(huIL10)2-6-9, lane 2 was loaded with a protein marker (MW marker 26619), lane 1 was loaded with the reduced Erb-(huIL10)2-6-9 heterodimer protein, and lane 3 was loaded with the non-reduced Erb-(huIL10)2-6-9 heterodimer protein; Erb-LC has a MW of about 25 KD, Erb-Fc9 has a MW of about 50 KD, (huIL10)2-Fc6 has a MW of about 65 KD, and the Erb-(huIL10)2-6-9 heterodimer protein has a MW of about 140 KD.

FIG. 2F shows the SDS-PAGE assay result for Tmab-husIL2-6-9, lane 2 was loaded with a protein marker (MW marker 26619), lane 1 was loaded with the reduced Tmab-husIL2-6-9 heterodimer protein, and lane 3 was loaded with the non-reduced Tmab-husIL2-6-9 heterodimer protein; T-LC has a MW of about 25 KD, T-Fc9 has a MW of about 50 KD, husIL2-Fc6 has a MW of about 41 KD, and the Tmab-husIL2-6-9 heterodimer protein has a MW of about 116 KD.

FIG. 2G shows the SDS-PAGE assay result for Erb-huIFNa2-6-9, lane 2 was loaded with a protein marker (MW marker 26619), lane 1 was loaded with the reduced Erb-huIFNa2-6-9 heterodimer protein, and lane 3 was loaded with the non-reduced Erb-huIFNa2-6-9 heterodimer protein; Erb-LC has a MW of about 25 KD, Erb-Fc9 has a MW of about 50 KD, huIFNa2-Fc6 has a MW of about 45 KD, and the Erb-huIFNa2-6-9 heterodimer protein has a MW of about 120 KD.

Similarly, the expression and purification results of the other proteinaceous heterodimers of the present disclosure were verified and confirmed with SDS-PAGE.

The proteinaceous heterodimers thus obtained are named as (from Group 1 to Group 75, respectively): Erb-(huIL10)₂-KH, Erb-(huIL10)₂-6-9, Erb-(huIL10)₂-9-6, Erb-muIFNa4-6-9, Erb-huIFNa2-6-9, Erb-muIFNb-6-9, Erb-huIFNb-6-9, Erb-huIFNL-6-9, Erb-huIL10-6-9, Erb-husIL2-6-9, Erb-husIL2-KH, Mab806-muIFNa4-6-9, Mab806-huIFNa2-6-9, Mab806-muIFNb-6-9, Mab806-huIFNb-6-9, Mab806-huIFNL-6-9, Mab806-huIL10-6-9, Mab806-husIL2-6-9, Mab806-(huIL10)₂-6-9, TmabmuIFNa4-6-9, Tmab-huIFNa2-6-9, Tmab-muIFNb-6-9, Tmab-huIFNb-6-9, Tmab-huIFNL-6-9, Tmab-huIL10-6-9, Tmab-husIL2-6-9, Tmab-(huIL10)₂-6-9, Pmab-muIFNa4-6-9, Pmab-huIFNa2-6-9, Pmab-muIFNb-6-9, Pmab-huIFNb-6-9, Pmab-huIFNL-6-9, Pmab-huIL10-6-9, Pmab-husIL2-6-9, Pmab-(huIL10)₂-6-9, C-mab-muIFNa4-6-9, C-mab-huIFNa2-6-9, C-mab-muIFNb-6-9, C-mab-huIFNb-6-9, C-mab-huIFNL-6-9, C-mab-huIL10-6-9, C-mab-husIL2-6-9, C-mab-(huTL10)₂-6-9, 28H1-muIFNa4-6-9, 28H1-huIFNa2-6-9, 28H1-muIFNb-6-9, 28H1-huIFNb-6-9, 28H1-huIFNL-6-9, 28H1-huIL10-6-9, 28H1-husIL2-6-9, 28H1-(huIL10)₂-6-9, 5E5-muIFNa4-6-9, 5E5-huIFNa2-6-9, 5E5-muIFNb-6-9, 5E5-huIFNb-6-9, 5E5-huIFNL-6-9, 5E5-huIL10-6-9, 5E5-husIL2-6-9, 5E5-(huIL10)₂-6-9, E-mab-muIFNa4-6-9, E-mab-huIFNa2-6-9, E-mab-muIFNb-6-9, E-mab-huIFNb-6-9, E-mab-huIFNL-6-9, E-mab-huIL10-6-9, E-mab-husIL2-6-9, E-mab-(huIL10)₂-6-9, A-mab-muIFNa4-6-9, A-mab-huIFNa2-6-9, A-mab-muIFNb-6-9, A-mab-huIFNb-6-9, A-mab-huIFNL-6-9, A-mab-huIL10-6-9, A-mab-husIL2-6-9, and A-mab-(huIL 10)₂-6-9, respectively.

### Example 4: Activity of Proteinaceous Heterodimer in Treating Malignant Effusion

### 4.1 Activity of Erb-interleukin heterodimer of the present disclosure in treating malignant ascites caused by liver cancer

A BALB/c mouse model was used to test the *in vivo* activity of Erb-(huIL10)2-6-9 in treating the malignant effusion caused by liver cancer. BALB/c mice were injected intraperitoneally with mouse H22-Luc cells (the mouse H22-Luc cells were purchased from Shanghai Model Organisms Center, Inc.) at a dose of 1 × 10⁵ cells per mouse. Two days after inoculation, *in vivo* imaging was performed on the mice. The mice were grouped according to the imaging data, and administration was started (see Table 7 for details), which was recorded as day 0 of the administration. The mice were injected intraperitoneally (i.p.) with the Erb-(huIL10)₂-6-9 heterodimer twice a week. Specifically, the mice were treated with the intraperitoneal injection of the Erb-(huIL10)₂-6-9 heterodimer at two different doses (0.5 mg/kg and 2 mg/kg, respectively) on day 7, day 10, and day 14. After inoculation, the body weight, abdominal girth, and abdominal imaging fluorescence data of the mice were measured twice a week.

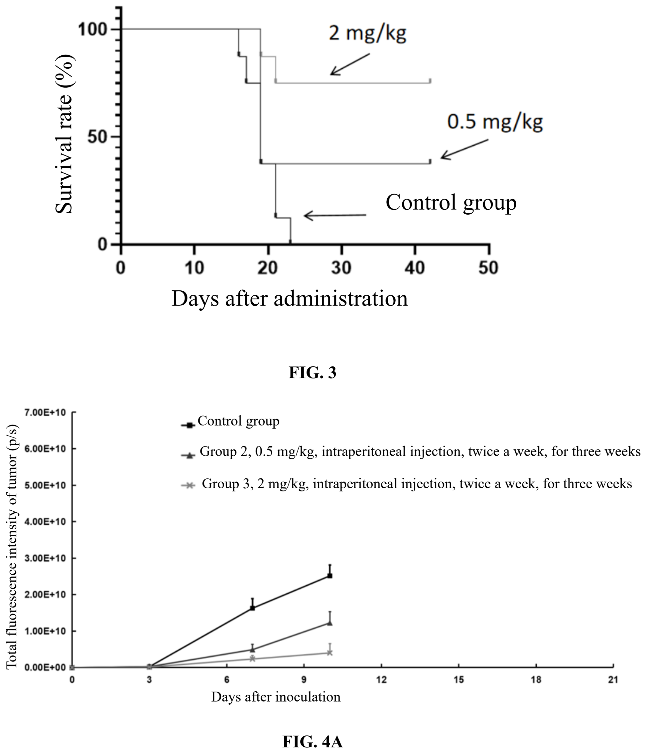

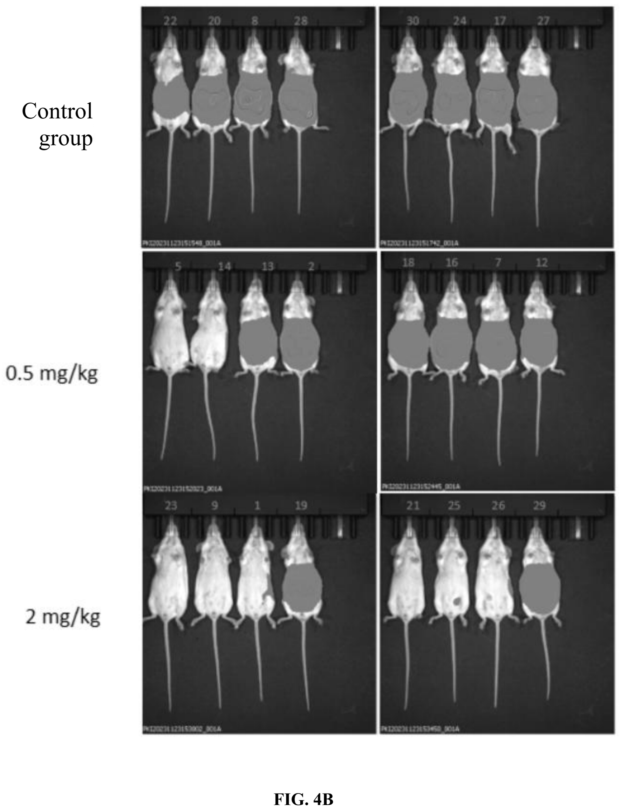

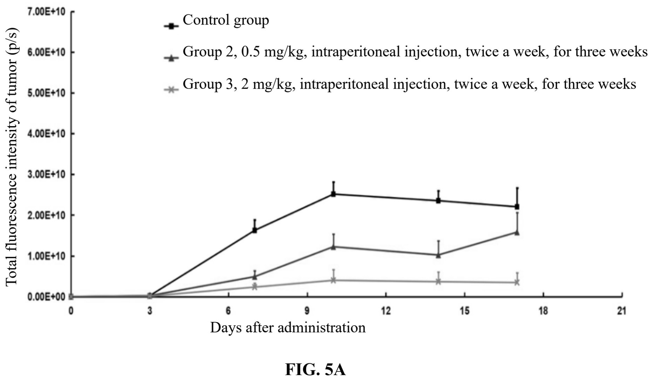

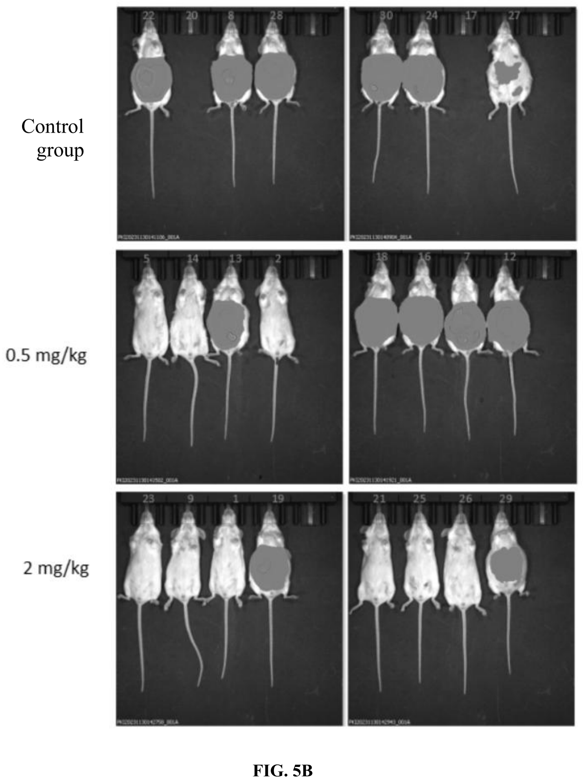

The experimental results are shown in FIGs. 3-5. The *in vivo* imaging data of the mice on day 10 and day 17 after the start of administration indicate that the ascites production of the mice in groups 2 and 3 treated with Erb-(huIL10)2-6-9 was significantly reduced (FIGs. 4-5), demonstrating that the Erb-(huIL10)₂-6-9 heterodimer has a good effect in treating malignant ascites caused by liver cancer. The administration was stopped on day 35 after the start of administration. The experimental data show that the survival of the mice in groups 2 and 3 treated with the drug was significantly prolonged, and the survival rate of the mice in the groups was significantly improved (FIG. 3), indicating that the Erb-(huIL10) ₂-6-9 heterodimer has a good effect in treating malignant ascites caused by liver cancer. As the dose increased, the survival rate of the mice was also higher, indicating that Erb-(huIL10) ₂-6-9 has a dose-dependent effect on prolonging the survival time of the mice.

**Table 7. Administration of Erb-(huIL10) ₂-6-9 heterodimer in treating malignant ascites caused by liver cancer**

| Group | Administration drug | Number of animals | Dose | Manner of administration | Frequency of administration |
|---|---|---|---|---|---|
| | | Animals | mg/kg | | |
| 1 | Control group PBS | 8 | - | IP | BIW |
| 2 | Erb-(huIL10)₂-6-9 heterodimer | 8 | 0.5 | IP | BIW |
| 3 | Erb-(huIL10)₂-6-9 heterodimer | 8 | 2 | IP | BIW |

### 4.2 Activity of Erb-interleukin heterodimer of the present disclosure in treating malignant ascites caused by colon cancer

A BALB/c mouse model was used to test the *in vivo* activity of Erb-(huIL10)₂-6-9 in treating the malignant effusion caused by colon cancer. BALB/c mice were injected intraperitoneally with mouse B16-EGFR cells or MC38-EGFR cells at a dose of 0.5 × 10⁵ to about 10 × 10⁵ cells per mouse. Two days after inoculation, *in vivo* imaging was performed on the mice. The mice were grouped according to the imaging data, and administration was started, which was recorded as day 0 of the administration. The mice were injected intraperitoneally (i.p.) with the Erb-(huIL10)2-6-9 heterodimer twice a week. Specifically, the mice were treated with the intraperitoneal injection of the Erb-(huIL10)2-6-9 heterodimer at two different doses (0.5 mg/kg and 2 mg/kg, respectively) on day 7, day 10, and day 14. After inoculation, the body weight, abdominal girth, and abdominal imaging fluorescence data of the mice were measured twice a week.

The experimental results show that the *in vivo* imaging data of the mice indicate that the ascites production of the mice in groups 2 and 3 treated with Erb-(huIL10)2-6-9 was significantly reduced, demonstrating that the Erb-(huIL10)2-6-9 heterodimer has a good effect in treating malignant ascites caused by colon cancer.

The experimental results show that the survival time of the mice in groups 2 and 3 treated with the drug was significantly prolonged, and the survival rate of the mice in the groups was significantly improved, indicating that the Erb-(huIL 10) ₂-6-9 heterodimer has a good effect in treating malignant ascites caused by colon cancer. As the dose increased, the survival rate of the mice was also higher, indicating that Erb-(huIL10) ₂-6-9 has a dose-dependent effect on prolonging the survival time of the mice.

### 4.3 Activity of Erb-interleukin heterodimer of the present disclosure in treating malignant ascites caused by pancreatic cancer

A BALB/c mouse model was used to test the *in vivo* activity of Erb-(huIL10)2-6-9 in treating the malignant effusion caused by pancreatic cancer. BALB/c mice were injected intraperitoneally with Pan02 mouse pancreatic cancer cells at a dose of 0.5 × 10⁵ to about 10 × 10⁵ cells per mouse. Two days after inoculation, *in vivo* imaging was performed on the mice. The mice were grouped according to the imaging data, and administration was started, which was recorded as day 0 of the administration. The mice were injected intraperitoneally (i.p.) with the Erb-(huIL10)2-6-9 heterodimer twice a week. Specifically, the mice were treated with the intraperitoneal injection of the Erb-(huIL10)2-6-9 heterodimer at two different doses (0.5 mg/kg and 2 mg/kg, respectively) on day 7, day 10, and day 14. After inoculation, the body weight, abdominal girth, and abdominal imaging fluorescence data of the mice were measured twice a week.

The experimental results show that the *in vivo* imaging data of the mice indicate that the ascites production of the mice in groups 2 and 3 treated with Erb-(huIL10)₂-6-9 was significantly reduced, demonstrating that the Erb-(huIL10)₂-6-9 heterodimer has a good effect in treating malignant ascites caused by pancreatic cancer.

The experimental results show that the survival time of the mice in groups 2 and 3 treated with the drug was significantly prolonged, and the survival rate of the mice in the groups was significantly improved, indicating that the Erb-(huIL10) ₂-6-9 heterodimer has a good effect in treating malignant ascites caused by pancreatic cancer. As the dose increased, the survival rate of the mice was also higher, indicating that Erb-(huIL10) ₂-6-9 has a dose-dependent effect on prolonging the survival time of the mice.

### 4.4 Activity of Erb-interleukin heterodimer of the present disclosure in treating malignant ascites caused by ascites cancer

A BALB/c mouse model was used to test the *in vivo* activity of Erb-(huIL 10)₂-6-9 in treating the malignant effusion caused by ascites cancer. BALB/c mice were injected intraperitoneally with S180 mouse sarcoma cells at a dose of 0.5 × 10⁵ to about 10 × 10⁵ cells per mouse. Two days after inoculation, *in vivo* imaging was performed on the mice. The mice were grouped according to the imaging data, and administration was started, which was recorded as day 0 of the administration. The mice were injected intraperitoneally (i.p.) with the Erb-(huIL10)2-6-9 heterodimer twice a week. Specifically, the mice were treated with the intraperitoneal injection of the Erb-(huIL10)2-6-9 heterodimer at two different doses (0.5 mg/kg and 2 mg/kg, respectively) on day 7, day 10, and day 14. After inoculation, the body weight, abdominal girth, and abdominal imaging fluorescence data of the mice were measured twice a week.

The experimental results show that the *in vivo* imaging data of the mice indicate that the ascites production of the mice in groups 2 and 3 treated with Erb-(huIL10)₂-6-9 was significantly reduced, demonstrating that the Erb-(huIL10)2-6-9 heterodimer has a good effect in treating malignant ascites caused by ascites cancer.

The experimental results show that the survival time of the mice in groups 2 and 3 treated with the drug was significantly prolonged, and the survival rate of the mice in the groups was significantly improved, indicating that the Erb-(huIL10) ₂-6-9 heterodimer has a good effect in treating malignant ascites caused by ascites cancer. As the dose increased, the survival rate of the mice was also higher, indicating that Erb-(huIL 10) ₂-6-9 has a dose-dependent effect on prolonging the survival time of the mice.

### 4.5 Activity of Erb-interleukin heterodimer of the present disclosure in treating malignant pleural effusion caused by lung cancer

C57BL/6 mice were anesthetized, and then the skin on the right side of the chest of the mice was disinfected. Lewis-luc cells (Lewis-luc cells were purchased from Shanghai Model Organisms Center, Inc.) were injected at a dose of 5 × 10⁵ cells per mouse approximately at the position flush with the 6th intercostal space near the midaxillary line on the right side of the chest. The state of the mice was observed daily after inoculation. The day after inoculation, *in vivo* imaging was performed. The mice were randomly grouped according to the imaging results, and the administration was started (see Table 8 for details), with 8 mice in each group. The *in vivo* imaging was then performed twice a week. After two weeks, 4 animals in each group were dissected to observe the production of pleural effusion. The pleural effusion was extracted with a 1 mL syringe without a needle, and the volume of the pleural effusion was calculated. The lung tissues were photographed to calculate the number of metastatic lesions on the lung surface, and in addition, the number of metastatic tumors in the chest wall was counted.

**Table 8. Administration of Erb-(huIL10)2-6-9 heterodimer in treating malignant pleural effusion**

| Group | Administration drug | Number of animals | Dose | Manner of administration | Frequency of administration |
|---|---|---|---|---|---|
| | | Animals | mg/kg | | |
| 1 | Control group PBS | 8 | - | Intraperitoneal injection | Twice a week |
| 2 | Oxaliplatin | 8 | 5 | Intraperitoneal injection | Once a week |
| 3 | Erb-(huIL10)₂-6-9 heterodimer | 8 | 2 | Intraperitoneal injection | Twice a week |

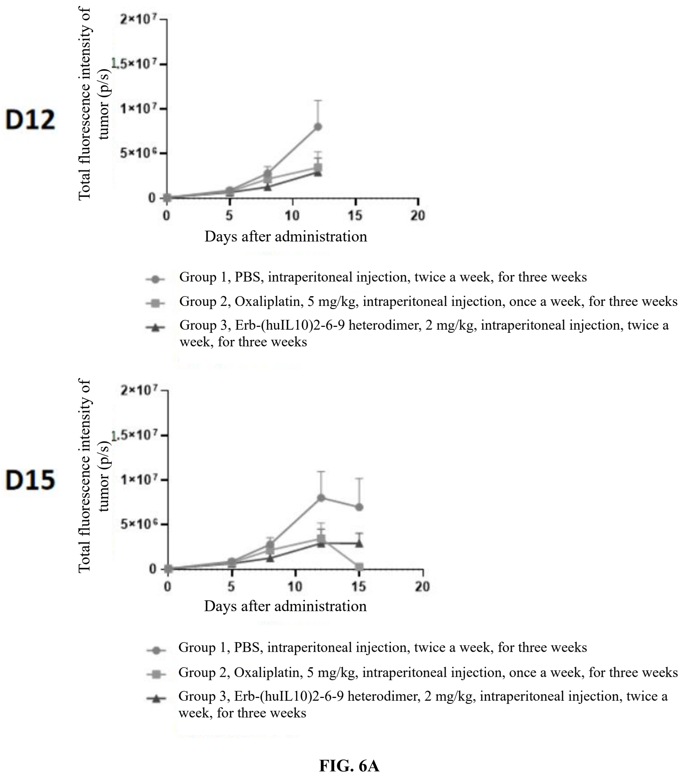

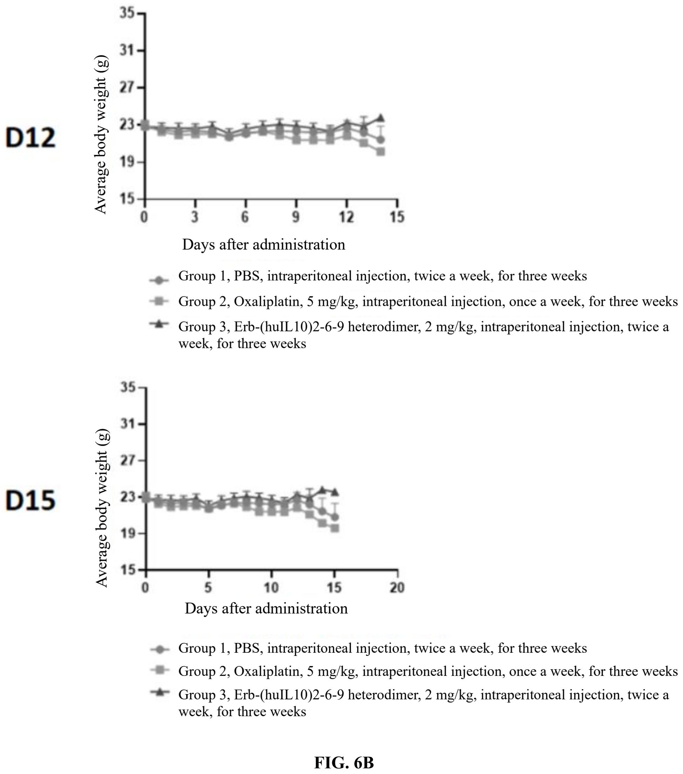

The experimental results are detailed in FIGs. 6-7. The results in FIG. 6 show that on day 12 after the start of administration, the mice in group 3 treated with Erb-(huIL 10)₂-6-9 had stable body weights, and the *in vivo* imaging results show less pleural effusion production; on day 15 after the start of administration, all the mice in the control group 1 and the positive control group 2 died, but the mice in group 3 had stable body weights and no death was observed. By the end of the experiment, all the mice in the control group 1 and the mice in the positive control group 2 died, but two mice from group 3 treated with Erb-(huIL10)₂-6-9 were still alive.

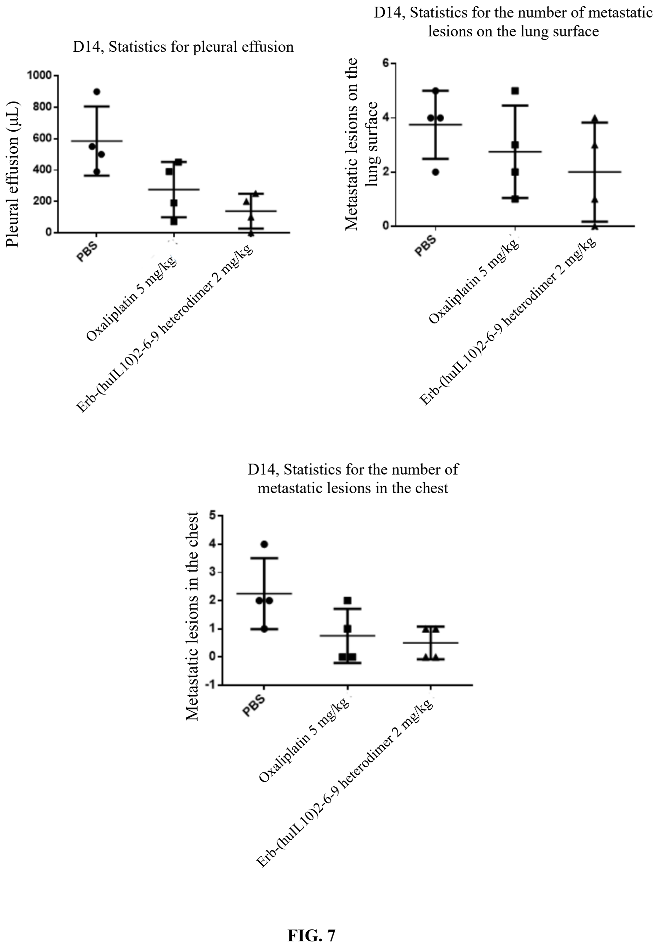

The results in FIG. 7 show the amount of pleural effusion, the number of metastatic lesions on the lung surface, and the number of metastatic lesions in the chest in the mice euthanized for each of groups 1-3 on day 14 after the start of administration. The results in FIG. 7 show that compared with the mice in the control group 1 and the positive control group 2, the mice in group 3 treated with Erb-(huIL10)₂-6-9 developed less pleural effusion after administration and had a significant reduction in the number of metastatic lesions on the lung surface and in the chest.

## Claims

1. Use of a proteinaceous heterodimer in the manufacture of a medicament for preventing and/or treating a malignant effusion, wherein the proteinaceous heterodimer comprises a first member and a second member different from the first member, wherein:
i. the first member comprises a light chain and a heavy chain comprising a first Fc region, and the light chain is complexed with the heavy chain to form a targeting moiety exhibiting binding specificity to a tumor antigen;
ii. the second member comprises a polypeptide comprising an immunoregulator fused to a second Fc region;
iii. the first member associates with the second member to form the heterodimer through complexation of the first Fc region with the second Fc region; and
iv. the first Fc region comprises a first modification and/or the second Fc region comprises a second modification, wherein the first modification and/or the second modification more effectively promotes heterodimerization between the first member and the second member than a knob-and-hole modification comprising a knob modification and a hole modification.

2. The use according to claim 1, wherein the polypeptide comprised in the second member is a fusion protein, and a C-terminus of the immunoregulator is fused to an N-terminus of the second Fc region to form the fusion protein, optionally via a linker.

3. The use according to any one of the preceding claims, wherein the tumor antigen is selected from the group consisting of EGFR, an EGFR mutant, HER2/neu, GPC3, FAP, Muc1, MUC5AC, and Mesothelin.

4. The use according to any one of the preceding claims, wherein the immunoregulator is a cytokine selected from the group consisting of an interferon, an interleukin, a chemokine, a lymphokine, and a tumor necrosis factor.

5. The use according to any one of the preceding claims, wherein the immunoregulator is an interferon selected from the group consisting of interferon alpha, interferon lambda, and interferon beta.

6. The use according to any one of the preceding claims, wherein the immunoregulator is an interleukin, and the interleukin comprises interleukin 10, interleukin 2, and/or super interleukin 2.

7. The use according to any one of the preceding claims, wherein the first Fc region and the second Fc region are from an Fc region of an immunoglobulin, and the immunoglobulin is a human IgG1.

8. The use according to any one of the preceding claims, wherein the polypeptide comprised in the second member comprises two or more immunoregulators, the two or more immunoregulators are fused in frame to each other and to the second Fc region, and wherein the two or more immunoregulators are located N-terminal to the second Fc region.

9. The use according to any one of the preceding claims, wherein the first modification comprises an amino acid substitution at position T366, and an amino acid substitution at one or more positions selected from the group consisting of: Y349, F405, K409, D399, K360, Q347, K392, and S354, wherein the position of the amino acid is determined according to the EU index of the KABAT number.

10. The use according to claim 9, wherein the amino acid substitution comprised by the first modification is selected from the group consisting of Y349C, Y349D, D399S, F405K, K360E, K409A, K409E, Q347E, Q347R, S354D, K392D, and T366W.

11. The use according to any one of claims 9-10, wherein the first modification comprises an amino acid substitution at a group of positions selected from any of the following groups:
1) Y349 and T366;
2) Y349, T366, and F405;
3) Y349, T366, and K409;
4) Y349, T366, F405, K360, and Q347;
5) Y349, T366, F405, and Q347;
6) Y349, T366, K409, K360, and Q347;
7) Y349, T366, K409, and Q347;
8) T366, K409, and K392;
9) T366 and K409;
10) T366, K409, Y349, and S354;
11) T366 and F405;
12) T366, F405, and D399; and
13) T366, F405, Y349, and S354.

12. The use according to any one of claims 9-11, wherein the first modification comprises a group of amino acid substitutions selected from any of the following groups:
1) Y349C and T366W;
2) Y349C, T366W, and F405K;
3) Y349C, T366W, and K409E;
4) Y349C, T366W, and K409A;
5) Y349C, T366W, F405K, K360E, and Q347E;
6) Y349C, T366W, F405K, and Q347R;
7) Y349C, T366W, K409A, K360E, and Q347E;
8) Y349C, T366W, K409A, and Q347R;
9) T366W, K409A, and K392D;
10) T366W and K409A;
11) T366W, K409A, and Y349D;
12) T366W, K409A, Y349D, and S354D;
13) T366W and F405K;
14) T366W, F405K, and D399S;
15) T366W, F405K, and Y349D; and
16) T366W, F405K, Y349D, and S354D.

13. The use according to any one of claims 9-12, wherein the second modification comprises amino acid substitutions at positions T366, L368, and Y407, as well as an amino acid substitution at one or more positions selected from the group consisting of D356, D399, E357, F405, K360, K392, K409, and Q347, wherein the position of the amino acid is determined according to the EU index of the KABAT number.

14. The use according to claim 13, wherein the amino acid substitution comprised by the second modification is selected from the group consisting of D356C, D399S, E357A, F405K, K360E, K392D, K409A, L368A, L368G, Q347E, Q347R, T366S, Y407A, and Y407V.

15. The use according to any one of claims 13-14, wherein the second modification comprises an amino acid substitution at a group of positions selected from any of the following groups:
1) D356, T366, L368, Y407, and F405;
2) D356, T366, L368, and Y407;
3) D356, T366, L368, Y407, and Q347;
4) D356, T366, L368, Y407, K360, and Q347;
5) D356, T366, L368, Y407, F405, and Q347;
6) D356, T366, L368, Y407, F405, K360, and Q347;
7) T366, L368, Y407, D399, and F405;
8) T366, L368, Y407, and F405;
9) T366, L368, Y407, F405, and E357;
10) T366, L368, Y407, and K409;
11) T366, L368, Y407, K409, and K392; and
12) T366, L368, Y407, K409, and E357.

16. The use according to any one of claims 13-15, wherein the second modification comprises a group of amino acid substitutions selected from any of the following groups:
1) D356C, T366S, L368A, Y407V, and F405K;
2) D356C, T366S, L368A, and Y407V;
3) D356C, T366S, L368A, Y407V, and Q347R;
4) D356C, T366S, L368A, Y407V, K360E, and Q347E;
5) D356C, T366S, L368A, Y407V, F405K, and Q347R;
6) D356C, T366S, L368A, Y407V, F405K, K360E, and Q347E;
7) T366S, L368A, Y407V, D399S, and F405K;
8) T366S, L368G, Y407A, and F405K;
9) T366S, L368A, Y407V, F405K, and E357A;
10) T366S, L368A, Y407V, and K409A;
11) T366S, L368A, Y407V, K409A, and K392D;
12) T366S, L368G, Y407A, and K409A;
13) T366S, L368A, Y407V, K409A, and E357A.

17. The use according to any one of the preceding claims, wherein the first Fc region comprises the first modification, the second Fc region comprises the second modification, and the first modification and the second modification comprise an amino acid substitution at a group of positions selected from any of the following groups:
1) the first modification: Y349 and T366; and the second modification: D356, T366, L368, Y407, and F405;
2) the first modification: Y349, T366, and F405; and the second modification: D356, T366, L368, and Y407;
3) the first modification: Y349, T366, and K409; and the second modification: D356, T366, L368, Y407, and F405;
4) the first modification: Y349, T366, F405, K360, and Q347; and the second modification: D356, T366, L368, Y407, and Q347;
5) the first modification: Y349, T366, F405, and Q347; and the second modification: D356, T366, L368, Y407, K360, and Q347;
6) the first modification: Y349, T366, K409, K360, and Q347; and the second modification: D356, T366, L368, Y407, F405, and Q347;
7) the first modification: Y349, T366, K409, and Q347; and the second modification: D356, T366, L368, Y407, F405, K360, and Q347;
8) the first modification: T366, K409, and K392; and the second modification: T366, L368, Y407, D399, and F405;
9) the first modification: T366 and K409; and the second modification: T366, L368, Y407, and F405;
10) the first modification: T366, K409, and Y349; and the second modification: T366, L368, Y407, F405, and E357;
11) the first modification: T366, K409, Y349, and S354; and the second modification: T366, L368, Y407, F405, and E357;
12) the first modification: T366 and F405; and the second modification: T366, L368, Y407, and K409;
13) the first modification: T366, F405, and D399; and the second modification: T366, L368, Y407, K409, and K392;
14) the first modification: T366, F405, and Y349; and the second modification: T366, L368, Y407, K409, and E357;
15) the first modification: T366, F405, Y349, and S354; and the second modification: T366, L368, Y407, K409, and E357;
wherein the position of the amino acid is determined according to the EU index of the KABAT number.

18. The use according to any one of the preceding claims, wherein the first Fc region comprises the first modification, and the second Fc region comprises the second modification, wherein the first modification and the second modification comprise a group of amino acid substitutions selected from any of the following groups:
1) the first modification: Y349C and T366W; and the second modification: D356C, T366S, L368A, Y407V, and F405K;
2) the first modification: Y349C, T366W, and F405K; and the second modification: D356C, T366S, L368A, and Y407V;
3) the first modification: Y349C, T366W, and K409E; and the second modification: D356C, T366S, L368A, Y407V, and F405K;
4) the first modification: Y349C, T366W, and K409A; and the second modification: D356C, T366S, L368A, Y407V, and F405K;
5) the first modification: Y349C, T366W, F405K, K360E, and Q347E; and the second modification: D356C, T366S, L368A, Y407V, and Q347R;
6) the first modification: Y349C, T366W, F405K, and Q347R; and the second modification: D356C, T366S, L368A, Y407V, K360E, and Q347E;
7) the first modification: Y349C, T366W, K409A, K360E, and Q347E; and the second modification: D356C, T366S, L368A, Y407V, F405K, and Q347R;
8) the first modification: Y349C, T366W, K409A, and Q347R; and the second modification: D356C, T366S, L368A, Y407V, F405K, K360E, and Q347E;
9) the first modification: T366W, K409A, and K392D; and the second modification: T366S, L368A, Y407V, D399S, and F405K;
10) the first modification: T366W and K409A; and the second modification: T366S, L368G, Y407A, and F405K;
11) the first modification: T366W, K409A, and Y349D; and the second modification: T366S, L368A, Y407V, F405K, and E357A;
12) the first modification: T366W, K409A, Y349D, and S354D; and the second modification: T366S, L368A, Y407V, F405K, and E357A;
13) the first modification: T366W and F405K; and the second modification: T366S, L368A, Y407V, and K409A;
14) the first modification: T366W, F405K, and D399S; and the second modification: T366S, L368A, Y407V, K409A, and K392D;
15) the first modification: T366W and F405K; and the second modification: T366S, L368G, Y407A, and K409A;
16) the first modification: T366W, F405K, and Y349D; and the second modification: T366S, L368A, Y407V, K409A, and E357A;
17) the first modification: T366W, F405K, Y349D, and S354D; and the second modification: T366S, L368A, Y407V, K409A, and E357A;
wherein the position of the amino acid is determined according to the EU index of the KABAT number.

19. The use according to any one of the preceding claims, wherein the first Fc region comprises the first modification, the second Fc region comprises the second modification, the first modification comprises the amino acid substitutions T366W and K409A, and the second modification comprises the amino acid substitutions T366S, L368G, Y407A, and F405K, wherein the position of the amino acid is determined according to the EU index of the KABAT number.

20. The use according to any one of the preceding claims, wherein the targeting moiety specifically binds to EGFR, the light chain of the first member comprises CDR1-3, the amino acid sequence of the CDR1 is as set forth in SEQ ID NO: 101, the amino acid sequence of the CDR2 is as set forth in SEQ ID NO: 102, and the amino acid sequence of the CDR3 is as set forth in SEQ ID NO: 103; the heavy chain of the first member comprises CDR1-3, the amino acid sequence of the CDR1 is as set forth in SEQ ID NO: 105, the amino acid sequence of the CDR2 is as set forth in SEQ ID NO: 106, and the amino acid sequence of the CDR3 is as set forth in SEQ ID NO: 107.

21. The use according to any one of claims 1-19, wherein the targeting moiety specifically binds to an EGFR mutant, the light chain of the first member comprises CDR1-3, the amino acid sequence of the CDR1 is as set forth in SEQ ID NO: 109, the amino acid sequence of the CDR2 is as set forth in SEQ ID NO: 110, and the amino acid sequence of the CDR3 is as set forth in SEQ ID NO: 111; the heavy chain of the first member comprises CDR1-3, the amino acid sequence of the CDR1 is as set forth in SEQ ID NO: 113, the amino acid sequence of the CDR2 is as set forth in SEQ ID NO: 114, and the amino acid sequence of the CDR3 is as set forth in SEQ ID NO: 115.

22. The use according to any one of claims 1-19, wherein the targeting moiety specifically binds to HER2/neu, the light chain of the first member comprises CDR1-3, the amino acid sequence of the CDR1 is selected from SEQ ID NOs: 117 and 125, the amino acid sequence of the CDR2 is selected from SEQ ID NOs: 118 and 126, and the amino acid sequence of the CDR3 is selected from SEQ ID NOs: 119 and 127; the heavy chain of the first member comprises CDR1-3, the amino acid sequence of the CDR1 is selected from SEQ ID NOs: 121 and 129, the amino acid sequence of the CDR2 is selected from SEQ ID NOs: 122 and 130, and the amino acid sequence of the CDR3 is selected from SEQ ID NOs: 123 and 131.

23. The use according to any one of claims 1-19, wherein the targeting moiety specifically binds to GPC3, the light chain of the first member comprises CDR1-3, the amino acid sequence of the CDR1 is as set forth in SEQ ID NO: 133, the amino acid sequence of the CDR2 is as set forth in SEQ ID NO: 134, and the amino acid sequence of the CDR3 is as set forth in SEQ ID NO: 135; the heavy chain of the first member comprises CDR1-3, the amino acid sequence of the CDR1 is as set forth in SEQ ID NO: 137, the amino acid sequence of the CDR2 is as set forth in SEQ ID NO: 138, and the amino acid sequence of the CDR3 is as set forth in SEQ ID NO: 139.

24. The use according to any one of claims 1-19, wherein the targeting moiety specifically binds to FAP, the light chain of the first member comprises CDR1-3, the amino acid sequence of the CDR1 is as set forth in SEQ ID NO: 141, the amino acid sequence of the CDR2 is as set forth in SEQ ID NO: 142, and the amino acid sequence of the CDR3 is as set forth in SEQ ID NO: 143; the heavy chain of the first member comprises CDR1-3, the amino acid sequence of the CDR1 is as set forth in SEQ ID NO: 145, the amino acid sequence of the CDR2 is as set forth in SEQ ID NO: 146, and the amino acid sequence of the CDR3 is as set forth in SEQ ID NO: 147.

25. The use according to any one of claims 1-19, wherein the targeting moiety specifically binds to Muc1, the light chain of the first member comprises CDR1-3, the amino acid sequence of the CDR1 is as set forth in SEQ ID NO: 149, the amino acid sequence of the CDR2 is as set forth in SEQ ID NO: 150, and the amino acid sequence of the CDR3 is as set forth in SEQ ID NO: 151; the heavy chain of the first member comprises CDR1-3, the amino acid sequence of the CDR1 is as set forth in SEQ ID NO: 153, the amino acid sequence of the CDR2 is as set forth in SEQ ID NO: 154, and the amino acid sequence of the CDR3 is as set forth in SEQ ID NO: 155.

26. The use according to any one of claims 1-19, wherein the targeting moiety specifically binds to Mesothelin, the light chain of the first member comprises CDR1-3, the amino acid sequence of the CDR1 is as set forth in SEQ ID NO: 165, the amino acid sequence of the CDR2 is as set forth in SEQ ID NO: 166, and the amino acid sequence of the CDR3 is as set forth in SEQ ID NO: 167; the heavy chain of the first member comprises CDR1-3, the amino acid sequence of the CDR1 is as set forth in SEQ ID NO: 169, the amino acid sequence of the CDR2 is as set forth in SEQ ID NO: 170, and the amino acid sequence of the CDR3 is as set forth in SEQ ID NO: 171.

27. The use according to any one of claims 1-19, wherein the targeting moiety specifically binds to MUC5AC, the light chain of the first member comprises CDR1-3, the amino acid sequence of the CDR1 is as set forth in SEQ ID NO: 157, the amino acid sequence of the CDR2 is as set forth in SEQ ID NO: 158, and the amino acid sequence of the CDR3 is as set forth in SEQ ID NO: 159; the heavy chain of the first member comprises CDR1-3, the amino acid sequence of the CDR1 is as set forth in SEQ ID NO: 161, the amino acid sequence of the CDR2 is as set forth in SEQ ID NO: 162, and the amino acid sequence of the CDR3 is as set forth in SEQ ID NO: 163.

28. The use according to any one of the preceding claims, wherein in the heavy chain of the first member, the amino acid sequence of the first Fc region is selected from SEQ ID NOs: 1, 4, 5, 6, 7, 9, 11, 13, 15, 17, 19, 21, 22, 24, 26, 27, and 29.

29. The use according to any one of the preceding claims, wherein the amino acid sequence of the immunoregulator comprised in the second member is selected from SEQ ID NOs: 173-180.

30. The use according to any one of the preceding claims, wherein the amino acid sequence of the second Fc region comprised in the second member is selected from SEQ ID NOs: 2, 3, 8, 10, 12, 14, 16, 18, 20, 23, 25, and 28.

31. The use according to any one of the preceding claims, wherein the amino acid sequence of the polypeptide comprised in the second member is selected from SEQ ID NOs: 77, 80, 82, 84, 86, 89, 91, and 97.

32. The use according to any one of the preceding claims, wherein the amino acid sequence of the light chain comprised in the first member is selected from SEQ ID NOs: 37, 45, 49, 53, 57, 61, 65, 69, and 73, the amino acid sequence of the heavy chain comprised in the first member is selected from SEQ ID NOs: 39, 47, 51, 55, 59, 63, 67, 71, and 75, and the amino acid sequence of the polypeptide comprised in the second member is selected from SEQ ID NOs: 77, 80, 82, 84, 86, 89, 91, and 97.

33. The use according to any one of the preceding claims, wherein the medicament comprises a pharmaceutical composition comprising the proteinaceous heterodimer according to any one of the preceding claims and a pharmaceutically acceptable carrier.

34. The use according to any one of the preceding claims, wherein the medicament comprises a nucleic acid molecule encoding the proteinaceous heterodimer according to any one of the preceding claims.

35. The use according to the preceding claim 34, wherein the medicament comprises a vector comprising the nucleic acid molecule.

36. The use according to any one of the preceding claims 34-35, wherein the medicament comprises a cell comprising the nucleic acid molecule or the vector.

37. The use according to any one of the preceding claims, wherein the medicament comprises a protein mixture comprising: 1) the proteinaceous heterodimer according to any one of claims 1-32; 2) a first homodimer formed by two of the first member of the proteinaceous heterodimer; and 3) a second homodimer formed by two of the second member of the proteinaceous heterodimer; wherein the percentage of the proteinaceous heterodimer in the protein mixture is at least 50%.

38. The use according to any one of the preceding claims, wherein a patient suffering from the malignant effusion comprises a tumor patient.

39. The use according to the preceding claim 38, wherein the patient has symptoms comprising fatigue, vomiting, edema in the dorsum of the foot, loss of appetite, dyspnea, cough, chest pain, and/or abdominal pain.

40. The use according to any one of the preceding claims 38-39, wherein the tumor comprises a solid tumor and/or a non-solid tumor.

41. The use according to any one of the preceding claims 38-40, wherein the tumor comprises lung cancer, lymph cancer, liver cancer, stomach cancer, intestinal cancer, breast cancer, pancreatic cancer, ovarian cancer, and/or mesothelioma.

42. The use according to any one of the preceding claims, wherein the malignant effusion comprises malignant cells.

43. The use according to any one of the preceding claims, wherein the malignant effusion comprises blood and/or exudative fluid.

44. The use according to any one of the preceding claims 38-43, wherein the malignant effusion is associated with metastasis of the tumor to the pleura.

45. The use according to any one of the preceding claims 38-44, wherein the malignant effusion is associated with metastasis of the tumor to the peritoneum.

46. The use according to any one of the preceding claims, wherein the malignant effusion is caused by a tumor comprising a primary tumor of the lung, lymph, liver, stomach, colon, breast, ovarian cancer, pancreatic cancer, bile duct, esophagus, brain, nose, and/or prostate.

47. The use according to any one of the preceding claims, wherein the malignant effusion is caused by metastasis to the thoracic cavity of a tumor comprising a primary cancer of the lung, lymph, liver, stomach, intestine, breast, ovarian cancer, and/or mesothelioma.

48. The use according to any one of the preceding claims, wherein the malignant effusion is caused by metastasis to the abdominal cavity of a tumor comprising ovarian cancer, pancreatic cancer, and a primary cancer of the bile duct, esophagus, sarcoma, and/or liver.

49. The use according to any one of the preceding claims, wherein the malignant effusion comprises malignant pleural effusion (MPE) and/or malignant ascites.

50. The use according to any one of the preceding claims, wherein the malignant pleural effusion is associated with non-small cell lung cancer.

51. The use according to any one of the preceding claims, wherein the medicament is formulated for oral administration, intravenous administration, intramuscular administration, in-situ administration at the site of a tumor, inhalation, rectal administration, vaginal administration, transdermal administration, or administration via subcutaneous repository.

52. The use according to any one of the preceding claims, wherein the medicament is configured to comprise a drainage device.

53. The use according to any one of the preceding claims, wherein the medicament is capable of inhibiting growth of a tumor or a tumor cell.

54. A method for constructing an animal model with a malignant effusion, comprising injecting a tumor cell into the thoracic cavity and/or the abdominal cavity of an animal.

55. The method according to claim 54, wherein a dose for the injection is about 1 × 10⁵ to about 2 × 10⁶ cells injected per animal.

56. The method according to any one of claims 54-55, wherein the animal comprises a mouse.

57. The method according to any one of claims 54-56, wherein the injection comprises intraperitoneal injection, intra-abdominal injection, and/or intrathoracic injection.

58. The method according to any one of claims 54-57, wherein the tumor cell is derived from a solid tumor and/or a non-solid tumor.

59. The method according to claim 58, wherein the tumor comprises lung cancer, lymph cancer, liver cancer, stomach cancer, intestinal cancer, breast cancer, pancreatic cancer, sarcoma, ovarian cancer, and/or mesothelioma.

60. The method according to any one of claims 54-59, wherein the tumor cell comprises an H22-Luc cell, a B16-EGFR cell, an MC38-EGFR cell, a Pan02 mouse pancreatic cancer cell, an S180 mouse sarcoma cell, and/or a Lewis-luc cell.

61. The method according to any one of claims 54-60, wherein the tumor cell is capable of causing a tumor in the animal.

62. The method according to claim 61, wherein the tumor metastasizes to the pleura and/or the peritoneum of the animal and produces the malignant effusion.

63. The method according to any one of claims 54-62, wherein the malignant effusion comprises malignant cells.

64. The method according to any one of claims 54-63, wherein the malignant effusion comprises blood and/or exudative fluid.

65. The method according to any one of claims 54-64, wherein the malignant effusion comprises malignant pleural effusion (MPE) and/or malignant ascites.

66. The method according to any one of claims 54-65, wherein the malignant pleural effusion is associated with non-small cell lung cancer.

67. A tissue or organ, or a culture thereof, in an animal model with a malignant effusion prepared by the method according to any one of claims 54-66.

68. An animal model with a malignant effusion or progeny thereof prepared by the method according to any one of claims 54-66.

69. Use of the tissue or organ, or the culture thereof, or the animal model with a malignant effusion or the progeny thereof according to any one of claims 67-68 in the screening, validation, evaluation, and/or research of an anti-tumor drug or as a model system for pharmacological, immunological, microbiological, and medical research.
